Europäisches Patentamt

European Patent Office

Office européen des brevets

(19)

(11) **EP 1 439 213 A1**

(12) **EUROPEAN PATENT APPLICATION**
published in accordance with Art. 158(3) EPC

(43) Date of publication:
**21.07.2004 Bulletin 2004/30**

(51) Int Cl.[7]: **C09K 5/18**, A61F 7/08

(21) Application number: **02777954.5**

(22) Date of filing: **25.10.2002**

(86) International application number:
**PCT/JP2002/011097**

(87) International publication number:
**WO 2003/035792 (01.05.2003 Gazette 2003/18)**

(84) Designated Contracting States:
**AT BE BG CH CY CZ DE DK EE ES FI FR GB GR IE IT LI LU MC NL PT SE SK TR**

(30) Priority: **25.10.2001 JP 2001328344**

(71) Applicant: **Mycoal Warmers Co., Ltd**
**Tochigi-Shi Tochigi 328-0067 (JP)**

(72) Inventors:
- **USUI, Kaoru, c/o Mycoal Warmers Co., Ltd.**
  **Tochigi-shi, Tochigi 328-0067 (JP)**
- **AIDA, Michio, c/o Mycoal Warmers Co., Ltd.**
  **Tochigi-shi, Tochigi 328-0067 (JP)**

- **SAKAMAKI, Yoshikazu,**
  **c/o Mycoal Warmers Co., Ltd.**
  **Tochigi-shi, Tochigi 328-0067 (JP)**
- **NAKAMURA, Masato,**
  **c/o Mycoal Warmers Co., Ltd.**
  **Tochigi-shi, Tochigi 328-0067 (JP)**
- **KIMURA, Hisao, c/o Mycoal Warmers Co., Ltd.**
  **Tochigi-shi, Tochigi 328-0067 (JP)**
- **DODO, Toshihiro, c/o Mycoal Warmers Co., Ltd.**
  **Tochigi-shi, Tochigi 328-0067 (JP)**

(74) Representative: **Körfer, Thomas, Dipl.-Phys.**
**Mitscherlich & Partner,**
**Patent- und Rechtsanwälte,**
**Sonnenstrasse 33**
**80331 München (DE)**

(54) **EXOTHERMIC COMPOSITION AND EXOTHERMIC ARTICLE USING THE COMPOSITION, AND METHOD FOR PRODUCING THE EXOTHERMIC ARTICLE**

(57)     It relates to a heat-generating composition having such dispersion stability that withstands continuous molding, and has excellent drainage property, excellent heat-generating characteristics, excellent molding property and excellent shape retaining property, without becoming viscous, and relates to a heat-generating body using the same and a process for producing the heat-generating body.

It contains, as essential components, a heat-generating substance generating heat upon reaction with oxygen, a carbon component, an oxidation promoter and water, characterized in that the composition further contains a water separation-preventing stabilizer in a proportion of from 0.001 to 0.25 part by mass per 100 parts by mass of the heat-generating composition, and has a water mobility value of from 7 to 40 and a separation degree of from 0 to 30.

**Fig.1**

**EP 1 439 213 A1**

**Description**

TECHNICAL FIELD

The present invention relates to a heat-generating composition containing a heat-generating substance generating heat upon reaction with oxygen, a carbon component, an oxidation accelerator and water, as essential components, with which an ultramicro amount of a water separation-preventing stabilizer is mixed, whereby the composition has such dispersion stability that withstands continuous molding, and has excellent drainage property, excellent heat-generating characteristics, excellent molding property and excellent shape retaining property, without becoming viscous, and relates to a heat-generating body using the same and a process for producing the heat-generating body.

BACKGROUND ART

Representative examples of applications of a heat-generating substance generating heat upon reaction with oxygen include a powder heat-generating composition used in a heat-generating body, such as a disposal body warmer.
As a heat-generating composition containing a metal as an essential component, a disposal body warmer has been conventionally exemplified, which generally contains a bag-shaped material formed with a base material, such as a nonwoven fabric, a porous film, paper, and a fine pore film, and a covering material, in which a mixture of a heat-generating composition, such as metallic powder such as iron powder, activated carbon, and a metallic chloride, and water is filled and sealed, and upon using the same, heat generation caused by an oxidation reaction of metallic powder with oxygen in the air is utilized.
With respect to a powder heat-generating composition, JP-A-U-H5-30432 proposes as follows. In the heat generating state of a powder heat-generating composition in a container bag, an aeration surface is provided in the container bag for the heat-generating composition, so that the pressure inside the container bag is maintained at a reduced pressure in comparison to the outside pressure in a certain range of pressure, whereby oxygen is introduced into the container bag for the heat-generating composition through the air permeability of the aeration surface to cause an oxidation reaction simultaneously. According to the procedures, the pressure inside the bag is lowered to maintain the heat-generating composition at a prescribed position, whereby the flat shape thereof is maintained within the period where it is applied to an application position of a body or the like. It requires, however, that it is necessary that both the particle constitutional components of the particulate heat-generating composition and the material of the container bag are significantly carefully selected. In particular, this technique only allows a slight degree of fluctuation in air permeability of the bag caused by lot-by-lot differences of the material characteristics of the air permeable materials thus produced. Furthermore, in the case of a powder heat-generating composition, it has large flowability to provide such a possibility that the heat-generating composition moves inside the bag upon storage and transportation to cause deviation inside the bag.
Such heat-generating bodies have been proposed in that a binder (JP-B-H4-8697), a thickener (JP-B-H7-112477 and JP-A-H9-75388), an excipient (JP-A-H7-194641), an aggregation assistant (JP-A-H11-508314) or the like is added to a heat-generating composition, whereby the powder of the heat-generating composition is bound, or the viscosity thereof is increased, to make a heat-generating composition in a viscous form, a cream form or a paste form, and it is laminated on the base material and covered with a covering material, followed by sealing the circumference.
Furthermore, a heat-generating composition in a suspension form or a slurry form using a large amount of water has been also proposed for forming by a paper making technique.
The shape of a body warmer is modified by forming the bag having air permeability into a horseshoe shape or a trapezoidal shape, and it is utilized for a heat-generating body for a footgear, such as shoes and slippers. As described in JP-A-S60-101448, JP-A-H10-216167, JP-A-H11-508314 and the like, it has also been proposed that a heat-generating composition is made into a viscous form or a cream form, and the shape thereof is changed from the conventional square shape to a rectangular form or a circular form to fit to a body to be warmed.
In all the foregoing techniques, a heat-generating composition containing metallic powder as a major component is charged in a bag material, and upon using, the function thereof is exerted through an oxidation reaction of oxygen in the air and the metallic powder in the heat-generating composition. The heat-generating material is generally in a powder form, and as described in the foregoing, those in a cream form or a paste form are also present by adding a thickener or the like.
A sheet heat-generating body described in Japanese Patent No. 2,572,621 is formed into a sheet form by a paper making technique. In this technique, a fibrous material, activated carbon, an electrolyte and other additives are mixed and agitated in water to form a suspension, which is then subjected to a paper making machine, in which the suspension is filtered with wires and aspirated for dehydration, and it is further dehydrated by pressing between canvas sheets, followed by shaping into a desired thickness, so as to form a heat-generating body in a sheet form.

Such a proposal has been made in the case of a heat-generating composition in a cream form that a thickener is added to a heat-generating composition to make it in a cream form by increasing the viscosity thereof, and it is laminated on a water absorbing support, such as paper, by printing or coating to produced a thin heat-generating body. Thereafter, part of free water and/or water content in a water-containing gel is absorbed by the support or a covering material to improve contact with the air for starting the heat-generating reaction.

As a method for throwing down the heat-generating composition of the powder heat-generating composition, there are such a method that a bag base material is intermittently moved, and the powder heat-generating composition is thrown down during the bag base material is under suspension, as appearing in a method of charging the heat-generating body in a container bag, and such a method that the base material is moved at a constant speed, and the powder heat-generating composition is thrown down from a throw down outlet onto the base material, as appearing in a method of dispersing the powder-heat generating body in a nonwoven fabric, to which water is added to form a sheet heat-generating body. However, both cases involve problems from the standpoint of speeding up.

The powder heat-generating composition also has such a problem that the heat-generating composition causes a heat-generating reaction in the production process thereof to cause deterioration in product quality, such as loss of the heat-generating composition and fluctuation of quality. Furthermore, it is also the case in the production process of the heat-generating body.

A composition used in the invention is generally in a form or powder outside water, and it is difficult to stick to itself or other materials and is difficult to maintain the shape of the mixture by itself.

A semi-kneaded heat-generating composition using no water as a reaction inhibitor but containing a binder in a powder composition causes complication in process since it is molded by inserting a compression or tabulating step in the production process.

In a heat-generating composition using water as a reaction inhibitor, a cream heat-generating composition maintaned in dispersibility formed by increasing the viscosity of the total heat-generating composition, for example, with a thickener or the like can be improved in water separation property, molding property and shape maintaining property, but is considerably deteriorated in heat-generating characteristics, whereby it causes an unsatisfactory product.

In the case where a viscosity increasing agent, such as a thickener, is removed therefrom, and water is used as a molding agent, the heat-generating characteristics can be maintained, but significant water separation occurs to cause a possibility of failure of production with stable quality.

That is, in the case of the heat-generating composition containing a thickener or the like but being imparted with the molding property and the shape maintaining property by controlling the compounding ratio of water in view of importance of flowability and heat-generating property, the supplying amount of the heat-generating composition upon molding, such as mold-through molding or the like, is restricted due to maintenance of the dispersion state upon supplying the heat-generating composition to the mold. A large amount of the heat-generating composition is necessarily supplied to a mold upon high-speed operation, and thus molded products cause fluctuation. In order to avoid the fluctuation, it is necessary that an agitation device is provided near the mold, and sufficient agitation is carried out on the mold to maintain dispersibility, whereby the machine is necessarily complicated. Furthermore, in the case where the plant is temporarily suspended due to trouble of the machine, it is an important issue on production that component separation in the heat-generating composition is prevented to prepare for the subsequent operation. Therefore, in the case of the suspension of the machine, it is necessary that continuous agitation with an agitator or the like is carried out to prevent separation of solid contents and water content and sedimentation of solid contents, and thus there is a problem upon carrying out high speed continuous production.

Upon supplying the heat-generating composition, it is desired that separation of water and solid contents is prevented to attempt homogenization of the composition, whereby the quality of the composition is improved and maintained.

There have been some cases where heat-generating body produced from the heat-generating composition causes cracks in the heat-generating body itself upon use when the heat-generating body itself is moved, and it is broken to fail to exert sufficient performance.

A slurry heat-generating composition containing a large amount of excessive water, which is another example of the heat-generating composition using water as a heat generation controlling agent, has problems in dispersion stability and moldability of the composition, and the step for removing water becomes complicated to cause problem in productivity. It also has a problem on heat-generating characteristics.

Accordingly, advantages of using excessive water as an oxidation controlling agent, such as control of heat-generation, have not been enjoyed.

In order to meet the market needs, cost reduction and quality stabilization by high speed production with a simple machine are demanded, and the usability for users is to be improved by diversification in thickness and shape.

Therefore, in the heat-generating composition controlling the heat-generating reaction with water capable of being formed into various kinds of shapes, when the viscosity of the total heat-generating composition is increased, the heat-generating characteristics are significantly deteriorated to cause unsatisfactory products, and when the stable moldability and shape maintenance property are intended to attain mainly by water without increase of the viscosity, the excessive water is immediately separated to make mass production difficult. Therefore, it is a tough problem that such a heat-generating composition is produced with stable quality that is controlled in heat-generating reactivity with water, can be formed in various kinds of shapes, and exerts excellent heat-generating property. Thus, such a heat-generating composition and a product utilizing the same are demanded that solve the problems to have excellent dispersion stability, high speed productivity by lamination by printing or the like or a transfer technique or the like, formability into various kinds of shapes, shape maintaining property, and excellent heat-generating characteristics (such as rising property, maximum temperature, duration of desired temperature and the like).

Under the circumstances, the inventors have made earnest studies for solving the conventional problems and have carried out various kinds of systematic experiments, and the invention has been completed as a result of investigations for obtaining such a composition that can be conveniently produced at low cost, is conveniently handled, has excellent workability, can be molded in a simple and inexpensive manner, and has excellent functions, such as heat generation and the like.

It has been found that in the case where a heat-generating composition contains, as essential components, a heat-generating substance generating heat upon reaction with oxygen, a carbon component, an oxidation promoter and water, to which a water separation-preventing stabilizer is mixed in a proportion of from 0.001 to 0.25 part by mass per 100 parts by mass of the heat-generating substance, and has a separation degree of from 0 to 30 and a water mobility value of from 7 to 40, the oxidation reaction is controlledwith excessive water, the dispersion stability of preventing separation of the components due to water separation is exponentially improved with maintenance of excellent drainage property and oxidation reactivity, and deterioration upon storage in an airtight bag can be significantly prevented, whereby the invention has been completed.

According thereto, the water separation prevention, which is the greatest problem of a heat-generating composition controlled in oxidation reactivity with water and depending on water in moldability and shape maintenance property, can be significantly improved. In addition, loss of the heat-generating composition and the heat-generating body due to the heat-generating reaction can be suppressed even upon production in an atmosphere containing oxygen, such as in the air. That is, because the moldability and the shape maintenance property in the invention are not obtained by high viscosity of the entire heat-generating composition, the heat-generating characteristics can be maintained at a level equivalent to a powder body, and the separation degree of the heat-generating composition can be suppressed to a half or less in comparison to the case where the water separation-preventing stabilizer is not added, whereby nonuniformity in the production steps of the heat-generating composition or the heat-generating body is prevented, so as to suppress deterioration in quality and to enable stable continuous working productivity. Furthermore, various kinds of shapes can be formed with a simple equipment, so as to provide such a heat-generating composition, a heat-generating body using the same and production processes therefor, that are excellent in water separation preventing dispersion stability, water holding property, deterioration prevention property of the water holding property, flowability, shape maintenance property, drainage property, air permeability, heat-generating property (such as rising property, maximum temperature, duration of desired temperature and the like), and prevention property of time-lapse deterioration of the heat-generating property.

It is expected in the invention, while not clear in detail, that the water separation-preventing stabilizer in an ultramicro amount provides a crosslinked structure, in which the components of the composition are flexibly connected with spotted intervention of the water separation-preventing stabilizer, to provide a flexible skeleton having gaps, and an aqueous solution is retained in the gaps to provide a heat-generating composition excellent in flowability, i.e., moldability, and in drainage property, i.e., heat-generating characteristics. It is also expected that owing to the maintenance of the suitable water amount by the water separation-preventing stabilizer, excellent moldability is obtained, and after molding, excessive water can be easily drained by water absorption of a water absorbing material or a water absorbing agent or dehydration under pressure. Furthermore, formation of pores in the molded body can be ensured by the flexible structure to provide a porous body, so as to obtain excellent heat-generating characteristics.

That is, the inventors have considered that the heat-generating composition and water are mediated by utilizing affinity or adhesion (cohesion) of the dispersion medium and the dispersoid, so as to utilize the so-called protective effect of colloid or the similar effects, in which stably dispersed adhesive or adhesive particles, such as hydrophilic colloid particles and emulsion, are adsorbed on the surface of an inorganic compound or an organic compound, such as metallic powder, a water absorbing water retainig agent, a water absorbing carbon component and the like, which are hydrophobic particles having different values of specific gravity and having such shapes that are not interlaced with each other, such as a powder form, a particulate form, an acicular form, a squamous form or the like.

However, in the case where the components of the heat-generating composition and water are mixed with a large amount of a dispersion stabilizer, a slurry of the components of the heat-generating composition thus formed has a large viscosity to deteriorate the flowability, and the function, such as heat-generating and characteristics, of the composition is considerably deteriorated.

However, it has been found that in the case where the excessive water amount of the composition is in a certain range, and an ultramicro amount of the water separation-preventing stabilizer is mixed, increase of non-reactive points or hardly reactive points on the metallic component due to attachment of the dispersion stabilizer or the like reasons is prevented, and the metallic component and other components of the composition are stably dispersed to prevent precipitation and separation in the composition having various kinds of particles being mixedly present therein, whereby the dispersion stability of the composition is ensured, and such a composition is obtained that is not increased in viscosity in comparison to that before the additionof thedispersion stabilizer, andhas such functions as good flowability, excellent oxidation reactivity and the like. It is expected that the phenomena are caused by the following reasons.

Hydrophilic colloid formed by aggregation of fine particles with a dispersion stabilizer containing a polymer substance is a water soluble polymer substance and has a large amount of a hydrogen bonding group, and thus it is adsorbed on particles of a water retainig agent such as wood flour, activated carbon and the like, with the hydrogen bonding group, irrespective to electricity and ions. In the case where a small amount of the polymer is adsorbed on the particles of the components of the composition, it is not adsorbed over the entire surface of the particles but is adsorbed nondensely. Therefore, a part of the polymer adsorbed on one particle is adsorbed on another particle at an empty space thereon, i.e., one polymer is connected to two particles. It is expected that the particles of the components of the heat-generating composition are aggregated by the mechanism. This is a phenomenon referred to as "crosslinking aggregation", and it is expected that the particles are lightly aggregated to form a network structure in the heat-generating composition, so as to prevent sedimentation of the heat-generating composition.

Furthermore, gaps formed on mixing or draining excessive water are not united but remain as gaps having a continuous fine pore structure owing the network structure, so as to provide a uniform gap structure having good air permeability, and thus excellent drainage property and air permeability are imparted to a functional composition or a functional body having functions, such as heat generation. It is accordingly expected that such a heat-generating composition can be obtained that has excellent dispersion stability and sufficient flowability with excellent heat-generating characteristics maintained.

Furthermore, it is possible that a dispersant, such as a surfactant or the like, is added to the particles of the components of the heat-generating composition having been subjected to crosslinking aggregation to improve the flowability thereof.

With respect to the order of mixing of the dispersion stabilizer and the surfactant, the dispersion stabilizer may be added simultaneously with, before or after the other.

It is expected that dispersion stability is attained by the crosslinking aggregation or the like phenomena with the adhesive substance or the adhesive substance in the case where an emulsion is used.

The heat-generating composition of the invention is supplied by applying a shearing force to the heat-generating composition in a storage state or a suspension state. That is, a shearing force is applied by a method of applying pressure on the heat-generating composition, a method of extruding it with a screw or the like, or a combination of them.

## DISCLOSURE OF THE INVENTION

Accordingly, as described in claim 1, the heat-generating composition of the invention contains, as essential components, a heat-generating substance generating heat upon reaction with oxygen, a carbon component, an oxidation promoter and water, characterized in that the composition further contains a water separation-preventing stabilizer in a proportion of from 0.001 to 0.25 part by mass per 100 parts by mass of the heat-generating substance, and has a water mobility value of from 7 to 40 and a separation degree of from 0 to 30.

The heat-generating composition as described in claim 2 is a heat-generating composition as described in claim 1, characterized in that at least one selected from a water retainig agent, a surfactant, a defoaming agent, a pH controlling agent, a hydrophobic polymer compound, a pyroelectric substance, a far infrared radiating substance, a negative ion generating substance, a hydrogen formation inhibitor, an antioxidant, an aggregate, a fibrous material, a fertilizer component and a heat-generating assistant is mixed with the heat-generating composition.

The heat-generating composition as described in claim 3 is a heat-generating composition as described in claim 2, characterized in that the water retainig agent contains an organic water retainig agent, and particles having a particle diameter of 1,000 μm or less occupies at least 50% or more by mass of the water retainig agent.

The heat-generating composition as described in claim 4 is a heat-generating composition as described in one of claims 1 to 3, characterized in that an incremental degree of viscosity (at a temperature of 20°C) is less than 1,000 cP.

The heat-generating composition as described in claim 5 is a heat-generating composition as described in claim 4, characterized in that at least one selected from a binder, a thickening agent, an excipient, an aggregating agent, a soluble adhesive material and a water absorbing polymer is mixed with the heat-generating composition.

The heat-generating composition as described in claim 6 is a heat-generating composition as described in claim 5, characterized in that the water absorbing polymer does not contain a cyclic anhydride, an acid derived therefrom, and a salt thereof.

The heat-generating body as described in claim 7 is characterized by charging a heat-generating composition as described in claim 1 in a container bag having air permeability in at least a part thereof.

The heat-generating body as described in claim 8 is a heat-generating body as described in claim 7, characterized in that the container bag contains a base material in a film form, a sheet form or a nonwoven fabric form, and a covering material in a film form, a sheet form or a nonwoven fabric form, at least one of, or at least a part of the base material and the covering material has air permeability.

The heat-generating body as described in claim 9 is characterized by laminating a heat-generating composition as described in claim 1 on a laying material in a film form, a sheet form or a nonwoven fabric form, and optionally further laminating another laying material thereon, and at least one, or at least a part of the laying material has air permeability.

The heat-generating body as described in claim 10 is a heat-generating body as described in claim 9, characterized in that at least a part of the heat-generating composition of the heat-generating body is in a state dehydrated to enable substantial heat generation in air by at least one means selected from physical forced drainage by compression, decompression, compression and decompression, and the like, radiation of water content by allowing to stand, and water absorption with a material such as a water absorbing base material, a water absorbing agent, a water absorbing material or the like.

The heat-generating body as described in claim 11 is a heat-generating body as described in claim 10, characterized by charging the heat-generating body in a container bag having air permeability in at least a part thereof.

The heat-generating body as described in claim 12 is a heat-generating body as described in claim 8 or 9, characterized in that a part of water content of the heat-generating composition is absorbed and/or held by at least one, or at least a part of the container bag, the base material, the covering material and the laying material.

The heat-generating body as described in claim 13 is a heat-generating body as described in claim 12, characterized in that at least one selected from iron powder, a carbon component, a fibrous material, a binder, a thickener, an excipient, an aggregating agent, a soluble adhesive material, a far infrared radiating substance, a negative ion generating substance, a pyroelectric substance, an organic silicon compound, a water absorbing agent, a water absorbing polymer and a water separation-preventing stabilizer is laminated, diffused or coated on one surface or both surfaces of the heat-generating composition.

The heat-generating body as described in claim 14 is a heat-generating body as described in claim 13, characterized in that the water absorbing polymer does not contain a cyclic anhydride, an acid derived therefrom, and a salt thereof.

The heat-generating body as described in claim 15 is a heat-generating body as described in claim 12, characterized in that a part or whole of a surface of at least one selected from the covering material, the heat-generating composition and those laminated or diffused on the heat-generating composition is covered with an air permeable polymer.

The heat-generating body as described in claim 16 is a heat-generating body as described in claim 15, characterized in that in at least a periphery of the heat-generating composition, the base material and the covering material of the container bag are sealed by adhesion, cohesion or thermal fusion over a full circumference or in a part thereof.

The heat-generating body as described in claim 17 is a heat-generating body as described in claim 16, characterized in that at least one, or at least a part of the base material, the covering material and the laying material is formed with a water absorbing material having water absorbing property in a film form, a sheet form or a nonwoven fabric form.

The heat-generating body as described in claim 18 is a heat-generating body as described in claim 17, characterized in that a water absorbing layer containing a water absorbing material or a water absorbing agent is provided on at least a contact part or a part of the contact part of the base material and/or the covering material and/or the laying material in contact with the heat-generating composition.

The heat-generating body as described in claim 19 is a heat-generating body as described in claim 18, characterized in that a part having water absorbing power of the base material, the covering material and the laying material, and the water absorbing layer have a water absorbing power of 1 $g/m^2$ or more.

The heat-generating body as described in claim 20 is a heat-generating body as described in claim 19, characterized in that at least one of the base material, the covering material and the laying material has a elongation property.

The heat-generating body as described in claim 21 is a heat-generating body as described in claim 20, characterized in that at least one, or at least a part of the base material, the covering material and the laying material is laminated with, is coated with, or contains at least one of a far infrared radiating substance, a negative ion generating material and a pyroelectric substance.

The heat-generating body as described in claim 22 is a heat-generating body as described in claim 21, characterized in that unevenness is formed on a whole surface or a part thereof of a surface layer of the heat-generating composition.

The heat-generating body as described in claim 23 is a heat-generating body as described in claim 22, characterized in that unevenness is formed on a part or whole of a surface of a layer of the heat-generating composition or a part or whole of a surface of a material having the heat-generating composition laminated thereon.

The heat-generating body as described in claim 24 is a heat-generating body as described in claim 12, characterized in that an adhesive agent layer or a gel layer is laminated on an exposed surface or a part thereof of one of the base material and the covering material.

The heat-generating body as described in claim 25 is a heat-generating body as described in claim 24, characterized in that the adhesive agent layer or the gel layer is a fomentation layer containing a fomentation drug, or a drug-containing layer having a percutaneous absorption drug contained or supported therein.

The heat-generating body as described in claim 26 is a heat-generating body as described in claim 25, characterized in that a heat-generating composition as described in claim 1 is provided in a prescribed form on at least one selected from a base material, a laying material and a covering material, and is thenmade into such a state that it is charged in a container bag having air permeability on at least a part thereof, whereby the heat-generating composition under a heat-generating state is retained at a prescribed position irrespective to a pressure inside the container bag with respect to an outer pressure, the bag is maintained to have a flat form during a period where the body is applied to an applicable position, and a deviation of the heat-generating composition is 10% or less in comparison to that before heat generation.

The process for producing a heat-generating body as described in claim 27 is characterized by molding such as laminating, a heat-generating composition as described in claim 1 on at least one prescribed region on a base material in a film form, a sheet form or a nonwoven fabric form; overlaying a covering material in a film form, a sheet form or a nonwoven fabric form to cover and charge the heat-generating composition; and imparting air permeability to at last one, or at least a part of the base material and the covering material.

The process for producing a heat-generating body as described in claim 28 is characterized by, upon laminating a heat-generating composition as described in claim 1 on a base material in a film form, a sheet form or a nonwoven fabric form, laminating, diffusing or coating at least one selected from iron powder, a carbon component, a fibrous material, a binder, a thickener, an excipient, an aggregating agent, a soluble adhesive material, a far infrared radiating substance, a negative ion generating substance, a pyroelectric substance, an organic silicon compound, a water absorbing agent, a water absorbing material, a water absorbing polymer, a water hoding agent, and a dispersing stabilizer on one surface or both surfaces of the laminated body of the heat-generating composition; overlaying a covering material in a film form, a sheet form or a nonwoven fabric form to cover and charge the laminated body of the heat-generating composition and those thus laminated, diffused or coated; and imparting air permeability to at last one, or at least a part of the base material and the covering material.

The process for producing a heat-generating body as described in claim 29 is characterized by laminating a heat-generating composition as described in claim 1 on a base material in a film form, a sheet form or a nonwoven fabric form; overlaying a covering material in a film form, a sheet form or a nonwoven fabric form thereon; adhering the base material and the covering material with at least one selected from the basematerial, the laminated material andthe covering material, or an air permeable polymer provided on a part thereof; punching out the resulting laminated material into an arbitrary shape; and imparting air permeability to at last one, or at least a part of the base material and the covering material.

The process for producing a heat-generating body as described in claim 30 is characterized by laminating a heat-generating composition as described in claim 1 on a water permeable material; covering the laminated material with a water permeable material; carrying out physical forced drainage by aspiration, centrifuge, compression, decompression, drying, or compression and decompression, to form a laminated body, and optionally laminating it to form a laminated body; punching out the laminated body into an arbitrary shape, and placing the punched laminated body on a base material, or placing the laminated body without punching on a base material; overlaying a covering material in a film form, a sheet form or a nonwoven fabric form thereon; sealing at least the base material and the covering material among the base material, the covering material and the water permeable material on a periphery thereof by at least one selected from cohesion, adhesion and thermal fusion; and in the case where the laminated body has not been punched out into an arbitrary shape, punching the laminated body into an arbitrary shape; and imparting air permeability to at last one, or at least a part of the base material and the covering material.

The process for producing a heat-generating body as described in claim 31 is a process for producing a heat-generating body as described in claim 30, characterized in that at least one, or at least a part of the base material, the covering material and the laying material is laminated with, is diffused with, or contains at least one of a far infrared radiating substance, a negative ion generating substance and a pyroelectric substance.

The process for producing a heat-generating body as described in claim 32 is a process for producing a heat-generating body as described in claim 30 or 31, characterized in that the heat-generating body is inserted between air non-permeable films or sheets; and simultaneously with the insertion or after the insertion, the films or sheets are sealed on a periphery of the heat-generating body to a size exceeding a size of the heat-generating body, and simultaneously with the sealing or after the sealing, the resulting assembly is punched out, or simultaneously with the insertion or after the insertion, the two films or sheets are punched out to a size exceeding a size of the heat-generating body, and simultaneously with the punching or after the punching, the films or sheets are sealed on a periphery of the heat-generating body to a size exceeding a size of the heat-generating body.

The heat-generating composition according to the invention contains, as essential components, a heat-generating substance generating heat upon reaction with oxygen, a carbon component, an oxidation promoter and water, and preferably has an incremental degree of viscosity is less than 1,000 cP to control the reactivity of the heat-generating composition with excessive water, whereby loss on production and nonuniformity and failure of quality are prevented, the dispersion stability is ensured, and deterioration of the heat-generating characteristics is prevented. The composition has a water mobility value of from 7 to 40 and a separation degree of from 0 to 30, whereby the amount of excessive water can be accurately ensured to obtain a functional composition having functions, such as heat generation, deoxygenation and the like, and having dispersibility, dispersion stability, water holding property, prevention of deterioration in water holding, moldability, shape maintenance property, adhesiveness, heat-generating characteristics and prevention of deterioration in heat-generating characteristics. In particular, the prevention of deterioration in heat-generating characteristics, which is considered to be attained by the network structure, is improved, and thus the time-lapse deterioration of the heat-generating duration can be significantly prevented to increase the commercial value to a large extent.

Furthermore, in the case where the heat-generating composition is molded into a heat-generating body, because the shape maintenance property and the adhesiveness are excellent, such a heat-generating body can be provided that causes no deviation in the bag, does not impair application feeling upon use, causes less fluctuation in desire temperature characteristics and heat generation among the products, and has stable quality, even in the case where the heat-generating body is contained in a bag formed with a material having a large air permeation'amount, which can be easily produced, needless to say in a air permeable container bag having a small air permeation amount.

Furthermore, in the case where a fibrous material is mixed, such a functional material can be obtained that the heat-generating function can be effectively exerted through contact with the air, and the fibrous material thus mixed and the heat-generating composition are interlaced with each other to cause such a state that is as closed as that gaps among the extrafine fibers are filled with the heat-generating composition, whereby the molded heat-generating composition can be easily formed in to a sheet form having excellent shape maintenance property with a rolling machine such as a press roll.

That is, a functional composition of heat-generating property and the like having a uniform composition and maintaining a stable dispersion state can be produced, and by stably molding the functional composition, a uniform heat-generating or deoxygenating body having various kinds of shapes and stable and high heat-generating characteristics or high deoxygenating characteristics can be produced. Printing and transferring, such as embossing molding, molding by pressing on a mold, gravure printing and the like, can be carried out by applying flowability and shape maintenance property, whereby such a production process is established that a heat-generating body having high heat-generating characteristics and a deoxygenating body having a high deoxygenating characteristics having various kinds of shapes and having from an ultrathin form to a thick form can be continuously molded at a high speed.

BRIEF DESCRIPTION OF THE DRAWINGS

Fig. 1 is a cross sectional view of a heat-generating body according to Example 1 of the invention.
Fig. 2 is a heat-generation characteristic diagram of heat-generating bodies of the invention and the comparative examples.
Fig. 3 is a heat-generation characteristic diagram of a separation degree test of heat-generating bodies of the invention and the comparative examples.
Fig. 4 is a heat-generation characteristic diagram of a deterioration test of heat-generating bodies of the invention and the comparative examples.
Fig. 5 is a cross sectional view of a heat-generating body according to Example 2 of the invention.
Fig. 6 is a cross sectional view of a heat-generating body according to Example 3 of the invention.
Fig. 7 is a cross sectional view of a heat-generating body according to Example 4 of the invention.
Fig. 8 is a plane view of a heat-generating body according to Example 6 of the invention.
Fig. 9 is a cross sectional view on line IX-IX in Fig. 8.
Fig. 10 is a schematic diagram of mold-through molding in the invention.
Fig. 11 is a schematic diagram of molding using a leveling plate.
Fig. 12 is a schematic diagram of molding using a pressing and leveling plate.
Fig. 13 is a schematic diagram of a heat-generating body production apparatus for preferably producing a heat-generating body of the invention.

Fig. 14 is a cross sectional view of another example of a heat-generating body of the invention.

Fig. 15 is a schematic diagram of an important part of a sealing apparatus according to production of the same example.

Fig. 16 is a plane view of filter paper for measuring a water mobility value in the heat-generating composition of the invention.

Fig. 17 is a schematic diagram showing a measuring method of a water mobility value in the heat-generating composition of the invention.

Fig. 18 is a cross sectional side view of the same.

Fig. 19 is a cross sectional side view of the same.

Fig. 20 is a plane view of filter paper after carrying out measurement of a water mobility value in the invention.

Fig. 21 is a schematic diagram showing a measuring method of a separation degree in the invention.

BEST MODE FOR CARRYING OUT THE INVENTION

A comparison of the invention and representative examples of the conventional proposals assuming the same constitutional components is shown below.

Table 1

|  | Invention | Slurry form | Cream form | Semi-kneaded form | Powder form |
|---|---|---|---|---|---|
| Thickener/binder | none | none/present | constant amount | constant amount | none |
| Water separation-preventing stabilizer | ultramicro amount | none | none | none | none |
| Control with water | present | present | present | none | none |
| Water mobility value | 7-40 | >50 | <7 | <7 | <7 |
| Separation degree | 0-30 | > 120 | 0 | 0 | 0 |
| Dispersion stability | A | C | A | A | A |
| High-speed molding property | A | C | B* | C | C |
| Shape maintenance property | A | C | A | A | C |
| Heat-generating property | A | C | C | B | B |

Evaluation: excellent A > B > C poor Amount: constant amount > ultramicro amount *) In the case of uniform lamination on a soft base material, such as nonwoven fabric upon molding using a mold, such as press molding, the laminated material is released, and lamination is difficult to be carried out.

The heat-generating composition of the invention has been realized by obtaining such findings that the composition contains, as essential components, a heat-generating substance generating heat upon reaction with oxygen, a carbon component, an oxidation accelerator and water, with which an ultramicro amount of a water separation-preventing stabilizer is mixed, whereby the dispersibility and the dispersion maintenance property of the heat-generating composition can be considerably improved without deterioration of the heat-generating characteristics.

The heat-generating composition of the invention can considerably easily eject free water, can be easily laminated by such molding means as a printing method, a transferring method and the like, such as mold through and leveling molding, pressing molding, pressing and leveling molding, gravure printing utilizing an engraved plate (such as pressing and transferring molding) , screen printing as a kind of stencil printing, coating and the like, can be produced into heat-generating bodies having various kinds of shapes including an ultrathin form, a thick form, a square form, a circular form and the like, at a high speed with good yield, and can prevent formation of powder dusts upon production of the heat-generating bodies to cause no contamination of working environment and to provide high cleanness.

Furthermore, the heat-generating composition also has such characteristics that it can be uniformly distributed in a bag material, and when it is laminated on an absorbing base material, the heat-generating composition breaks into fine pores of the base material, the covering material or the laying material owing to the high break-in and anchor effect of the heat-generating composition, and thus migration and deviation thereof are prevented, and the effect is increased when the water absorbing property is increased.

A magnet may be combined upon molding the composition of the invention. The transferring method and the molding method can be easily conducted by combining a magnet with the composition of the invention. For example, the heat-generating composition can be easily charged into the mold, and after charging, the magnet is released, and the mold is released, so as to complete molding conveniently. In addition, maintenance of the shape can be attained.

That is, in the case where a magnet is combined with the flowing characteristics of the heat-generating composition, molding, which is constituted with shape formation and shape maintenance, can be easily carried out, whereby a heat-generating body having an arbitrarily shape forming property and excellent heat-generating characteristics can be obtained.

In the case where powder is used as the heat-generating composition, deviation of the heat-generating composition upon use has been prevented, for example, by adjusting the air permeability of an air permeable part of an inner bag to adjust the decompression degree inside the inner bag, as described in JP-UM-B-H5-30432. The heat-generating body produced from the heat-generating composition of the invention is prevented from deviation of the heat-generating composition before, during and after use even when any type inner bag is used to maintain the prescribed position of the heat-generating composition owing to the excellent shape maintenance property of the heat-generating composition of the invention, and thus a flat shape of the bag can be maintained during the period where it is applied to the application position of the body or the like irrespective to the presence or absence of heat generation. Furthermore, the range of options for the air permeable packing materials is broadened to provide large cost reduction.

Since the composition of the invention realizes improvement in productivity, such as reduction in unevenness of quality upon production, the molded body has excellent heat generating characteristics with a high initial stage reaction rate and continuous stability, and exhibits good adhesion to the base material, the covering material and the laying material. Thus, even when the heat-generating body is moved upon use, needless to say upon storing and transporting, cracking is hard to form in the heat-generating body itself, and even when cracking occurs, it is not largely broken, and it is good in air instreaming and air contacting, and proceeds the reaction in a totally uniform and effective manner, whereby the heat-generating characteristics can be sufficiently exerted.

The heat-generating composition expresses a deoxygenation function and an antifungal function, in addition to the heating function, and thus it may also be used as a functional composition or a functional body having various kinds of functions, such as a deoxygenating agent, an antifungal agent and the like.

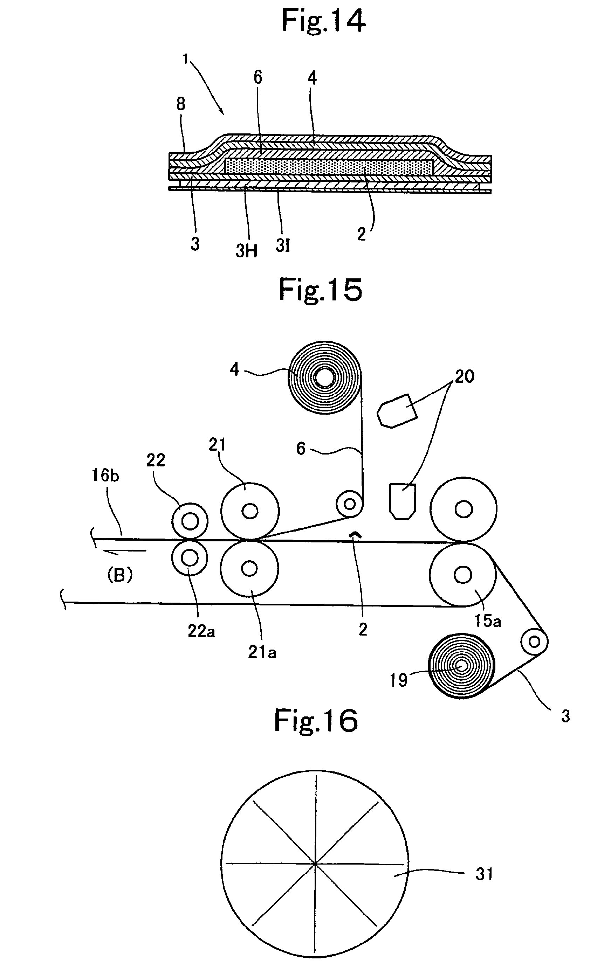

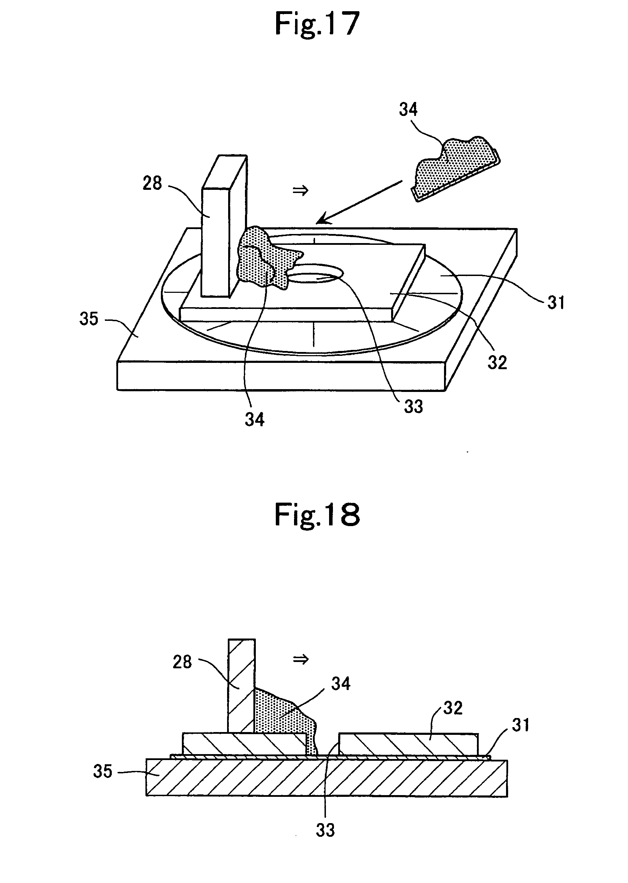

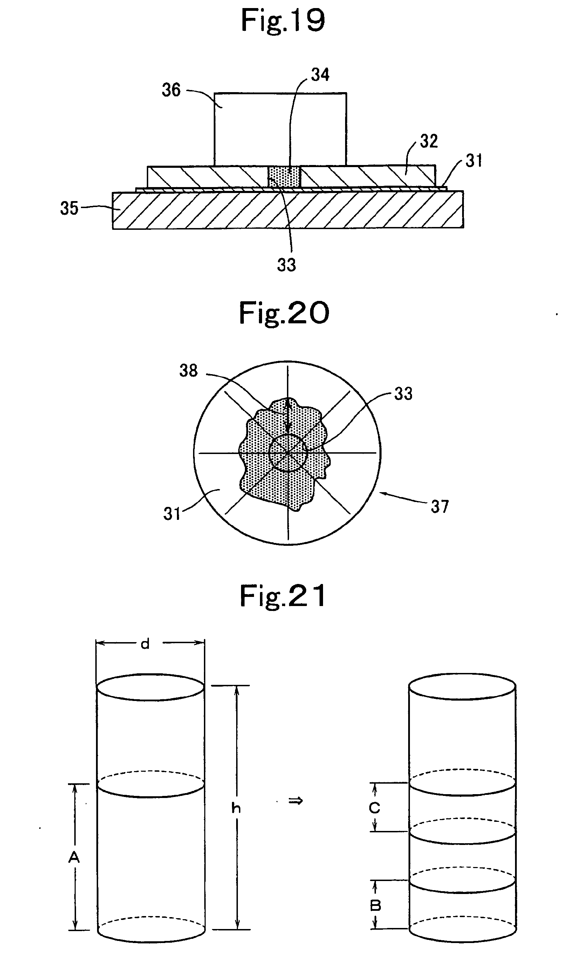

The water mobility value of the heat-generating composition of the invention is a value showing an amount of excessive water that can be moved outside the composition among the water content of the heat-generating composition. The water mobility value will be described with reference to Figs. 16 to 20. As shown in Fig. 16, No. 2 filter paper 31, on which eight lines extending from the center with intervals of a 45 degree have been written, is placed on a stainless steel plate 35 as shown in Figs. 17 and 18, and a template 32 having a hole 33 of a hollow cylinder shape having an inner diameter 200 mm and a height of 4 mm is placed on the center of the filter paper 31. A sample 34 is placed in the vicinity of the hole 33 having a hollow cylinder shape, and a pressing plate 28 is moved along the template 32, so as to place the sample 34 into the hole 33 having a hollow cylinder shape with pressing (press molding). Furthermore, as shown in Fig. 19, the hole 33 of a hollow cylinder shape having the sample 34 therein and the periphery thereof are covered with a wind guard 36, and they are maintained for 5 minutes. Thereafter, the filter paper 37 is brought out (Fig. 20) , and the excursion of soaking of water or an aqueous solution is read as distances 38 from the circumference part, which is an edge of the hole 33, to the front edge of soaking along the radial line in terms of mm units. The distances 38 along the respective lines are read to obtain eight values in total. The thus-read eight values are designated as measured water content values (a, b, c, d, e, f, g and h).

An arithmetic average of the eight measured water content values is designated as a water content value (mm) of the sample.

A water content value for measuring the true water content value is a blended water amount of the heat-generating composition corresponding to the weight of the heat-generating composition having a diameter of 20 mm and a height of 4 mm, and the measurement is carried out by using only water corresponding to the water amount to obtain a true water content value (mm) through the similar calculation. A value obtained by dividing the water content value by the true water content value is multiplied with 100 to obtain the water mobility value.

That is, water mobility value = (water content value (mm)/true water content value (mm)) x 100. The water mobility value herein is a value upon lamination, for example, by pressing molding or the like.

The water mobility value (0 to 100) of the heat-generating composition of the invention is generally from 7 to 40, preferably from 7 to 35, and more preferably from 7 to 30. In the case where it is less than 7, when the composition is laminated on a base material through a mold, it cannot be laminated due to poor flowability, and in the case where it exceeds 40, the composition runs off the mold shape to fail to maintain the shape.

The separation degree of the heat-generating composition of the invention is a value showing an extent of separation of the composition obtained from the rate of change of the water mobility value after allowing the heat-generating composition to stand for 5 minutes. The separation degree will be described with reference to Fig. 21. As shown in Fig. 21, a sample having a water mobility value S is charged in a testing cup having an inner diameter (d) of 110 mm and a height (h) of 250 mm to a sample height of 150 mm (A) with the bottom of the testing cup being 0 mm, and after allowing to stand for 5 minutes, the sample (lower 50) present at a height of from 0 to 50 mm (B) and the sample (upper 50) present at a height of from 100 to 150 mm (C) are brought out. The samples are agitated respectively for 2 minutes, the water mobility values of the samples are measured. The lower 50 water mobility value (water mobility value of the sample present at a height of from 0 to 50 mm after testing) and the upper 50 water mobility value (water mobility value of the sample present at a height of from 100 to 150 mm after testing) are obtained. Thereafter, a separation degree (T) is calculated by the following equation.

$$T = 100 \times (W-L)/S$$

wherein T represents the separation degree, L represents the lower 50 water mobility value (watermobility value of the sample present at a height of from 0 to 50 mmafter testing) , W represents the upper 50 water mobility value (water mobility value of the sample present at a height of from 100 to 150 mm after testing) , and S represents the water mobility value of the sample before testing.

The separation degree of the heat-generating composition of the invention is generally from 0 to 30, preferably from 0 to 20, more preferably from 0 to 15, and further preferably from 0 to 10. When it exceeds 30, unevenness is liable to occur in the components of the heat-generating composition, whereby a problem occurs in stability of the quality, and the dispersion state is difficult to be maintained in a standing state upon operation trouble, to cause a problem on operation. In any cases, a problem is liable to occur in quality.

The incremental degree of viscosity shows a difference between a BH type viscosity (BH type) S of the heat-generating composition containing the heat-generating substance, the carbon component, the oxidation accelerator and water, and a BH type viscosity (BH type) T of a heat-generating composition obtained by adding other substances thereto, and the value T-S is generally less than 1,000 cP (centipoise), preferably less than 500 cP, and more preferably less than 300 cP, which includes 0 and a negative value. There is no limitation in the negative value, and thus the viscosity may be decreased by any extent. As the BH type viscosity, such a value is employed that is obtained by placing a No.#7 rotor in a center of a sample to obtain a value in a stable state after lapsing 5 minutes or more from the start of rotation. A BH type viscometer (BH type) with a rotor of No.#7 at 2 rpm has a full scale of 200,000 cP.

In the case where the value T-S is 1, 000 cP or more, adverse influences occur in heat-generating characteristics, such as considerable deterioration in heat-generating property.

The heat-generating substance, the carbon component, the oxidation accelerator and the water used in the invention have no particular limitation as far as they are those used in an ordinary chemical body warmer.

Examples of additives for improvements of water permeability, flowability, dispersibility, releasing property, shape maintenance property, adhesion to the base material, addition of functions and the like without increase of viscosity include a water retainig agent, a pH adjusting agent, a surfactant, a defoaming agent, a hydrophobic polymer compound, a pyroelectric substance, an antioxidant, an aggregate and a heat-generating assistant. Those that are used in an ordinary chemical body warmer can be similarly used.

In addition to the heat-generating substance, the carbon component, the oxidation accelerator and the water, which are the essential components of the composition of the invention, those generally referred to as a water absorbing polymer, a binder, a thickening agent, an excipient, an aggregation assistant, a soluble adhesive material and the like may be blended as far as they have an incremental degree of viscosity in the foregoing range.

The mixing ratio of the heat-generating composition of the invention may be any value as far as the molding property and the heat-generating property can be maintained while it varies depending on the kind of the water separation-preventing stabilizer, the heat-generating substance, the kind of the carbon component, the kind of the oxidation accelerator and the like.

In general, it contains from 1 to 40 parts by mass of the carbon component, from 0. 2 to 30 parts by mass of the oxidation accelerator and from 0.001 to 0.25 parts by mass of the water separation-preventing stabilizer per 100 parts by mass of the heat-generating composition, and a heat-generating composition having good water permeability and excellent shape maintenance property can be formed by making a water mobility value of from 7 to 40. In order to obtain a water mobility value of from 7 to 40, the amount of the components, such as a water absorbing agent and the like, is fixed at a target of from 10 to 100 parts by mass while it varies depending on the water absorbing capability. Furthermore, it is preferred that the carbon component is from 1.0 to 20 parts by mass, and preferably from 1.5 to 15 parts by mass, the oxidation accelerator is from 0.3 to 15 parts by mass, and more preferably from 0.5 to 10 parts by mass, and the water separation-preventing stabilizer is from 0.001 to 0:1 part by mass, preferably from 0.01 to 0.1 part by mass, more preferably from 0.01 to 0.05 part by mass, and further preferably from 0.02 to 0.05 part by mass, per 100 parts bymass of iron powder, and it is preferred that the water mobility value is from 15 to 40, and preferably from 20 to 40. Such a heat-generating composition can be formed that is excellent in dispersibility and dispersion stability, has good water permeability, and is excellent in shape maintenance property in total.

Furthermore, other components may be mixed with the heat-generating composition, and the mixing ratios thereof are not particularly limited as far as the dispersibility, the dispersion stability, the water permeability and the shape maintenance property are maintained. For example, from 0.01 to 20 parts by mass of a water absorbing agent, from 0.01 to 20 parts by mass of a water retainig agent, from 0.01 to 10 parts by mass of a pH adjusting agent, from 0.01 to 10 parts by mass of a surfactant, from 0.01 to 10 parts by mass of a defoaming agent, from 0.05 to 20 parts by mass, and preferably from 0.05 to 10 parts by mass, of each of a far infrared radiating substance, a negative ion generating substance and a pyroelectric substance, from 0.01 to 20 parts bymass of a fibrous material, from 0.01 to 10 parts by mass of a hydrophobic polymer compound, and from 0.01 to 0.5 part by mass of each of a binder, a thickening agent, an excipient, an aggregation assistant, a soluble adhesive material and a water absorbing polymer may be added to 100 parts by mass of the heat-generating substance. At least one kind selected from thesemaybemixed. In particular, the mixing ratio of the fibrous material is preferably from 0.01 to 10 parts by mass, more preferably from 0.01 to 3 parts by mass, and further preferably from 0.05 to 1 part by mass.

Any mixing method may be employed as far as a heat-generating composition having good dispersion property and dispersion stability of the components can be obtained, and examples thereof include a method of uniformly mixing the solid components, and then mixing water or an aqueous solution or dispersion of the oxidation accelerator, a method of adding a suitable amount of water or an aqueous solution or dispersion of the oxidation accelerator to the solid components, and then uniformly mixing the total components, and the like.

In particular, in the case where the mixing ratio of the water separation-preventing stabilizer is as too small as less than 0.001% by mass, the uniformity in components of the molded body is deteriorated, whereby the releasing property from a mold is deteriorated to break the molded body or to cause cracking and breakage in the heat-generating body itself, and adhesion of the molded body to the base material, the covering material and the laying material is deteriorated, so as to fail to the heat-generating performance in a sufficient manner. In the case where it exceeds 0.25 part by mass, on the other hand, the heat-generating performance is considerably deteriorated, and correlating therewith, heat generation for practical use is difficult to obtain when the incremental degree of viscosity is 1,000 cP or more. Furthermore, adhesion to equipments is liable to occur to cause considerable contamination of the environments, and the desired heat-generating temperature and heat-generating time are difficult to be ensured. Even when water or the aqueous solution is added in a relatively large amount, the drainage property of excessive water from the mixed heat-generating composition is poor, whereby the most of the water separation-preventing stabilizer remains in the heat-generating composition to deteriorate the heat-generating performance to a significant extent.

The water separation-preventing stabilizer may.be added to the solid components, may be added as an aqueous solution, or may be added to an aqueous solution or dispersion of the oxidation accelerator.

In the case where the mixing ratio of the fibrous composition is as too small as less than 0.01 part by mass, the shape maintenance property, particularly the impact resistance, of the molded body becomes insufficient to make the bolded body being liable to be broken, and in the case where it exceeds 20 parts by mass, the molding property is deteriorated, and the absolute amount of the heat-generating substance becomes insufficient, whereby there is such a possibility that the desired heat-generating temperature and heat-generating time are difficult to be ensured.

In addition, upon inserting the heat-generating composition of the invention between the base material and the covering material, it is possible that at least one kind selected from iron powder, a carbon component, a water absorbing agent, a water absorbing polymer, a binder, a thickening agent and an aggregating assistant is laminated or diffused on one surface or both surfaces of the heat-generating composition, whereby the rising property of the heat-generating temperature upon use is increased, or the temperature characteristics upon use are changed. In this case, the amount of lamination or diffusion is not particularly limited as far as the temperature characteristics are not deteriorated, and it is generally preferable in a range of from 1 to 300 g/m$^2$. Examples of the water absorbing agent include pulp, cotton, paper, a volcanic ash substance, a water retainig agent and a water absorbing polymer.

As the iron powder herein, iron powder coated with a carbon component and a mixture obtained by adding water to iron powder (A) and a carbon component (B) in an amount of 5% by mass or less based on the total amount of (A) and (B) may be used.

Anymaterial that generates heat upon reaction with oxygen can be used as the heat-generating substance, and a metal is generally used. For example, iron powder, zinc powder, aluminumpowder, magnesiumpowder, powder of an alloy containing at least one of these metals, mixed metal powder containing at least one of them are used, and among these in particular, iron powder is preferably used because it is excellent in total standpoint including safety, handling property, cost, storage property, stability and the like. As the iron powder, cast iron powder, atomized iron powder, electrolytic iron powder, reduced iron powder and the like may be used. Iron powder containing carbon is also useful.

In particular, iron powder having from 0.3 to 3.0% by mass of an electroconductive carbonaceous substance coated partially on the surface of the iron powder is useful. Examples of the electroconductive carbonaceous substance include carbon black, activated carbon and the like, and examples of the iron powder include reduced iron powder, atomized iron powder, sponge iron powder. In particular, the case where the electroconductive carbonaceous substance is activated carbon, and the iron powder is reduced iron powder is useful as a chemical body warmer.

Examples of a method for coating the carbon component on the iron powder in this case include such a method that a cathode thin film is formed by a coating treatment for from 30 minutes to 3 hours in a ball mill, a conical blender or the like. Specific examples thereof include such a method that from 0.1 to 10 parts by mass of the carbon component is used per 100 parts by mass of the iron powder, which are kneaded in an extruding mixer (AM-15F, produced by Hosokawamicron Corp., or the like) at a rotation number of from 500 to 1,500 rpm for from 10 to 80 minutes.

It is also possible that the iron powder, the carbon component and water or salt water are mixed and extruded with a screw or the like capable of extruding, and then mixing and extruding are carried out after adding other components, such as the water retainig agent and the like, to obtain a generating agent using iron powder having been treated with the carbon component.

Furthermore, in order to prevent generation of a gas, i.e., a hydrogen gas, on an oxidation reaction in the presence of iron powder, particularly untreated iron powder, or an acidic salt, such iron powder may be used in the invention that is treated with an alkali salt of a weak acid, such as sodium hydroxide, potassium hydroxide, sodium hydrogencarbonate, sodium carbonate, calcium hydroxide, calcium carbonate and sodium propionate, followed by drying in some cases. Iron powder treated with a sulfate and/or a sulfite may be used in the invention for preventing generation of hydrogen.

Examples of the carbon component include carbon black, graphite, activated carbon and the like. Activated carbon prepared from husks of coconuts, wood, charcoal, coal, bone charcoal or the like is useful, and those prepared from other raw materials, such as an animal originated substance, a natural gas, a fat, an oil and a resin, are also useful in the heat-generating composition of the invention. The species of the activated carbon is not limited, and activated carbon exerting excellent absorption maintenance property is preferred. The performance of the carbon component is preferably an iodine absorbing capability of from 500 to 1,200 mg/g and a methylene blue decoloring capability of from 50 to 300 mg/g, and more preferably an iodine absorbing capability of from 800 to 1,200 mg/g and a methylene blue decoloring capability of from 100 to 300 mg/g. A mixture of carbon may be used in the invention.

The carbon component in the heat-generating composition of the invention may have any particle size as far as molding can be carried out, and a carbon component having a particle diameter of from 150 to 600 μm is preferably contained in an amount of from 2 to 85% by mass.

The organic water retainig agent in the heat-generating composition of the invention may have any particle size as far as molding can be carried out, and an organic water retainig agent having a particle diameter of 1, 000 μm or less is preferably contained in an amount of 50% by mass or more, more preferably that having a particle diameter of 150 μm or less is contained in an amount of 50% by mass or more, and further preferably that having a particle diameter of 150 μm or less is contained in an amount of 65% by mass or more.

The organic fibrous material is not particularly limited in size thereof, and in general, that having a width of 10 mm or less and a length of 25 mm or less, more preferably that having a width of 5 mm or less and a length of 25 mm or less, and further preferably that having a width of 0.5 mm or less and a length of from 0.2 to 10 mm, are employed.

In the case of fibrous powder, the particle diameter thereof is preferably 500 μm or less. When it exceeds 500 μm, it is not preferred since the dispersibility is deteriorated, and the affinity with the other components is lowered, and it is desirably in a range of from 0.1 to 350 μm from these standpoints.

In the case of a fibrous material in an inorganic fibrous material or an organic fibrous material, those having a fiber diameter of 300 μm or less, particularly from 0.5 to 150 μm, and an aspect ratio (fiber length/fiber diameter) of from 2 to 10,000 are desired from the standpoints of dispersibility, handling property, productivity and the like.

The organic fibrous material is preferably those obtained by making powder or short fibers formed with a synthetic resin or a synthetic fiber into fibrils having a fiber diameter of 1 μm or less under a strong shearing force.

That is, the fibrousmaterial is forcedlymade into fibrils, and the fibrils of the fibrous material are blended and mixed or kneaded with water or an aqueous solution, whereby the fibrous material becomes an ultrafine fibril form having an extremely fine form to become fibers through entanglement with each other. The functional composition is entangled with the ultrafine fibers to provide such a state that gaps among the flocculent fibers are filled with the heat-generating composition.

As a result, the state where the fibrous material is made into a fiber form becomes dense and is considerably good in molding property, whereby the heat-generating material can be molded into a sheet form, formation of dusts due to the form of powder can be prevented, and the mixing ratio of the fibrous material may be considerably small.

The fibrous material includes that previously formed into fibrils and that is to be formed into fibrils. In the case where that is to be formed into fibrils, an inorganic fibrous material and an organic fibrous material that are easily made into fibrils are preferred since it is simply mixed and uniformly mixed, followed by kneading under shearing force, or kneading, for example, under a roll pressure, whereby the fibrous material can be easily and certainly made into fibrils. The easiness in making into fibrils depends on the crystallinity of fibrils in the filaments.

Fibrils are formed through such a phenomenon that continuous long filaments rapidly receive a large tensile stress or shearing stress to break in a longitudinal direction to cause small fibers having a diameter of about 1 μm, and among the sheeting agents, those that are easily formed into fibrils are the most appropriate.

Specific examples of the inorganic fibrous material and the organic fibrous material that are easily formed into fibrils include asbestos, fibrous carboxymethyl cellulose, cellulose, a cellulose derivative including viscose rayon and acetate fibers, biofibers including bacterial cellulose and the like, a polyethylene fluoride including a polytetrafluoroethylene resin and the like, and the like.

Examples of the bacterial cellulose include those disclosed in JP-A-S59-120159, JP-A-S60-79291, JP-A-S63-199201 and JP-A-H6-341093.

In this case, a fibrous material that is easily formed into fibrils is more preferred. That is, the following has been found. In the case where the fibrous material is one easily formed into fibrils or one which has been formed into fibrils, the fibrous material is mixed, and water or an aqueous solution in an amount exceeding the necessary amount with a controlled excessive amount is mixed or kneaded therewith, whereby the fibrous material becomes extremely fine ultramicro filaments, and fibers are formed through entanglement thereof. The heat-generating composition is entangled with the extremely fine fibers to form such a state that gaps among the flocculent fibers are filled with the heat-generating composition. Because the state where the fibrous material is formed into fibers is dense, the heat-generating composition can be molded, and formation of dusts due to the form of powder can be prevented. Moreover, the mixing ratio of the fibrous material may be extremely small.

The solid contents other than the carbon component, the organic water retainig agent and the fibrous material may have any size as far as molding can be carried out, and the size thereof is preferably 600 μm or less in an amount of 70% by mass or more, and more preferably 200 μm or less in an amount of 50% by mass or more, more preferably 80% by mass or more, and most preferably 90% by mass or more, with an average particle size being 150 μm or less. According to the configuration, a sherbet form heat-generating composition having good drainage property and excellent molding property can be formed.

In the case where the solid contents contains those having particle diameter of 200 μm or more in an amount of 50% by mass or more, the printing property and the shape maintenance property are deteriorated.

The oxidation accelerator may be any material that can accelerate oxidation of the heat-generating substance. Examples thereof include a metallic halogenide, such as sodium chloride, potassium chloride, magnesium chloride, calcium chloride, ferrous chloride, ferric chloride, cupric chloride, manganese chloride, cuprous chloride and the like, a metallic sulfate, such as potassium sulfate, sodium sulfate, magnesium sulfate, calcium sulfate, copper sulfate, ferrous sulfate, ferric sulfate and manganese sulfate, a nitrate, such as sodium nitrate, potassium nitrate and the like, and an acetate, such as sodium acetate and the like. Carbonates and other salts of metals other than the foregoing may also be used. These may be used solely or combination.

The water may be one from a suitable source. The purity and the kind thereof are not limited.

The water separation-preventing stabilizer may be any material as far as they disperse the components of the heat-generating composition, and separation of water is prevented, so as to maintain stable dispersion, including inorganic materials and organic materials. Examples thereof include a water soluble polymer (those having at least one kind of an OH group, a carboxyl group and a sulfone group, and the like), a saccharide (a monosaccharide, an oligosaccharide and a polysaccharide), a coagulating agent, a water dispersed emulsion and the like, and those having affinity with water are preferred.

As the water separation-preventing stabilizer thus added, one kind thereof may be added, and plural kinds thereof may be added simultaneously or separately.

The mixing method of the water separation-preventing stabilizer may be any method, and it is preferably mixed in at least one form selected from a solid, an aqueous solution and an emulsion.

Examples of the water soluble polymer include a natural polymer, a semisynthetic product and a synthetic product.

Examples of the natural polymer include a starch series (a starch derivative) , a syrup series, amannan series, a seaweed series, a vegetable mucic matter, a mucic matter from microorganisms, a protein series, a polysaccharide series and the like, and specific examples thereof include raw starch, such as nard starch, potato starch, Japanese potato starch, tapioca starch, cone starch, wheat starch, rice starch and the like, modified starch, such as dextrin, baked dextrin, enzyme-modified dextrin, cyclodextrin, dialdehyde starch, gelatinized starch, modified starch, oxidized starch, esterified starch, etherified starch, cationated starch, crosslinked starch and the like, α-starch, hydroxypropyl starch, British rubber, cone syrup, crystalline sorbitol syrup, noncrystalline sorbitol syrup and a mixture thereof, mannan paste, laver, agar (galactan), alginic acid, sodium alginate, alginic acid propylene glycol ester, carrageenan, yam mallow, tragant rubber (gum), gum arabic, locust, bean gum, cyamoposis gum, queen's seed gum, tamarind seed gum, meskid gum, gutch gum, cherry gum, arabinogalactan, dextran, levan, glue, gelatin, casein, collagen, albumin, pectin, xanthan gum, pullulan, chitosan, ureylane gum, chitin and a derivative thereof (such as sodium casein and the like), sucrose (sugar), arginic acid, an arginate, such as sodium arginate and the like, carrageenan, molasses, grape sugar, glucose, sorbitol and the like.

Examples of the semisynthetic product include a cellulose series and a starch series, and examples of the cellulose series include viscose, methyl cellulose (MC), ethyl cellulose (EC), hydroxyethyl cellylose (HEC), carboxymethyl cellulose (CMC), sodium carboxymethyl cellulose, carboxymethylethyl cellulose (CMEC), ethyl cellulose acetate, hydroxypropyl cellulose (HPC), hydroxypropylmethylcellulose, ethyl cellulose, cationated gum, cellulose ether and the like.

Examples of the starch series include soluble starch, carboxymethyl starch (CMS), carboxylated starch, cationated starch and the like.

The synthetic product include polyvinyl alcohol (Poval), a heterocyclic compound, such as polyvinylpyrrolidone, polyvinylpyridine and the like, polyethyeleneimine, a polycarboxylic acid (such as poly(meth)acrylic acid, polyitaconic acid and the like), a copolymer and a salt (an alkali metal salt, such as sodium, potassium and the like, a water soluble metallic salt formed by substituting the entire or a part of carboxyl groups with an alkaline earth metal, such as calcium, magnesium, aluminum and the like, an ammonium salt and the like) thereof, a polyoxyalkylene, such as polyethyleneoxide, polyethylene glycol, polyvinyl methylene ether and the like, a polymaleic acid copolymer, a methoxyethylene-maleic anhydride copolymer, an isobutylene-maleic anhydride copolymer, polyethyelneimine, a (meth)acrylamide (co)polymer, sodium polyvinylsulfonte, a stearate, a urea-melamine resin, polyvinyl acetate, a partially saponified product of polyvinyl acetate, polyvinyl acetal, polyurethane, water soluble urethane, an acrylsulfonic acid series polymer substance, poly-N-vinylacetamide, a water soluble epoxy resin, such as a cyanoacrylate compound and the like, and a salt (an alkali metal salt, such as sodium, potassium and the like, a water soluble metallic salt formed by substituting the entire or a part of carboxyl groups with an alkaline earth metal, such as calcium, magnesium, aluminum and the like, an ammonium salt and the like) and a derivative thereof, glycerin and the like.

Examples thereof also include mixtures of two or more kinds thereof.

The water dispersed emulsion may be any material as far as it is in an emulsion form and has particle binding property, and in general, those obtained by forming a polymer compound to be an adhesive agent (adhesive agent) into an emulsion are used.

Examples thereof include an aqueous adhesive agent, tackifier emulsion and the like.

Examples of the aqueous adhesive agent include an adhesive emulsion formed by adding a tackifier (adhesion imparting resin) emulsion, an antioxidant emulsion, a thickening agent emulsion or the like to an aqueous emulsion resin.

Examples of the aqueous emulsion resin include an emulsion of an acrylic polymer containing, as a major component, acrylic acid, an acrylate, a methacrylate (hereinafter referred to as (meth)acrylic acid), examples of a monomer of which include methyl (meth)acrylate, ethyl (meth)acrylate, butyl (meth)acrylate, 2-ethylhexyl (meth)acrylate, glycidyl (meth)acrylate, 2-hydroxyethyl (meth)acrylate, a long chain alkyl (meth)acrylate (examples of which include those having an alkyl group having from 9 to 13 carbon atoms, such as nonyl, isononyl, decyl, isodecyl, dodecyl, isododecyl, tridecyl, isotridecyl and the like) and the like.

Furthermore, examples thereof include a vinyl acetate series emulsion, such as polyvinyl acetate emulsion, a vinyl acetate-ethylene series emulsion, which is a derivative thereof, such as a vinyl acetate-ethylene copolymer (EVAC) and the like, a vinyl acetate-acrylate ester series emulsion, such as a vinyl acetate-acrylate copolymer emulsion and the like, a vinyl acetate-vinyl ester series emulsion, such as a vinyl acetate-vinyl ester copolymer emulsion and the like, a vinyl acetate-vinyl ether series emulsion, such as a vinyl acetate-vinyl ether copolymer emulsion and the like, and examples thereof also include a urethane series emulsion, such as polyurethane emulsion and the like, a styrene resin series emulsion, such as a styrene-butadiene copolymer emulsion and the like, a curable vinyl acetate emulsion, an epoxy resin series emulsion, and a rubber latex, such as natural rubber, synthetic rubber and the like.

Examples of the synthetic rubber latex include a nitrile rubber latex, a polybutadiene latex, a styrene-butadiene latex, a chloroprene rubber latex, a styrene-isoprene rubber latex and the like.

The copolymer may have other components copolymerized.

Examples of the other copolymerization components include an alkyl (meth)acrylate having 14 or more carbon atoms, a vinyl ester compound, such as vinyl acetate, an aromatic vinyl compound, such as styrene, a nitrile monomer, such as acrylonitrile and the like, an amide monomer, such as acrylamide and the like, (meth)acrylic acid, and a carboxylic acid monomer, such as maleic acid, maleic anhydride and the like.

Examples of the tackifier emulsion include a rosin series resin emulsion, a terpene series resin emulsion, a phenol series resin emulsion, an alkylphenol series resin emulsion, a coumarone series resin emulsion, a petroleum resin emulsion and the like.

Examples of the rosin compound include gum rosin, wood rosin, tall oil rosin, heterogeneous rosin, hydrogenerated rosin, polymerized rosin and the like, as well as a rosin derivative thereof, such as a rosin ester, a rosin-modified maleic acid resin, an ester type rosin-modified maleic acid resin, a rosin-acrylic acid copolymer and the like.

Examples thereof include one selected therefrom or a combination of two or more thereof. Furthermore, a dispersing agent or a gelation stabilizer may be combined therewith.

In the case where the water disperses emulsion, the mixing amount thereof is determined in terms of the solid content.

Examples of the dispersing agent include a surfactant and the like.

Examples of the gelation stabilizer include a synthetic alkaline earth metal salt of silicon (such as a magnesium salt and a calcium salt), a glycoside derived from plants, such as saponin, acrylamide, polyacrylate (such as a sodium salt and a potassium salt), natural rubber and the like. Breakage of the composition due to water separation can be prevented for a long period of time by adding the gelation stabilizer.

A coating composition, an adhesive (adhesive) agent and the like containing them may also be similarly used.

The coagulating agent may be any material that has a coagulating function, and as an anionic coagulating agent, sodium polyacrylate, a copolymer of acrylamide and sodium acrylate, a partial hydrolysis product of polyacrylamide and the like may be used. Examples of a cationic coagulating agent include polyvinylimidazoline, polyalkylamino (meth)acrylate, a mannich modified product of polyacrylamide, and examples of a nonionic coagulating agent include polyacrylamide, polyethyelenoxide and the like.

The gelation stabilizer preferably has a number average molecular weight of 100,000 or more, and more preferably from 100,000 to 1, 000, 000 since the viscosity is increased when it is too high.

As the water retainig agent, examples of an organic water retainig agent include wood powder, pulp powder, activated carbon, sawdusts, cotton cloth having a large amount of cotton wool, short fibers of cotton, paper dusts, a plant material, a plant porous material having a capillary function and hydrophilicity, and the like. Examples of an inorganic water retainig agent include activated clay, a silicon-containing magnesium clay mineral, such as zeolite and the like, perlite, cristobalite, vermiculite, a silica series porous substance, a coral substance, silica powder, a silicate, such as calcium silicate and the like, silica rock, diatom earth, alumina, a silicic acid substance, such as mica powder, clay and the like, a magnesia silicate substance, such as talc and the like, silica powder, oplite, a volcanic ash series substance (such as terraballoon (a minute hollow foamed body having independent bubbles formed by rapidly cooling volcanic glass), Shirasu balloon, Taisetsu balloon) and the like. A raw material of Shirasu balloon is a volcanoic product, such as falling floatstone containing floatstone tuff conglomerate, and in general, the texture thereof contains $SiO_2$ and $Al_2O_3$ (alumina silicate) , as well as a hydrous magnesium silicate clay mineral (hereinafter, referred to as a clay mineral) represented by zeolite containing FeO, $Fe_2O_3$, CaO, $Na_2O$, $K_2O$ and the like. For example, they include sepiolite, shirotairu, rafurinaito, farukondoaito, palygorskite containing magnesium-containing aluminum silicate as a major component, and the like, one or them or a mixture of two or more of them is used. Minerals commonly referred to as mountain cork, mountain wood, mountain leather, sea-foam, attapulgite, and the like correspond thereto.

Those having been treated, such as baking and/or pulverization or the like, may be used for increasing the water holding capability, increasing the shape maintenance property and the like of the organic water retainig agent and the inorganic water retainig agent.

In particular, the organic water retainig agent is useful since it has such a function of preventing plumping of the packing material (outer bag) having the heat-generating body charged therein.

The water absorbing polymer may be any polymer that smoothly absorbs water and an aqueous solution of a metallic chloride in a large amount. It may be those exerting binding property after absorbing water.

Examples thereof include one kind solely or a mixture of two or more kinds selected from an isobutyrene-maleic anhydride copolymer, a polyvinyl alcohol-acrylic salt copolymer, a starch-acrylic salt graft copolymer, a polyacrylic acid crosslinked product, an acrylic salt-acrylate copolymer, a polyacrylic salt-acrylamide copolymer, a hydrolysis product of a polyacrylonitrile crosslinked product, a starch-polyacrylonitrile copolymer, crosslinked polyalkyleneoxide, a saponified product of a vinyl ester-ethylenic unsaturated carboxylic acid copolymer, a self-crosslinked polyacrylic salt, a reaction product of a polyvinyl alcohol series polymer and a cyclic anhydride, a polyacrylic salt crosslinked product, an N-vinylacetamide crosslinked product and the like. Furthermore, they may be improved in hydrophilicity by treating with a surfactant or combining with a surfactant.

The water soluble polymer is not particularly limited as far as it is gelled upon absorbing water in an amount of twice its own weight, and in particular, a water absorbing polymer having controlled solubility in water by introducing crosslinking bonds. In particular, those having a water absorption capability of 50 times or more are preferred.

In the case where the heat-generating body is stored with a airtight bag (outer bag) using the water absorption polymer that does not contain a cyclic anhydride and an acid or a salt derived therefrom, plumping of the airtight bag becomes considerably small, and the commercial value of the heat-generating body is considerably improved.

The hydrophobic polymer compound may be any polymer compound that has a contact angle with water of 40° or more, more preferably 50° or more, and further preferably 60° or more, for improving water drainage of the composition. The shape thereof is not particularly limited, and examples thereof include a powder form, particulate form, a granular form, a tablet form and the like, however, with a powder form, a particulate form and a granular form is preferred.

Examples thereof include a polyolefin, such as polyethylene, polypropylene and the like, a polyester, such as polyethylene terephthalate and the like, a polyamide, such as nylon and the like,polyvinylidene chloride, polyvinyl chloride, polystyrene, a fluorine resin, such as polytetrafluoroethylene, polytrifluoroethylene, polychlorotrifluoroethylene and the like, and an acrylic resin, such as polymethyl methacrylate, polymethylacrylate and the like.

The organic silicon compound may be any material including a monomer, a low condensate, a polymer and the like, as far as it is a compound having a bond of Si-O-R and/or Si-N-R and/or Si-R', and examples thereof include an organic silane compound such as a methyltrialkoxysilane including methyltriethoxysilane and the like, a tetraalkoxysilane including tetraethoxysilane and the like, a polyorganosiloxane (polysiloxane resin) such as a dimethylsilicone oil, a diphenylsilicone oil, a cyclic siloxane such as hexaorganocyclotrisiloxane, a silicone resin composition containing the same, and the like.

Examples of a group represented by R and R' include an alkyl group such as a methyl group, an ethyl group, a propyl group, a butyl group, a hexyl group, an octyl group, a decyl group, a dodecyl group, an octadecyl group, a vinyl group, an allyl group, a propenyl group, a butenyl group, a butadienyl group, an aryl group such as a hexadienyl group, an aralkyl group such as a benzyl group, a phenylethyl group, and a styryl group, a cycloalkyl group, and a group obtained by substituting a hydrogen atom of these groups with another group, such as a halogen atom or a hydroxyl group, an amino group, an alkoxy group, an acetoxy group, an oxime group, an aminoxyl group, an amide group and the like, which have 8 or less carbon atoms.

Upon providing on the surface of the molded body, water content can be prevented from scattering from the molded body.

Examples of the pH adjusting agent include a weak acid salt or a hydroxide of an alkali metal, a weak acid salt or a hydroxide of an alkaline earth metal, and the like, examples thereof include $Na_2CO_3$, $NaHCO_3$, $Na_3PO_4$, $Na_2HPO_4$, $Na_5P_3H_{10}$, $NaOH$, $KOH$, $CaCO_3$, $Ca(OH)_2$, $Mg(OH)_2$, $Ba(OH)_2$, $Ca_3(PO_4)_2$, $Ca(H_2PO_4)_2$ and the like.

The hydrogen generation suppressing agent may be any material that suppresses generation of hydrogen, and examples thereof include a metallic sulfide, such as calcium sulfide and the like, an oxidizing agent, an alkali substance, a material containing at least one kind solely or two or more kinds selected from sulfur, antimony, selenium, phosphorous and tellurium, and the foregoing pH adjusting agent. It is more effective by mixing it with the metallic powder as a heat-generating agent since the addition amount can be reduced.

Examples of the oxidizing agent include a nitrate, a nitrite, an oxide, a peroxide, a halogenated oxyacid salt, a permanganic acid salt, a chromic acid salt and the like, and examples thereof include $NaNO_3$, $KNO_3$, $NaNO_2$, $KNO_2$, $CuO$, $MnO_2$, $H_2O_2$, $NaClO$, $NaClO_3$, $NaClO_4$, $NaMnO_4$, $KMnO_4$, $Na_2ClO_4$, $K_2ClO_4$ and the like.

Examples of the alkali substance include a silicate, a borate, a dibasic phosphate, tribasic phosphate, a sulfite, a thiosulfate, a carbonate, a hydrogen carbonate, $Na_2SiO_3$, $Na_4SiO_4$, $NaBO_4$, $Na_2B_4O_7$, $KBO_2$, $Na_2HPO_4$, $Na_2SO_3$, $K_2SO_3$, $Na_2S_2O_3$, $Na_2CO_3$, $NaHCO_3$, $K_2S_2O_3$, $CaS_2O_3$, $Na_3PO_4$, $Na_5P_3O_{10}$ and the like.

In the case where the hydrogen generation suppressing agent is used in combination, examples thereof include a combination of an alkali weak acid salt and an alkali weak acid salt, such as $Na_2SO_3$-$Na_2SiO_3$, $Na_2SO_3$-$Na_2SiO_3$, $Na_2SO_3$-$Na_2B_4O_7$, $Na_2B_4O_7$-$Na_3PO_3$ and $Na_2CO_3$-$Na_2SO_3$, and a combination of an oxidizing agent and an alkali weak acid salt, such as $Na_2PO_4$-$Na_2SO_3$, $Na_5P_3O_{10}$-$Na_2SO_3$, $NaNO_2$-$Na_2SiO_3$, $NaNO_2$-$Na_2HPO_4$, $NaNO_2$-$Na_2SO_3$, $NaNO_2$-$Na_2S_3O_3$, $NaNO_3$-$Na_2SiO_3$, $NaNO_2$-$Na_2S_2O_3$, $NaNO_3$-$Na_3SiO_3$, $NaNO_3$-$Na_2HPO_4$, $NaNO_3$-$Na_2SO_3$, $NaNO_3$-$Na_2S2O_3$, $MnO_2$-$NaSiO_3$, $MnO_2$-$Na_2HPO_4$, $MnO_2$-$Na_2S_2O_3$, $NaClO$-$Na_2SiO_3$, $NaCl$-$Na_2HPO_4$, $NaClO$-$Na_2SO_3$, $KMnO_4$-$Na_2SiO_3$, $KMnO_4$-$Na_2HPO_4$, $KMnO_4$-$Na_2HPO_4$, $S$-$Na_2SO_3$, $S$-$Na_2S_2O_3$ and the like.

The using amount of the hydrogen generation suppressing agent in terms of a total amount of the respective hydrogen generation suppressing agents is preferably from 0.01 to 12.0% by mass, more preferably from 0.05 to 8% by mass, and further preferably from 0.5 to 2.0% by mass, based on the iron powder. When it is less than 0.01% by mass, the effect of suppressing generation of hydrogen is poor, and when it exceeds 12.0% by mass, it is not suitable since the heat-generation temperature is lowered while the effect of suppressing hydrogen generation is exerted.

The addition method is preferably addition in the form of an aqueous solution from the standpoint of workability and uniformity upon mixing, but even when it is added in a solid state separately from water, the effect of suppressing generation of hydrogen is not differ from the case of the aqueous solution.

The oxidizing agent, such as a peroxide, a halogenated oxyacid salt and the like causes catalytic cracking upon addition to the heat-generating agent, and thus the effect of suppressing hydrogen generation is effectively obtained when the iron powder is immersed in an aqueous solution of the oxidizing agent.

The nitrite and the nitrate causes generation of ammonia gas upon adding to the heat-generating agent. Therefore, ammonia odor can be easily eliminated by subjecting the iron powder to an immersing treatment with an aqueous solution of the nitrite or the nitrate, followedbyaneutralizingtreatment, and such an operation is preferred in comparison to the direct addition.

The surfactant includes anionic, cationic, nonionic and amphoteric surfactants. However, a nonionic surfactant is preferred.

Ethylene oxide, ethylene glycol, propylene oxide, propylene glycol and a polymer containing the same are also useful as an additive.

Examples of the nonionic surfactant include polyoxyethylene alkyl ether, an ethylene oxide adduct of ricinus, an ethylene oxide adduct of alkylphenol, such as an ethylene oxide adduct of nonylphenol or octylphenol, and the like, an ethylene oxide adduct of an intermediate alcohol or higher alcohol, a mono-, di-, tri- or tetra-fatty acid ester of a polyhydric alcohol, a polyoxyethylene polyhydric alcohol fatty acid ester or ether, a higher alcohol phosphate ester and the like.

Specific examples of other surfactants include sodium dodecylsulfate, sodium dodecylbenzenesulfonate, sodium caproate, sodium caprylate, sodium alkylnaphthalenesulfonate, sodium laurate, sodium oleate, a phosphate ester surfactant, a surfactant, such as a disodium salt of a higher alcohol phosphate mono ester, a sodium salt of a higher alcohol diphosphate and the like, a fatty acid and a metallic salt thereof, such as oleic acid, linoleic acid, lauric acid, palmitic acid, myristic acid, stearic acid and the like, a salt of a polycarboxylic acid having a low polymerization degree, such as sodiumpolyacrylate having a low polymerization degree, butyl polyacrylate having a low polymerization degree and sodium polymethacrylate having a low polymerization degree, a surfactant, such as sulfonated polystyrene and the like, and a silicone.

One kind solely or a mixture of two or more kinds among these may be used. A commercially available synthetic detergent containing these compounds may also be used.

Examples of the defoaming agent include an ordinary pH adjusting agent, such as sodium polyphosphate and the like, and other compounds that are used in this field of art are also used.

The far infrared radiating substance may be any substance that radiates a far infrared ray. Examples thereof include ceramics, alumina, zeolite, zirconium, silica and the like, and one kind solely or a mixture of two or more kinds among these may be used.

The negative ion generating substance may be any substance that directly or indirectly generates a negative ion as a result. Examples thereof include a pyroelectric substance, such as tourmaline, a substance containing a silicon element as a major component (such as a silicone resin, such as polydimethylsiloxane and the like, silicone rubber and the like) , granite, heruguron-stone, thorogummite, pegmatite (Mineon Health produced by Sumisho Plachem Co., Ltd. ) , $BaTiO_3$, $PbTiO_3$, $PbZrO_3$, $Pb(Zr,Ti)O_3$, $KNbO_3$, $KTaO_3$, $K(Ta,Nb)O_3$, $LiNbO_3$, Rochelle salt, glycin sulfate, potassium phosphate, a ferroelectric material, such as calcium strontium propionate and the like, negatively ionized Si, SiO2, an excitation agent and the like, and one kind solely or a mixture of two or more of them may be used. Moreover, a further effect is obtained by using a material having a hydroxyl group in combination or that maintained by the base material or the like.

Examples of the excitation agent (powder emitting a radiation ray) include a radioactive mineral containing a radioactive element, such as uranium, thorium and the like, i.e., devidite, brannerite, uraninite, nintyoite, autunite, carnotite, tuyamun-stone, meta-tuyamun-stone, francevillite, thorite, coffinite, samarskite, throamite, throgummite, moznite, feldspar, granite, sedimentary rock and the like.

It is also possible that powder of the excitation agent and tourmaline or the like are mixed to generate a negative ion through excitation of tourmaline.

In alternative, examples also include those containing, as a major component, uranium, thorium and ceramics such as $SiO_2$ and $Al_2O_3$ containing these radioactive elements.

Examples of the pyroelectric substance include dravite (X = Mg) , schorl (X = Fe, Mn) , elbaite (X = Li, Al) and tourmaline, such as rubellite, pink, baraiba, indecolite, water melon and the like, and one kind solely or a mixture of two or more of dravite, schorl, elbaite and the like may be used.

The aggregate may be any material that is useful for forming a porous body of the heat-generating composition, and examples thereof include activated white earth, activated carbon charcoal, bentonite, perlite, silica-alumina powder, silica-magnesia powder, calcined magnesia, kaolin, colloidal silica, attapulgite, floatstone, zeolite, magnesia powder, precipitated alumina powder, activated alumina, calcium carbonate, silica gel, cristobalite, vermiculite, silica series porous substance, a silicate such as calcium silicate, silica rock, diatomaceous earth, an aluminum oxide such as alumina, an aluminum oxide silicate substance such as mica powder, clay, a magnesia silicate substance such as talc, silica powder, a foamed synthetic resin, such as foamed polyester and polyurethane, wood powder, pulp powder, metasilicate, zirconium, ceramics, oplite, cement such as portland cement, magnesia cement, a silicate such as sodium silicate, potassium silicate, a phosphate such as zinc phosphate cement, aluminum phosphate, a sulfate such as gypsum, calcium triphosphate, sodium silicoaluminate, sodium sulfate, barium sulfate, an iron oxide, an inorganic balloon such as silicon series glass balloon, silica balloon, fly ash balloon, perlite, vermiculite, non-silicon series balloon of alumina, zirconia, carbon or the like, and the like.

Examples of the binder include sodium silicate, sodium alginate, a polyvinyl acetate emulsion and the like.

An organic fibrous substance (such as natural fibers including wool, cotton, linen and the like and various kinds of organic synthetic fibers) and lignocellulose having various kinds of shapes (such as a fiber form, a bar form, a powder form, a squamous form) may be mixed in the case where the solid content is 50% by mass or more.

Examples of the thickening agent include those generally used as a thickening agent, such as cone starch, $\alpha$-starch, bentonite.

Examples of the excipient include those generally used as an excipient such as sodium casein.

Examples of the aggregating assistant include those generally used as an excipient, such as cone syrup, mannitol syrup.

Examples of the soluble adhesive material include those generally used as an excipient such as polyvinylpyrrolidone.

Examples of the fertilizer component include a natural fertilizer, such as crushed bone, a mineral fertilizer, a chemical fertilizer, such as urea, ammonium sulfate, ammonium chloride, superphosphate of lime, concentrated superphosphate of lime, potassium chloride, potassium sulfate, calcium chloride, calcium sulfate, and one kind thereof solely or a mixed fertilizer obtained by mixing two or more kinds thereof may be used, with those suitably containing the three elements, i.e., nitrogen, phosphoric acid and potassium,being preferred. Moreover, those having charcoal, ash contents and the like, which have such effects as a growth controlling function of saprophytic bacteria, a neutralizing and improving function of soil property and the like function, may be used.

Examples of the heat-generating assistant include metallic powder, a metallic salt, a metallic oxide and the like, and examples thereof include Cu, Sn, Ni, Cr, Mn, $CuCl_2$, $FeCl_2$, $FeCl_3$, $CuSO_4$, $FeSO_4$, CuO, $MnO_2$, MgO, CaO, manganese dioxide, cupric oxide, triiron tetroxide, a compound containing these elements, a mixture thereof and the like.

Examples of the foaming agent include any material that can generate a gas to attain foaming. Examples thereof include a decomposition foaming substance, which is a single substance that generate a gas through decomposition by heating, a reaction foaming agent, which generates a gas through reaction of two or more substances, and the like. The decomposition foaming agent is not particularly limited, and an inorganic decomposition foaming agent is preferably used. Representative examples thereof include sodium bicarbonate, ammonium carbonate, ammonium bicarbonate and the like.

The reaction foaming agent is also not limited, and the following can be preferably used. That is, representative examples thereof include a combination of a carbonate, a bicarbonate or a light metal, such as magnesium, zinc, aluminum, with an acidic substance, such as sulfamic acid, citric acid.

Furthermore, examples also include a combination of a light metal such as magnesium, zinc, aluminum, silicon, with a basic substance such as sodiumhydroxide, potassiumhydroxide, calcium hydroxide, sodium carbonate. Moreover, for example, calcium carbide may be used, which generates an acetylene gas in the presence of water.

The reaction foaming agent foams without heating, but it may be heated.

Examples of the fibrous material include inorganic fibrous material and/or an organic fibrous material, and the like. Examples of the inorganic fibrous material include short fibers, such as rock wool, glass fibers, carbon fibers, asbestos fibers, boron fibers, alumina fibers, metallic fibers.

Examples of the organic fibrous material include powder and short fibers formed with polyamide, polyester, such as polyethylene terephthalate, polyurethane, polyvinyl alcohol, polycarbonate, polyvinyl acetate, fibrous carboxymethyl cellulose, cellulose, a cellulose derivative including viscose rayon and acetate fibers, bacterial cellulose, polyvinylidene chloride, polyvinyl chloride, an acrylic resin, polyethylene, polypropylene, a polyethylene fluoride including a polytetrafluoroethylene resin and the like, or an ethylene-vinyl acetate copolymer. An inorganic fibrous material and/or an organic fibrous material that are easily formed into fibrils can also be included. A binder may be attached to the fibrous material.

The fibrous material may be formed into fibrils before mixing or formed into fibrils during mixing, as far as it can be formed as a heat-generating body in such a condition that the heat-generating composition is well contact with the air under mixing with a water content more than that necessary for oxidation, and may be any of the inorganic fibrous material and the organic fibrous material without particular limitation.

A heat-generating composition having Japanese lacquer wear appearance can be obtained by using a component containing pulp, such as paper, or a component containing cellulose in an amount of 70% or more.

As the organic fibrous substance, pulp, paper, nonwoven fabric, fabric, natural fibers, synthetic fibers and a pulverized product thereof can be used, and examples thereof include at least one kind selected therefrom. The size thereof is not particularly limited, and those having a width of 10 mm or less, and a length of about 25 mm or less, more preferably those having a width of 5 mm or less and a length of about 25 mm or less, and further preferably those having a width of 0.5 mm or less and a length of from 0.2 to 10 mm, are generally used.

The pulverized paper, the pulverized nonwoven fabric and the pulverized fabric may be those having hydrophilicity through pulverization or beating a paper material, a nonwoven fabric material or a fabric material into small chips, and examples of the materials constituting them include biosubsatnces, such as pulp, viscose rayon, cotton, linen and wool, and a mixture thereof.

The pulp is an aggregation of fibers that are mechanically or chemically taken out from a plant tissue.

The component containing pulp is a material containing the pulp, papers produced therefrom or both of them.

Examples of the synthetic fibers include fibers of polyacrylate, polyethylene, polypropylene, polyvinyl chloride, polyvinylidene chloride, an ethylene-vinyl acetate copolymer, polyester, polyamide, polyvinyl alcohol and the like, and a mixture thereof.

Among these, papers and fibers formed with polyethylene, polypropylene, polyacrylate or the like as a raw material are generally preferred.

Examples of the inorganic fibrous material include short fibers of rockwool, glass fibers, carbon fibers, asbestos fibers, boron fibers, alumina fibers, metallic fibers and the like. As the organic fibrous material, pulp powder, pulverized paper, pulverized nonwoven fabric, pulverized fabric, natural fibers and synthetic fibers can be used, and examples thereof include at least one kind selected therefrom.

The pulverized paper, the pulverized nonwoven fabric and the pulverized fabric maybe those having hydrophilicity through pulverization or beating a paper material, a nonwoven fabric material and a fabric material into small chips, and examples of the materials constituting them include biosubsatnces, such as pulp, viscose rayon, cotton, linen and wool, and a mixture thereof.

Among these, paper, fibers and the like formed with pulp, cotton or the like as a raw material are particularly preferred.

Another water retainig agent and binder may be added as far as a high viscosity is not considerably exhibited, and the plumping is below 20%. That is, examples of the fibrous material having a binder attached thereto include a toilet roll tissue, tissue paper, newspaper, waste paper and the like.

Specific examples of the powder and the short fibers formed with a synthetic resin or a synthetic fiber include powder or short fibers formed with polyamide, a polyester, such as polyethylene terephthalate, polyurethane, polyvinyl alcohol, polycarbonate, polyvinyl acetate, fibrous carboxymethyl cellulose, cellulose, a cellulose derivative including viscose rayon and acetate fibers, bacterial cellulose, polyvinylidene chloride, polyvinyl chloride, an acrylic resin, polyethylene, polypropylene, a polyethylene fluoride including a polytetrafluoroethylene resin and the like, or an ethylenevinyl acetate copolymer.

The heat-generating body of the invention will be described in detail. The characteristic feature of the heatgenerating body of the invention is that the heat-generating composition is laminated and charged in a container bag, at least a part of which has air permeability, in which a part of the water content of the heat-generating composition is drained to the exterior of the system or is absorbed with the container bag, or in alternative, the heat-generating composition is laminated on a laying material or inserted in a layingmaterial, and further charged in a packing material, and the packing material is constituted with a base material and a covering material, in which the heat-generating composition of the invention is laminated and charged in a sheet form packing material, and a part of the water content of the heat-generating composition is absorbed with the sheet form packing material, the base material and/or'the coveringmaterial or the laying material, or the water content is evaporated by allowing stand upon lamination and/or after lamination, or the excessive water is moved to the exterior of the heat-generating composition through aspiration dehydration, compression and aspiration dehydration, centrifuge dehydration or compression dehydration, or a water absorbing substance, such as a water retainig agent and the like, is made in contact with the heat-generating composition by lamination, diffusion or the like, to move the excessive water is moved to the water absorbing substance, or a part of the water content is drained to the exterior of the heat-generating composition by a combination of these means, whereby a structure capable of generating heat is provided.

In the heat-generating body of the invention, it is desired that the container bag or the packing material is constituted with a base material having a film form, a sheet form or a nonwoven fabric form and a covering material having a film form, a sheet form or a nonwoven fabric form, and at least one, or a part of the base material and the covering material has air permeability. Furthermore, it is desired that it has water absorbing property.

The material of the base material, the covering material or the laying material includes a single layer material and a laminated material of plural layers. In this case, the term lamination means that the layers are totally or partially connected by heat setting, adhesion, cohesion, lamination or the like, or in alternative, the layers are simply superimposed and locally connected, for example, at the peripheral part, the central part or the like, by heat sealing, or with a hot melt adhesive, an adhesive agent.

It is preferred that the heat-generating body of the invention is formed such a manner that the heat-generating composition of the invention having a small thickness is laminated on one prescribed region of a material having a film form, a sheet form or a nonwoven fabric form, and then a covering material having a film form, a sheet form or a nonwoven fabric form is overlaid to cover the heat-generating composition, followed by adhering the base material and the covering material through the heat-generating composition. It is also preferred that the base material and the covering material are sealed by cohesion, heat adhesion or heat fusion at a peripheral part of the heat-generating composition for further improvement in quality and reliability. At this time, compression and/or heating may be appropriately used.

It is also possible that an air permeable polymer is provided on a part or the whole of the surface of the at least one selected from the covering material, the heat-generating composition or those laminated or diffused on the heat-generating composition by melt blowing, coating, spraying, coating or the like, whereby the fixation amount the covering material, the heat-generating composition or those laminated or diffused on the heat-generating composition is ensured. In the case of the covering material, a covering material having an air permeable polymer previously provided may be used in addition to the provision during the process steps.

It is also possible in the invention that an adhesive agent and/or an adhesive polymer, which is a heat sealing material, is provided on at least one of the base material, the covering material and the laying material, and at least the peripheral part of the heat-generating composition intervening between the base material and the covering material is sealed by cohesion, heat adhesion, heat fusion (heat sealing) or the like.

Examples of the material of the base material, the covering material, the laying material and the like include a film, a sheet, a nonwoven fabric and the like, which are not foamed, and in the case where a base material, a covering material or a laying material of a laminated type is constituted, a part thereof may be constituted with a film or a sheet having air permeability. Examples of the film, the sheet or the nonwoven fabric having air permeability include a foamed or nonfoamed film or sheet, paper, a nonwoven fabric, a woven fabric or a porous film or sheet of synthetic fibers or natural fibers, a cloth, various kinds of synthetic resin sheets, a composite sheet thereof, and the like. Examples of the cloth include a woven cloth, knitted cloth, nonwoven cloth. Examples of the fibers constituting the cloth include natural fibers, regenerated fibers using a natural material, such as viscose fibers, semisynthetic fibers, synthetic fibers, a mixture of two or more of them.

Examples of the synthetic resin film include those obtained by providing air permeability by forming fine pores with needles, laser or the like in a film of polyethylene, polypropylene, nylon, polyester, polyvinyl chloride or the like. These may be used solely or in appropriate combinations, and it is preferred from the standpoint of covering workability that fibers or a film having a lower melting point is provided on the side in contact with a support, and nonmelting fibers or film or those having a higher melting point is provided on the other side. In particular, a film, a sheet, a nonwoven fabric and the like having water absorbing property are preferred.

Examples of a polymer of the material constituting the base material, the covering material and the laying material include a polymer material, such as polyethylene, polypropylene, polyester, polyvinyl chloride, polyvinylidene chloride, polystyrene, a saponified product of an ethylene-vinyl acetate copolymer, an ethylene-vinyl acetate copolymer, polycarbonate, aromatic or aliphatic polyamide, polysulfone, polyvinyl alcohol, polyacrylonitrile, a vinyl chloride-vinylidene chloride series resin, polyimide, hydrochloric rubber, polyphenylene oxide, polyphenylene sulfide, polyamideimide, an epoxy resin, polyaminobismaleimide, polyacetal, polyetherether ketone, polyether sulfone, polyallylate, polyoxybenzyl and the like, a natural material, such as paper, pulp, fibers, cotton and the like, and a fabric, a cloth, a nonwoven fabric, a film, a sheet, a foamed sheet and the like formed with a combination of these materials. A stretch material having an adhesive provided thereon, a material having no stretch property through biaxial stretch or the like, and those having substantially no stretch property are included in a base material having no stretch property. These may be used solely or through lamination of two or more kinds thereof.

The stretch material is not particularly limited as far as it has stretch property. Examples thereof include a single material of natural rubber, synthetic rubber, an elastomer, a stretch shape-memory polymer or the like, a mixture or a mixed yarn with a nonstretch material, a fabric, a film, a spandex thread, a thread, a string, a flat plate, a ribbon, a slit film, a foamed body and a nonwoven fabric constituted with a combination thereof, a composite stretch material formed by lamination or the like of these materials with a nonstretch material, and the like. Those obtained by such a method is also included in the stretch material that a nonstretch long fiber or continuous filament are randomly entangled, and randomly adhered or fused to provide a stretch material. A stretch thread, such as a urethane thread, may be formed into a protective stretch thread by winding a nylon thread or the like.

Among elastomers, a thermoplastic elastomer having thermoplasticity is preferred since it has a thermal fusing property, and a laminated body with a nonwoven fabric or the like can be easily produced. A material having no heat fusing property may be imparted with heat fusing property by mixing or mixedly making with a thermoplastic resin, or may be adhered by using a hot melt adhesive (including an adhesive agent) or the like. Furthermore, in the case where the stretch material is air nonpermeable, stretch property can be, or stretch property and air permeability can be imparted by opening pores by a means for opening pores, such as a heat pin method, an embossing method and the like. That is, it is sufficient to have stretch property in total, and may be a single material or a composite material of plural stretch materials or a combination with a nonstretch material.

Specific examples of the synthetic rubber include butadiene rubber, 1,2-polybutadiene, isoprene rubber, styrene-butadiene rubber, a styrene-butadiene-styrene copolymer, butyl rubber, acrylonitrile-butadiene rubber, chloroprene rubber, isobutyrene-isoprene rubber, polyalkylene sulfide, silicone rubber, poly(chlorotrifluoroethylene), a vinylidene fluoride-hexafluoropropylene copolymer, urethane rubber, propylene oxide rubber, epichlorohydrin rubber, an acrylate-acrylonitrile copolymer, an acrylate-2-chloroethylvinyl ether copolymer and the like.

Specific examples of the thermoplastic elastomer include an olefin elastomer, a urethane elastomer, an ester elastomer, a styrene elastomer, an amide elastomer, a vinyl chloride elastomer, syndiotactic poly(1,2-butadiene), poly(trans-1,4-isoprene), a silicone elastomer and the like.

Examples of the olefin elastomer include an ethylene-propylene copolymer, an ethylene-propylene-diene terpolymer, chlorosulfonated polystyrene, chlorinated polyethylene, an ethylene-vinyl acetate copolymer and the like. Among these, ethylene-$\alpha$-olefin formed by using a cyclopentadienyl complex, i.e., a metallocene catalyst, is particularly preferred. As the $\alpha$-olefin, 1-hexene, 1-octene, 1-heptene, 4-methylpentene-1 and the like are particularly preferred.

Examples of the urethane elastomer include a urethane elastomer constituted with a block having a urethane bond and a block of a polycarbonate polyol, an ether polyol, a polyether polyester polyol or a caprolactone polyol.

In particular, a polyurethane film formed therefrom has such characteristics that it is nonporous and moisture permeable and also has stretch property.

Examples of the ester elastomer include an ester elastomer constituted with a block having aromatic polyester and a block having aliphatic polyester or aliphatic polyether.

Examples of the stretch shape-memory polymer include a polyisoprene series, a copolymer, such as a styrene-butadiene series or the like, a polyurethane series, a polymer alloy series and the like.

The thickness of the base material, the laying material and the covering material is not particularly limited while it largely varies depending on purposes. Specifically, it is preferably from 10 to 500 $\mu$m for foot, and is preferably from 10 to 500 $\mu$m, and more preferably from 12 to 250 $\mu$m, in the case where it is used by direct application to a human body. It is preferably from 10 to 2, 500 $\mu$m, and more preferably from 12 to 1,000 $\mu$m, for general purposes.

Examples of the natural fibers include plant fibers, such as cotton, linen, pulp, rayon, and animal fibers, such as silk, animal hair.

The stretch material and the nonstretch material may be any material that is transparent, opaque, colored, non-colored or the like.

A composite stretch material, which is a material having stretch property in total by containing the stretch material and combining with another material that is different from the material thus used in one of morphology, property and kind, may also be used as the stretch material.

Examples of the nonwoven fabric include a single nonwoven fabric of single fibers or composite fibers formed with such a material as rayon, nylon, polyester, acryl, polypropylene, vinylon, polyethylene, urethane, cupra, cotton, cellulose, pulp and the like, a mixedly woven fabric thereof, a laminated body with a heterogeneous fiber layer, and the like. From the standpoint of production process, a dry method nonwoven fabric, a wet method nonwoven fabric, a spunbonded nonwoven fabric, a spunlace nonwoven fabric may be used. A nonwoven fabric constituted with composite fibers having a core/shell structure may also be used. The basis weight of the nonwoven fabric is preferably from 10 to 200 g/m$^2$. In the case where it is less than 10 g/m$^2$, sufficient strength cannot be expected, and that exceeding 200 g/m$^2$ is not necessary from the standpoint of strength.

In the case where covering is carried out in the invention, it is worked into a sheet form having a prescribed thickness through the process of heat fusion. As the method for covering, it is carried out by superposing the covering material on the surface of a support and passing through heat rolls, by adhering through pressing with a pressing machine over the entire thereof or at the peripheral part of the heat-generating composition, by subjecting an opening of a bag having a flat shape using the covering material to heat fusion, or by carrying out heat fusion under compression of the entire bag.

In the case where high water absorbing fibers are used as a nonwoven fabric having water absorbing property in the invention, those having a water absorption capability of 50 mL/gormore, and more preferably 100 mL/g or more, arepreferred. In general, acrylic fibers having a hydrophilic group, a crosslinked structure and the like formed through hydrolysis with an alkali are preferred, and fibers of, for example, a crosslinked product of a polyacrylate salt, an acrylate salt-acrylate ester copolymer, a hydrolyzed product of a crosslinked product of polyacrylonitrie, an acrylate salt-acrylamide copolymer, a polyvinyl alcohol-acrylate salt copolymer or the like, having a thickness of from 1 to 10 denier and a fiber length of about from 10 to 100 mm are preferred.

The nonwoven fabric to be the base material, the laying material or the covering material may be those formed from the high water absorbing fibers solely, and in general, a product obtained by blended spinning with other fibers is used from the standpoint of strength or the like. The kind of the other fibers to be subjected to blended-fiber spinning with the high water absorbing fibers is not particularly limited, and examples thereof include synthetic fibers, such as polyethylene, polypropylene, nylon, acryl, polyester, polyvinyl alcohol, polyurethane, natural fibers, such as cotton, pulp, viscose rayon, and the like. In the case where both surfaces of the resulting heat-generating body are further covered with a film or a nonwoven fabric, synthetic resin fibers, such as polyethylene, polypropylene, nylon, acryl, polyester, are preferred from the standpoint of excellent heat fusing property and the like.

The blending ratio of the high water absorbing fibers with respect to the total nonwoven fabric is generally 20% by mass or more, and preferably about from 30 to 80% by mass. The formation of the nonwoven fabric to be the support may be carried out by either a dry method or a wet method, and it generally has a thickness of from 2 to 15 mm, and preferably from 3 to 12 mm, and preferably has a basis weight of from 20 to 120 g/m$^2$, and more preferably from 30 to 100 g/m$^2$.

In the heat-generating body of the invention, upon laminating the heat-generating composition on the base material, and further covering the heat-generating composition with the covering material, it is possible that a water absorbingmaterial in a film or sheet form is cut into a laminated shape of the heat-generating composition and placed on one surface of the heat-generating composition, or in alternative, both surfaces of the heat-generating composition are covered therewith and sealed with the covering material.

The water absorbing material is not particularly limited as far as it has water absorbing property as a result, irrespective to as to whether or not the material itself has water absorbing property.

Specific examples of the water absorbing material include paper, such as absorbent paper, thin paper for domestic use such as tissue paper, and the like, a nonwoven fabric or a fabric formed with fibers having water absorbing property, a nonwoven fabric or a fabric containing fibers having water absorbing property, a water absorbing porous film or sheet, and the like.

Furthermore, examples thereof also include a water absorbing film or sheet and the like, which is formed in such a manner that a foamed film or sheet, a nonwoven fabric, a fabric or a porous film or sheet, which may have or may not have water absorbing property, is impregnated with a solution of a water absorbing agent, followed by evaporating the solvent, or a water absorbing agent is sprayed, coated, kneaded, attached with pressure, laminated, mixed, transferred or carried on a film or sheet to impart or increase water absorbing property, or in alternative, water absorbing fibers are woven into a nonwoven fabric or a fabric.

Furthermore, examples thereof further include those material formed in such a manner that a cut piece of a water absorbing foamed film or sheet, paper, nonwoven fabric, fabric or porous film or sheet, or the like cut into the plane shape of the heat-generating composition is laminated or fixed on one surface or both surfaces (inner surfaces of the packing material) of an air nonpermeable or permeable film or sheet, such as a foamed film or sheet, paper, a nonwoven fabric, a fabric, which may or may not have water absorbing property, so as to impart water absorbing property.

The paper is not particularly limited as far as it is paper having water absorbing property, and examples thereof include thin paper such as tissue paper, crape paper, craft paper, thick paper such as liner paper, a center core of cardboard, a coated paper board, and a laminated body of one kind or two or more kinds thereof.

The water absorbing agent may be any material that has water absorbing property, and examples thereof include the water retainig agent and the water absorbing polymer exemplified for the heat-generating composition.

In the case of a base material and a covering material that have water absorbing property but are poor in heat fusing property and heat adhesion property, the base material and the covering material are heat-adhered or attached with tackiness at the outer periphery of the heat-generating composition laminated on the base material through a layer of a hot melt adhesive or a layer of a hot melt adhesive agent. Compression and heating may be appropriately applied.

The base material having water absorbing property is a laminated body formed with a heat sealing nonwoven fabric, a water absorbing nonwoven fabric and a synthetic resin film or sheet (an air permeable or nonpermeable film or sheet), it is preferred since the heat sealing nonwoven fabric is hydrophobic, and the water absorbing nonwoven fabric is hydrophilic, so as to exert excellent heat sealing property and water absorbing property.

Examples of the heat sealing nonwoven fabric include a nonwoven fabric formed of a polyolefin resin, a polyurethane series nonwoven fabric, a polyester series nonwoven fabric and the like, as well as a laminated nonwoven fabric of polyester fibers and polyethylene fibers and a composite spunbonded nonwoven fabric.

Other examples of the heat sealing nonwoven fabric include a nonwoven fabric formed with fibers of a double structure, which contains a fiber core and a coated layer coated on the outer periphery of the core, in which the core is formed with polyester fibers or polypropylene fibers, and the coated layer is formed with polyethylene.

Furthermore, still other examples of the heat sealing nonwoven fabric include that using super fine spunbond obtained by dividing composite fibers, which contain polyethyelne fibers and polyester superfine fibers surrounding thereon, in the axial direction of the fibers.

In the case where the base material, the laying material and the covering material is constituted with a laminated body, such constitutions can be exemplified that the base material or the laying material has a structure of a reinforcing layer/an air permeability controlling and soaking preventing layer/a water absorbing layer, and the covering material or the laying material has a structure of a water absorbing layer/an air permeable layer or an air nonpermeable layer. Examples of the reinforcing layer include various kinds of nonwoven fabrics, examples of the air permeability controlling and soaking preventing layer include a film or sheet having or not having air permeability formed with a synthetic resin, such as polyolefin, polyester and the like, and examples of the water absorbing layer include a nonwoven fabric formed with a water absorbing material, such as paper, pulp, cotton, rayon.

In a heat-generating body in a sheet form formed by laminating a sherbet heat-generating composition on a support having a film or sheet form having water absorbing property, it is preferred that the support has a water absorbing capability of 5 $g/m^2$ or more.

The nonwoven fabric including the heat sealing nonwoven fabric, the water absorbing nonwoven fabric and the like preferably has a basis weight of from 5 to 500 $g/m^2$, and more preferably from 10 to 350 $g/m^2$, for exerting the prescribed mechanical strength and heat sealing property and exerting water absorbing property.

The film formed of a polyolefin series resin, the film formed of a polyurethane series resin and the film formed of a polyester series resin preferably has a thickness of from 5 to 500 μm, and more preferably from 10 to 350 μm, for exerting the prescribed mechanical strength and heat sealing property.

The nonwoven fabric formed of a thermoplastic resin preferably has a basis weight of from 5 to 500 $g/m^2$, and more preferably from 10 to 350 $g/m^2$, for attaining improvement of the prescribed mechanical strength and heat sealing property.

In the heat-generating body of the invention, an ultrathin heat-generating body is formed by printing, coating or the like, and in the case where the heat-generating body is formed to a small thickness, a heat-generating reaction amount per unit period of time is lowered only with decompression based on consumption of oxygen in the air by the heat-generating composition inside the bag, and as a result, there are some cases where such a decompression state cannot be maintained that can prevent migration and deviation of the heat-generating composition. It is also further preferred when it can be used irrespective to the decompression state.

In this case, it is preferred that the whole or a part of the heat-generating composition thus molded is fixed on the base material and/or the covering material or the like by applying, spraying, coating, printing or melt-blowing a polymer (preferably an adhesive agent and a thermoplastic polymer) or an emulsion containing the same to cover in a mesh or staggered form (covered with a mesh polymer) , so as to prevent migration and deviation thereof. It is also effective for prevention of loss of shape. The polymer may be any of thermoplastic or thermosetting, and preferred examples thereof include an adhesive agent used in the polymer material, the thermoplastic elastomer and the adhesive agent layer described later. Examples of the thermoplastic polymer include the polymer materials used in the base material and the covering material. It is needless to say that combinations thereof and those having a small adhesive force and a large adhesive force can be used.

The term mesh form means any shape that has air permeability, and an air permeable film is also useful.

The base material, the laying material and the covering material necessarily has a necessary mechanical strength, such as a tensile strength and the like, and are preferably flexible in total for improving affinity to the surface of the body.

That is, the heat-generating body of the invention is further preferably applied to an arthral part, such as elbows, knees, shoulders and the like, and a curved part, such as arms, and in order to follow the arthral part more smoothly, it is preferred that the base material and the covering material, i.e., the packing materials of the heat-generating body, are a stretch film or sheet, and in particular, are formed with a stretch film or sheet.

The stretch base material or covering material, i.e., a stretch film or sheet, is not particularly limited as far as it is formed with a stretch material, and examples thereof include natural rubber, synthetic rubber and a thermoplastic elastomer.

As described in the foregoing, the base material and/or the covering material used in the heat-generating body of the invention include those formed by laminating plural layers having various kinds of functions in the thickness direction.

Unevenness may be formed on the whole surface or a part of the surface part of the heat-generating composition to increase the surface area of the heat-generating composition, whereby the reactivity is improved.

The shape of the unevenness may be any form, and examples thereof include grooves or holes containing continuous or discontinuous patterns of unevenness, and a combination thereof. Furthermore, unevenness may be formed on the whole surface or a part of the surface part of the heat-generating composition and the material, on which it is laminated.

It is also possible that unevenness is physically formed on the surface of the base material, the laying material and the covering material, and thus the adhesion associated with absorption of water from the heat-generating composition and the binding property with the heat-generating composition are improved with the unevenness to prevent migration and deviation.

In the case where the surface of the base material, the laying material and the covering material is a smooth film or a smooth sheet, it is possible that the surface is roughened (uneven surface), or a foamed film, a foamed sheet, paper, a nonwoven fabric, a fabric, a porous film or a porous sheet is used, whereby migration and deviation of the heat-generating composition are prevented.

It is possible that the surface of the base material, the laying material and the covering material is physically roughened (imparting unevenness), and/or the entire or a part of the heat-generating composition is buried in or bonded to a layer formed with the iron powder, the activated carbon, the water absorbing polymer, the thickening agent, the aggregation assistant and/or the binder described in the foregoing or the water absorbing layer formed with the water absorbing material in a film form or a sheet form described in the foregoing, whereby migration and deviation of the heat-generating composition may further be prevented.

It is possible that the base material, the laying material and the covering material are formed with an air nonpermeable or air permeable film or sheet of a water absorbing material, or in alternative, a water absorbing material having water absorbing property is laminated on one surface or both surfaces thereof, so as to form unevenness at a contact part with the heat-generating composition on the base material and/or the covering material and/or the laying material, whereby the adhesion property associated with absorption of water from the heat-generating composition and the binding property with the heat-generating composition are improved with the unevenness to prevent migration and deviation.

It is preferred that the unevenness formed on the entire surface or a part of the surface part of the heat-generating composition and/or the layingmaterial of the invention is formed to a thickness of from 1/5 to 4/5 of the layer thickness of the heat-generating composition layer.

It is preferred that the unevenness formed on the entire surface or a part of the surface part of the heat-generating composition and/or the laying material of the invention is formed with an emboss pattern role, and the emboss angle of the unevenness is in a range of from 90 to 120°.

The thickness of the base material, the laying material and the covering material is preferably in a range of from 10 to 5,000 μm, more preferably from 10 to 2,500 μm, and further preferably from 12 to 1,000 μm, from the standpoint that a prescribed mechanical strength can be obtained, and a prescribed flexibility is obtained.

In the case where the thickness of the base material, the laying material and the covering material is less than 10 μm, the necessary mechanical strength cannot be obtained, and in the case where the thickness of the base material, the laying material and the coveringmaterial exceeds 5, 000 μm, flexibility is lowered even in the case of a foamed body, such as sponge and the like, whereby it is not preferred since the affinity to the surface of the body is considerably lowered, the texture is deteriorated to stiff feeling, and further, the thickness of the entire heat-generating body is increased.

The air permeability of the base material, the laying material and the covering material is not particularly limited as far as the heat generation of the heat-generating composition is exerted, and may be appropriately selected since the desired temperature varies depending on purposes. In order to obtain a heating effect on an organism, the moisture permeability in terms of the Lyssy method (Lyssy method Model L80-400H) is preferably from 50 to 10,000 $g/m^2 \cdot 24hr$, and more preferably from 200 to 6,000 $g/m^2 \cdot 24hr$.

In the case where the moisture permeability is less than 50 $g/m^2 \cdot 24hr$, it is not preferred since the heat generation amount is small to fail to obtain a sufficient heating effect, and in the case where it exceeds 10,000 $g/m^2 \cdot 24hr$, there is a possibility that the heat generation temperature is increased to cause a problem on safety, and the heat generation time is shortened.

The laying material may be air permeable or air nonpermeable. It may be selected depending on the conditions.

In the case where the laying material has water permeability, it is possible that the heat-generating composition provided on the laying material is subjected to compression dehydration, such as press roll and the like, to drain excessive water to the exterior of the system of the heat-generating composition to initiate the heat-generating reaction of the heat-generating composition. The excessive water may also be drained to the exterior of the system by blowing or evaporation with air blasting (hot air, warm air or cold air) or absorption. The excessive water can be drained to the exterior of the system by a combination thereof.

The fact that at least one, or at least a part of the base material, the covering material and the laying material contains or is laminated with at least one of a far infrared radiating substance, a negative ion generating material and a pyroelectric substance means that, a sheet having the far infrared radiating substance or the like kneaded therein is applied to the entire or a part of the base material, the covering material and the laying material, a sheet material, such as a nonwoven fabric, paper and the like, containing fibers having the far infrared radiating substance or the like kneaded therein is applied to the entire or a part of the base material, the covering material and the laying material, an adhesive substance having the infrared radiating substance or the like kneaded therein is applied to the entire or a part of the base material, the covering material and the laying material, and a base material, a covering material and a laying material formed with a plastic film having the far infrared radiating substance or the like kneaded therein and a sheet material of a nonwoven fabric adjusted in air permeability.

In more detail, the base material, the covering material and the laying material having the far infrared radiating substance or the like used in an embodiment of the invention are not particularly limited as far as the far infrared radiating substance or the like can be kneaded in a part or the whole of the base material, the covering material and the laying material, and a plastic film, a nonwoven fabric, paper and the like are used. In the case of the nonwoven fabric, the paper or the like, it is preferably kneaded in the state of fiber before producing the nonwoven fabric, the paper or the like. The base material, the covering material and the laying material having the far infrared radiating substance or the like kneaded therein are not particularly limited as far as they are provided at least on the objective side where the far infrared ray effect is to be exerted.

An example of the base material, the covering material and the laying material having the far infrared radiating substance or the like kneaded therein may be produced in such a manner that a nonwoven fabric, paper or the like is produced with fibers having the far infrared radiating substance or the like kneaded therein, which is then laminated on a sheet to be the base material, or is formed into a sheet solely, or a plastic film having the far infrared radiating substance or the like is produced, which is then laminated on a sheet to be the base material, the covering material or the laying material, or is formed into a sheet solely, or in alternative, an adhesive substance having the far infrared radiating substance or the like kneaded therein is coated on the whole or a part of one surface of a sheet.

The fibers used in the invention are not particularly limited and are preferably formed with a resin that is suitable for kneading a far infrared radiating substance or the like therein, and for example, a thermoplastic resin, such as polyethylene, polypropylene, polyurethane, nylon, polyethylene terephthalate, polyester and the like, and a regenerated resin, such as rayon, a cellulose resin and the like, are preferred.

The production process of the fibers used in the embodiment of the invention is not particularly limited, and examples thereof include a method of making a resin into fibers that is formed by mixing and dispersing the far infrared radiating substance in the raw material of the fibers. The method for producing the basematerial, the covering material and the laying material with the fibers is also not particularly limited, and a conventional production method of a sheet adjusted in air permeability can be employed. For example, a sheet material, such as a nonwoven fabric, paper and the like, is produced from the fibers having the far infrared radiating substance or the like kneaded therein to make the base material, the covering material and the laying material, and furthermore, abagmaterial can be produced by sealing with such a means as heat sealing or the like on three edges or four edges thereof.

The adhesive agent used in the invention is not particularly limited, andvarious kinds of adhesives of a solvent system, an aqueous system, a hot melt type, a reaction type, a pressure-sensitive system or the like. For example, a vinyl acetate resin emulsion, an acrylic resin emulsion, an ethylene-vinyl acetate resin hot melt, a synthetic rubber hot melt and the like are used. In the case where an adhesive substance having the far infrared radiating substance or the like kneaded therein is used as the adhesive agent, the kind of the adhesive agent is not particularly limited, and it is kneaded in the foregoing adhesive agent.

The polymer used in the air permeable polymer is not particularly limited as far as it has a fixing function where the base material, the heat-generating composition and the covering material are adhered, particularly the base material and the covering material are sealed, to fix with an adhesive force or an adhesive force, so as to prevent from releasing, and also has air permeability, and examples thereof include a thermoplastic polymer compound, such as a polymer used in the adhesive agent, which includes those in the form of an adhesive agent.

A far infrared radiating substance, a negative ion generating substance, a pyroelectric substance, a coloring agent, adefoamingagent, amodifyingagent, anantifungalagent, an antibacterial agent, a sterilizing agent, an odor eliminating agent, a deodorizing agent and the like may be mixed in appropriate amounts depending on necessity.

The form of an adhesive agent type is not particularly limited as far as it is formed with a polymer composition exhibiting tackiness at ordinary temperature, and specific examples thereof include a solvent type adhesive agent, an emulsion type adhesive agent and a hot melt adhesive agent.

Among these, an adhesive agent containing a rubber adhesive agent, an acrylic adhesive agent or a hot melt adhesive agent is preferred because of such reasons as high adhesive force, low cost, good long-term stability, small decrease in adhesive force upon application of heat, and the like.

The adhesive agent is generally formed with a base polymer and additives, such as an adhesiveness imparting agent, a softener, an antiaging agent, a filler, an adhesiveness adjusting agent, an adhesiveness improving agent, a thickening agent and the like, and the tackiness, the strength or the like may be adjusted by appropriately mixing the base polymer and the respective components of the additives, for example, by adding an olefin elastomer to a styrene elastomer.

The fact that one has air permeability means that a layer provided has air permeability, and for example, in the case where the layer is an adhesive agent layer, the adhesive agent itself may have air permeability, or may be that having air permeability in total through the presence and absence of the polymer in various kinds of forms, such as a mesh polymer, a stripe form polymer, a dot form polymer and the like. Upon laminating an adhesive agent to a layer form as it is on the base material and/or the covering material and/or the laminated body having air permeability, examples of the method for maintaining the air permeability include such a method where an adhesive agent is partially laminated by printing or transferring to make the non-laminated part as an air permeable part, a method where an adhesive agent is made into a thread form while it is moved in one direction to draw circles, or appropriately moved in two-dimensional directions, in back and forth directions, whereby the gaps among the thread of the adhesive agent maintain the air permeability or moisture permeability, a method of foaming an adhesive agent, i.e., forming by a melt blow method as described below, and the like methods.

That is, in the case where the adhesive agent layer is a layer formed by using a hot melt adhesive agent by a melt blow method, and the adhesive agent is laminated to a layer form with foaming on the base material and/or the covering material having air permeability, the adhesive agent is expanded by 1. 5 times to several times the initial volume thereof, whereby the amount of the raw material can be reduced, and continuous pores are formed to ensure air permeability or moisture permeability. Examples of the hot melt adhesive agent include a known hot melt adhesive agent having been imparted with adhesion.

Examples of the rubber series adhesive agent used in the heat-generating body of the invention include a chloroprene (neoprene) adhesive agent, a nitrile rubber adhesive agent, a polysulfide adhesive agent, a butyl rubber adhesive agent, a silicone rubber adhesive agent and the like.

Specific examples of the hot melt polymer compound used in the heat-generating body of the invention include a styrene adhesive agent using a A-B-A type block copolymer as a base polymer, such as SIS, SBS, SEBS (hydrogenated one of SBS) , SIPS (hydrogenated one of SIS) and the like, a vinyl chloride adhesive agent using a vinyl chloride resin as a base polymer, a polyester adhesive agent using polyester as a base polymer, a polyamide adhesive agent using polyamide as a base polymer, an acrylic adhesive agent using an acrylic resin containing an alkyl ester of acrylic acid or methacrylic acid as a component as a base polymer, a polyolefin adhesive agent using polyolefin, such as polyethylene, ultralow density polyethylene, polypropylene and an ethylene-vinyl acetate copolymer, as a base polymer, a 1,2-polybutadiene adhesive agent using 1,2-polybutadiene as a base polymer, and a polyurethane adhesive agent using polyurethane as a base polymer.

Other examples of the adhesive agent used in the heat-generating body of the invention include a vinyl acetate adhesive agent, a polyvinyl alcohol adhesive agent, a polyvinyl acetal adhesive agent, a polyethylene adhesive agent, a cellulose adhesive agent, an ethylene-vinyl acetate adhesive agent and the like.

Examples thereof also include an acrylic elastomer containing an alkyl ester of acrylic acid or methacrylic acid as a component, an olefin elastomer, such as polyethylene, ultralow density polyethylene, polypropylene and an ethylene-vinyl acetate copolymer, a urethane elastomer and the like.

In alternative, examples thereof include an adhesive agent formed with a modified product thereof and a mixture of at least two kinds of the adhesive agents. It may also be used as an aqueous emulsion.

In particular, that constituted from an elastomer, which is a polystyrene A-B-A type block copolymer, and an adhesiveness imparting resin (such as a petroleum resin and the like) is useful as the adhesive agent.

The modified product herein is formed by changing a part of polymer components necessary for the adhesive agent to another component, and examples thereof include those obtained by changing the properties of the hot melt polymer compound, such as improvement in adhesion and stability of the hot melt polymer compound by changing a part of the components of the hot melt polymer compound to another component.

In the A-B-A type block copolymer, the A block is a nonelastic polymer block of a monovinyl-substituted aromatic compound A, such as styrene, methylstyrene and the like, and the B block is an elastic polymer block of a conjugated diene, such as butadiene, isoprene and the like. Specifically, for example, a styrene-butadiene-styrene block copolymer (SBS), a styrene-isoprene-styrene block copolymer (SIS), or hydrogenated products thereof (SEBS and SIPS), and the like may be used solely or as a mixture thereof.

Gases in the pores are subjected to elastic deformation, and thus the foamed adhesive agent layer is significantly increased in elasticity, stretch property and flexibility. As a result, the flexibility of the packing material formed of the base material and the covering material is improved, and in particular, the sealed part of the base material and the covering material becomes considerably flexible, so as to improve the feeling upon use.

Examples of the method for foaming the hot melt adhesive agent include a known foaming method, such as a chemical foaming method and a physical foaming method. The chemical foaming method is such a method that an inorganic foaming agent, an organic foaming agent or a mixture thereof is used, and foaming is attained with a nitrogen gas or a carbon dioxide gas generated on a decomposition reaction thereof, and the physical foaming method is such a method that a gas, such as compressed air, compressed nitrogen, compressed carbon dioxide gas and the like, is mixed in the adhesive agent through a physical force, such as pressure or the like.

In the invention, an adhesive agent formed with a hot melt polymer substance, an alicyclic petroleum resin and a softener is useful, and the adhesive agent is foamed by the foregoing method.

Examples of the hot melt polymer substance in the invention include those described in the foregoing. The hot melt polymer substance is a base polymer in the adhesive agent, and a foamed body formed by using the same has excellent shape maintenance property, initial tackiness, good adhesion at ordinary temperature and upon heating, and a stable adhesive force after cohesion.

The adhesion imparting agent may be any material that can impart adhesion, and examples thereof include an alicyclic petroleum resin.

The alicyclic petroleum resin is a petroleum resin having a cyclic skeleton, and specific examples thereof include rosin, dehydrated rosin, a glycerin ester of dehydrogenated rosin, a glycerin ester of gum rosin, a hydrated rosin, a methyl ester of hydrated rosin, a glycerin ester of hydrated rosin, a pentaerythritolrosin of hydrated rosin, polymerized rosin, a glycerin ester of polymerized rosin, a coumarone-indene resin, a hydrated petroleum resin, a maleic anhydride-modified rosin, a rosin derivative, a C5 series petroleum resin and the like, which are appropriately used solely or in combination of two or more of them.

The softener is to soften the hot melt polymer substance through dissolution or dispersion, and is to exert appropriate shape maintenance property and flexibility and an adhesive force depending on the combination with the hot melt polymer substance and the alicyclic petroleum resin.

Examples of the softener include a higher fatty acid, liquefied rubber, and a mineral oil. Examples thereof include coconut oil, ricinus oil, olive oil, camellia oil, almond oil, persic oil, peanut oil, sesame oil, soybean oil, mink oil, cotton seed oil, cone oil, safflower oil, oleic acid, liquid paraffin and the like.

In the invention, from the standpoint of adhesiveness, adhesion, feeling upon use and releasing property from the skin, the adhesive layer is formed with from 5 to 40 parts by mass of the hot melt polymer substance, from 5 to 55 parts by mass of the alicyclic petroleum resin and from 5 to 55 parts by mass of the softener, is preferred, and that formed with from 10 to 30 parts by mass of the hot melt polymer substance, from 10 to 50 of the alicyclic petroleum resin and from 15 to 45 parts by mass of the softener is particularly preferred, since it has a high adhesive force to ensure sealing between the base material and the covering material.

Other examples of the adhesive agent layer that can be preferably used in the invention include a layer formed with an emulsion adhesive agent.

The emulsion adhesive agent contains a relatively large amount of water, or water and a solvent, and an adhesive force is exerted by forming it on a releasing sheet (a support, a liner or the like) , such as releasing paper, followed by drying.

In the case where no solvent is contained in the emulsion adhesive agent in this case, a large amount of water is contained, and an adhesive force is exerted by forming it on a releasing sheet (a support, a liner or the like), such as releasing paper, followed by drying.

The emulsion adhesive agent in the invention is not particularly limited, and specific examples thereof include an acrylic emulsion, an ethylene-vinyl acetate emulsion, a vinyl acetate emulsion, a urethane emulsion, a curable vinyl acetate emulsion and a rubber latex of natural rubber or synthetic rubber, and examples of the synthetic rubber latex include a nitrile rubber latex, a styrene-butadiene latex, a chloroprene rubber latex, a styrene-isoprene rubber latex and the like.

In the invention, the adhesive agent may be crosslinked depending on necessity.

The method for crosslinking the adhesive agent is not particularly limited, and examples thereof include known methods, such as a method where a chemical crosslinking agent is added to attain chemical crosslinking, a method where crosslinking is attained by radiation crosslinking, such as irradiation with an ultraviolet ray and irradiation with an electron beam, and the like method.

Specifically, it is possible that the resulting foamed body of the adhesive agent is directly irradiated with a radiation ray, such as an ultraviolet ray, an electron beam and the like, or it is subjected to radiation crosslinking in another apparatus, and then the foamed body of the adhesive agent is transferred to the support.

The crosslinking with irradiation with an ultraviolet ray or an electron beam may be carried out after forming the adhesive agent layer on the base material and/or the covering material, or in alternative, a formed adhesive agent layer in a prescribed shape may be crosslinked, and then the adhesive agent layer may be laminated, for example by transferring or the like, on the base material and/or the covering material.

In the case where the adhesive agent layer is formed with the hot melt adhesive agent in the invention, the elongation property is not largely lost even when the peripheral part of the base material and the covering material are subjected to a heat treatment over the circumference, and in particular, one partially subjected to a heat treatment with a prescribed interval is more preferred because the sealing between the base material and the covering material is further ensured without peeling, and furthermore, the elongation property and the stretch property of the heat-generating body are not lost because the base material and the covering material are partially subjected to a heat treatment with a prescribed interval.

In this case, the heat treatment is carried out with a compression treatment at a temperature more than the softening point of the base polymer in the hot melt adhesive agent but less than the melting point thereof.

The method for covering the heat-generating composition on the base material with the covering material is not particularly limited, and it is possible that the heat-generating composition is laminated on the base material in a roll film form or a roll sheet form conveyed at a high speed, and the covering material in a roll film form or a roll sheet form is guided thereon, followed by sealing the base material and the covering material with the adhesive agent layer under compression with a roll.

The coating amount of the adhesive layer formed with the mesh polymer is not particularly limited, and is generally in a range of from 5 to 1,000 $g/m^2$, particularly in a range of from 10 to 500 $g/m^2$, and further preferably from 15 to 250 $g/m^2$. In the case where the coating amount of the adhesive agent layer is less than 5 $g/m^2$, there are some cases where a uniform coated film is difficult to be obtained, and a desired adhesive force cannot be obtained, and in the case where it exceeds 1, 000 $g/m^2$, it is not preferred since it becomes voluminous to deteriorate the feeling upon use, and the economical efficiency is also deteriorated.

It is possible in the invention that the adhesive agent layer is laminated on at least a periphery on the base material and/or at least a periphery on the lower surface of the covering material and/or at least a part of the surface of the heat-generating composition, and the heat-generating composition intervening between the base material and the covering material is sealed with the adhesive agent layer.

In the invention, the adhesive agent layer is laminated on at least a periphery on the base material and/or at least a periphery of the lower surface of the covering material.

That is, examples thereof include a case where the adhesive agent layer is laminated on a periphery on the base material and/or a periphery of the lower surface of the covering material, a case where the adhesive agent layer is laminated on the whole surface of the base material and/or the whole lower surface of the covering material, a case where the adhesive agent layer is laminated on the whole surface of the base material and/or a periphery of the lower surface of the covering material, a case where the adhesive agent layer is laminated on a periphery of the base material and/or the whole lower surface of the covering material, and a case where the adhesive agent layer is partially laminated over the whole surfaces thereof.

In the case where the adhesive agent layer is laminated on a periphery of the base material and/or a periphery of the lower surface of the covering material, it is possible that the adhesive layer is scattered over the part on the base material and/or the covering material where the adhesive agent layer is eliminated.

It is possible in the invention that an adhesive polymer, which is one kind of a heat sealing material, is provided on at least one of the base material, the covering material and the laying material, and at least a periphery of the heat-generating composition intervening between the base material and the covering material is sealed by heat fusing (heat sealing) by using the adhesive polymer.

Examples of the adhesive polymer include a polyolefin series, such as polyethylene and the like, a polyester series, such as polyethylene terephthalate and the like, an ethylene-vinyl acetate series, a synthetic rubber series, such as a styrene-butadiene copolymer and the like, a polyacrylic series, such as an acrylate ester copolymer and the like, a polyamide resin series, a mixture thereof and the like.

Furthermore, a copolymer resin obtained by combining various kinds of components may be used as the adhesive polymer, as appearing in a modified ethylene-vinyl acetate resin copolymer resin, such as a chlorinated propylene (Cl-PP) modified ethylene-vinyl acetate copolymer resin, an acrylic resin modified ethylene-vinyl acetate copolymer resin and the like.

The mode of provision of the adhesive polymer may be any method as far as it can be used as a heat sealing layer, and examples thereof include preparation in the form of a coating composition in a molten state, a solution state or an aqueous dispersion state (emulsion type), and the like.

An adhesive agent layer or a gel layer may be formed on the whole or a part of one of the exposed surfaces of the heat-generating body of the invention. The adhesive agent layer or the gel layer may be air nonpermeable or air permeable. It is preferably one having air permeability at least one, or a part of the exposed surfaces.

The adhesive agent layer and the gel layer are not particularly limited as far as it is a layer that can be attached or fixed on an outer skin, clothing or the like, and specific examples thereof include a layer formed with a gel component and/or an adhesive agent.

The adhesive agent layer and the gel layer may be directly formed on one of the exposed surfaces of the base material and the covering material, and in this case, in order to improve the bonding force of the adhesive agent layer or the gel layer to the base material or the covering material, the exposed surface of the base material or the covering material is preferably constituted with a film or sheet having a roughened surface, such as paper, a woven fabric, a knitted fabric, a nonwoven fabric, a foamed film and the like.

Examples of the adhesive agent layer include that using the adhesive agent described for the air permeable polymer and a layer formed with an aqueous gel.

Among these, from the standpoint that the fluctuation in adhesive force upon application of heat is relatively small, and particularly in the case of the type that is directly attached to the skin, the attaching property to the skin is good, and irritation to the skin is small, a layer formed with an adhesive agent containing a rubber adhesive agent, an acrylic adhesive agent or a hot melt polymer substance is preferred, and in particular, an adhesive agent containing a hot melt polymer substance, which is formed with an elastomer, which is a polystyrene A-B-A block copolymer, and an adhesiveness imparting resin (such as a petroleum resin and the like), is useful as a releasable adhesive agent that has a high initial tacking force and is excellent in tackiness upon heating.

As the gel layer, in addition to an aqueous gel layer constituted with polyacrylic acid aqueous gel, an adhesive layer formed by adding a water absorbing polymer to the foregoing adhesive agent, i.e., that having an adhesive layer formed with the hot melt polymer substance, the alicyclic petroleum resin, the softener and the water soluble polymer is preferred from the standpoint of hygiene, since a body fluid, such as sweat, secretions and the like, of the skin is absorbed and adsorbed by the water absorbing polymer to maintain the outer surface of the skin clean.

In the invention, as a gel layer is preferable that formed with from 5 to 40 parts by mass of the hot melt polymer substance, from 5 to 55 parts by mass of the alicyclic petroleum resin, from 5 to 55 parts by mass of the softener and from 0.5 to 10 parts by mass of the water absorbing polymer, and in particular, is more useful that formed with from 10 to 30 parts by mass of the hot melt polymer substance, from 10 to 50 parts by mass of the alicyclic petroleum resin, from 15 to 45 parts by mass of the softener and from 1 to 8 parts bymass of the water absorbing polymer.

In the case where there is a possibility that the water absorbing polymer is poor in affinity with the adhesive agent and is not uniformly dispersed, it is preferably treated with a surfactant.

The surfactant is not particularly limited as far as the water absorbing polymer is easily dispersed in the adhesive layer of the adhesive agent, and examples thereof include an anionic surfactant, a cationic surfactant, a nonionic surfactant and an amphoteric surfactant.

The adhesive layer or the gel layer may contain, depending on necessity, an appropriate amount of a drug plant, a herb, an aromatic agent, a skin lotion, a milky lotion, a anti-inflammatory analgesic agent, a percutaneous absorption drug, another adhesive agent, an adhesiveness imparting agent, an antiaging agent, a filler, an adhesiveness adjusting agent, an adhesiveness improving agent, a coloring agent, a defoaming agent, a thickner, a modifying agent, an antifungal agent, an antibacterial agent, a sterilizing agent, an odor eliminating agent or a deodorizing agent, a far infrared ray radiating body, a magnetic body or the like.

The percutaneous absorption drug is not particularly limited as far as it has percutaneous absorption property, and specific examples thereof include a skin stimulating agent, an analgesia and antiflash agent, a drug acting on central nervous system (such as a sleeping and sedative drug, an antiepileptic drug and a neuropsychiatric drug), a diuretic drug, an antihypertension drug, a vasodilator drug, an antitussive drug, an antihistamic drug, an antiarrhythmic drug, a cardiotonic drug, an adrenal cortex hormone drug, a local analgesic drug and the like. These drugs may be used solely or by mixing two or more of them.

In the heat-generating body of the invention, it is particularly preferred that a percutaneous absorption drug is mixed in the adhesive agent layer, whereby the local curative effect is improved, the entire body curative effect is improved, and the drug is absorbed by blood that is actively circulated by the heating effect to circulate the drug through the respective parts of the living body in more effective manner, so as to improve the administration effect in the respective region.

In the heat-generating body of the invention, it is preferred that powder or a molded body of ceramics radiating a far infrared ray is provided, depending on necessity, in the heat-generating composition and/or on the adhesive agent layer side of the heat-generating body for exerting the far infrared ray effect.

The thickness of the adhesive agent layer or the gel layer is not particularly limited and is preferably from 5 to 1,000 µm, more preferably from 10 to 500 µm, and further preferably from 15 to 250 µm. When the thickness of the adhesive agent layer is less than 5 µm, there are some cases where a desired adhesive force cannot be obtained, and when it exceeds 1,000 µm, it is not preferred since it becomes voluminous to deteriorate the feeling upon use, and the economical efficiency is also deteriorated.

The content of the drug is not particularly limited as far as in the range where the medical benefits can be expected, and the content of the percutaneous absorption drug is preferably from 0.01 to 25 parts by mass, and more preferably from 0.5 to 15 parts by mass, per 100 parts by mass of the adhesive agent, from the standpoint of the pharmacologic effect, the economy and the adhesive force.

The adhesive agent layer or the gel layer is generally provided on the whole surface, and it is also possible that a polymer having various kinds of shapes, such as a mesh form, a stripe form, a dotted form and the like, is provided, so as to prevent occurrence of reddening and pain.

An adhesive layer having water permeability may be used as the adhesive layer, and in this case, a function is provided that a body fluid, such as sweat and the like, exuded from the skin is permeated to the side of the support, and thus the permeated body fluid is absorbed by the water absorbing layer to prevent lowering of the adhesive force, whereby the heating medical application is prevented from peeling, and the adhesion to the outer skin is prevented from lowering.

Specific examples thereof include a layer having continuous pores (through holes) formed by irradiating with a radiation ray, such as an electron beam, a laser beam and the like with high energy, and an adhesive layer formed with an adhesive agent containing the hot melt polymer substance formed in a mesh form.

The water absorption rate of the water absorbing material is preferably from 2.5 to 35% by mass, and more preferably from 5 to 30% by mass, in terms of a water absorption ratio based on the water content in the heat-generating composition.

The measuring method of the absorption rate (W% by mass) of water content in the heat-generating composition to the packing material will be described.

The base material and the covering material, which are the packing material, are punched into a circular form having a diameter of 60 mm and are dried at a temperature of 55°C under reduced pressure of from 1 to 2 Pa for 24 hours to prepare a base material piece and a covering material piece, which are then subjected to measurement. In this case, the weight of the base material piece is designated as K1 (g) , and the weight of the covering material piece is designated as H1 (g).

Subsequently, the heat-generating composition is laminated on the dried base material piece through a mold to make a circular shape having a diameter of 60 mm with a thickness of about 850 µm, and the laminated weight of the heat-generating composition S (g) is obtained by subtracting the weight of the base material piece K1 (g) from the total weight after lamination C (g).

Assuming that the water content ratio in the heat-generating composition is designated as P% by mass, the total water content (g) in the laminated heat-generating composition is S x P / 100.

Furthermore, a dried covering material piece is laminated on the exposed surface of the heat-generating composition on the base material, and then an acrylate plate having thickness of about 1 mm is placed thereon. A weight of 2.5 Kg is further placed thereon and is allowed to stand for 5 hours.

Thereafter, the base material piece and the covering material piece are separated from each other, and the heat-generating composition attached thereto is substantially completely removed with tweezers, followed by measuring the weights of the base material piece and the covering material piece. In this case, the weight of the base material piece is designated as K2 (g) , and the weight of the covering material piece is designated as H2 (g).

Subsequently, the base material piece and the covering material piece are dried at a temperature of 55°C under reduced pressure of from 1 to 2 Pa for 24 hours, and the weights thereof are measured. The weight of the base material piece is designated as K3 (g) , and the weight of the covering material piece is designated as H3 (g).

The water absorption rate W is calculated by the following equation.

$$W = 100 \times \{(K2+H2)-(K3+H3)\}/S \times P/100\}$$

The thus resulting heat-generating body is used for warming in winter season, and in addition, is used for affections, such as stiff neck, aching muscle, stiff muscle, low back pain, coldness in hands and feet, nerve pain, rheumatism, bruise, sprain and the like, whereby curative effect owing to heating can be expected. Furthermore, it can be used for heating and heat retention of machines and pet animals, and can be applied to a deoxygeniating agent, an antifungal agent and the like.

The production process of the heat-generating body of the invention will be described.

Upon producing the heat-generating composition of the invention, it is possible that only the solid components are placed in a mixing apparatus and uniformly mixed, and then water or an aqueous solution or dispersion of the oxidation accelerator, the dispersion stabilizer and the like is added, or it is possible that all the components of the heat-generating composition are placed in a mixing apparatus, and the components are mixed, or in alternative, it is also possible that the components are respectively placed, or they are divided into some groups, which are placed and mixed.

As the mixing apparatus for the components, the use of a kneading apparatus, such as a screw, a mixer or the like, is convenient for attaining dispersion stabilization of the heat-generating composition. In some cases, a kneading apparatus carrying out kneading, such as a kneader or the like, may be used.

The heat-generating composition of the invention means such one in that the heat-generating composition can be printed by using a known printing technique, such as leveling molding, pressing and leveling molding, pressing and transferring molding, gravure printing, screen printing, pasting printing, spraying and the like, or can be easily transferred and laminated by applying or coating with a head coater, a roller, an applicator or the like, and is not particularly limited as far as it is such a heat-generating composition.

After molding the heat-generating composition on the base material, the laying material or the like, or after further covering with the covering material, the laying material or the like and then adjusting to a flat form by passing through rollers, it is possible that at least a part of the base material, the covering material or the like is released to form a heat-generating body, or in alternative, it is used as it is to seal the covering material and the base material, and the assembly is sealed in an outer bag formed with a air nonpermeable packing material.

The production process may be carried out in the air or in an inert atmosphere, and in order to prevent the iron powder completely from oxidation under contact with oxygen in the air during the production process, it may be carried out in an inert gas atmosphere, such as nitrogen, argon or the like. The sheet form heat-generating body thus obtained may be used as it is or after cutting into a desired size for heating and heat retention of a human body, a machine, a part, a food and the like.

In order to prevent contamination of an article to be kept heating due to migration or the like of the components of the heat-generating body, it is possible that the sheet form heat-generating body is covered with a film having air permeability, or in alternative, a fibrous substance is further mixed in the surface of the heat-generating body.

The heat-generating composition of the invention can reduce the amount of excessive free water in the heat-generating body to improve the heat-generating capability to a large extent, can prevent generation of dusts upon production of the heat-generating body, and can be formed by employing a mold-through molding method, a mold pressing molding method, a printing and transferring method, such as gravure printing, screen printing, coating and the like, and the like. Therefore, the heat-generating composition can be uniformly distributed, the accuracy in thickness of the heat-generating composition and the distribution thereof can be increased to improve the quality of the products, and an ultrathin heat-generating body can be easily produced at a high speed. Furthermore, the heat-generating composition is molded by mold-through molding or mold pressing molding on the base material or the covering material having water absorption property, or on the water absorption layer or a packing material having water absorption property formed thereon, whereby the heat-generating composition can be fixed to the bag material in such a state that it is uniformly distributed therein, and as a result, maintenance of heat-generating capability and prevention of migration and deviation of the heat-generating composition can be attained. At least a part of the heat-generating body can be covered with a polymer in a mesh form by a melt blow method or the like, so as to prevent migration and deviation of the heat-generating body, whereby deviation in a bag can be difficult to occur even in the case where it is contained in a bag formed with a material having a large airflow amount.

The same effect as in the foregoing can be obtained when the heat-generating composition of the invention is laminated on a water permeable base material, and a part of excessive water content is drained by reduction of pressure or the like.

The heat-generating composition of the invention may have any property relating to flowability, such as a viscosity and the like, as far as it is in such a range that it can be molded, and the drainage property and the heat-generating capability are ensured.

A first production process of the heat-generating body of the invention (hereinafter, referred to as a "first process of the invention") contains a first step of preparing the heat-generating substance containing blending and mixing, a second step of carrying out molding, such as mold through, mold pressing, laminating or the like, and a fourth step of overlaying the coveringmaterial and sealing, and the first step, the second step and the fourth step are carried out in this order.

Examples of the second step include a second step B of carrying out molding by using a mold, a head with an agitator, or a head, a leveling plate and a magnet provided under the leveling plate on the opposite side with respect to the base material, and a second step A of carrying out molding by using a leveling plate having a vibration mechanism (or a pressing plate and a vibration bar) (provided that the heat-generating agent may be pressed by providing a magnet under the pressing plate having a vibration mechanism or the pressing plate on the opposite side with respect to the base material). Vibration may be applied to the head or the leveling plate, and vibration may be applied to the heat-generating composition by inserting the vibration bar into the heat-generating composition.

A second production process of the heat-generating body of the invention (hereinafter, referred to as a "second process of the invention") contains a first step of carrying out blending and mixing, a second step of carrying out molding, such as mold through, mold pressing, transferring, laminating or the like, a third step of laminating or scattering at least one selected from iron powder, a carbon component, ceramic powder radiating a far infrared ray, a fibrous material radiating a far infrared ray, an organic silicon compound, a water absorbing agent, a binder, athickener, an excipient, a coagulating agent, a soluble adhesive material and a water absorbing polymer on the heat-generating composition thus molded, a fourth step of overlaying the covering material, and a seventh step of punting out, and the first step, the second step, the third step, the fourth step and the seventh step are carried out in this order.

A third production process of the heat-generating body of the invention (hereinafter, referred to as a "third process of the invention") contains a third step B of laminating or scattering at least one selected from iron powder, a carbon component, ceramic powder radiating a far infrared ray, a fibrous material radiating a far infrared ray, an organic silicon compound, a water absorbing agent, a binder, a thickener, an excipient, a coagulating agent, a soluble adhesive material and a water absorbing polymer on the base material or the laying material, a first step of preparing the heat-generating substance containing blending and mixing, a second step of carrying out molding, such as mold through, mold pressing, transferring, laminating or the like, and a fourth step of overlaying the covering material and sealing, and the third step B, the first step, the second step and the fourth step are carried out in this order.

A fourth production process of the heat-generating body of the invention (hereinafter, referred to as a "fourth process of the invention") contains a first step of preparing the heat-generating substance containing blending and mixing, a second step of carrying out molding, such as mold through, mold pressing, transferring, laminating or the like, a fifth step of overlaying the covering material and adjusting the shape by compressing, and a fourth step of sealing, and the first step, the second step, the fifth step and the fourth step are carried out in this order. A desired pressure is applied to the laminated body of the heat-generating composition with a press roll or the like, whereby the shape is adjusted to improve the shape maintenance property.

A fifth production process of the heat-generating body of the invention (hereinafter, referred to as a "fifth process of the invention") contains a first step of carrying out blending and mixing, a second step of carrying out molding, such as mold through, mold pressing, transferring, laminating or the like, a third step A of providing a mesh form polymer on the heat-generating composition thus molded, a fourth step of overlaying the covering material and sealing, and a seventh step of punching, and the first step, the second step, the third step A, the fourth step and the seventh step are carried out in this order.

A sixth production process of the heat-generating body of the invention (hereinafter, referred to as a "sixth process of the invention") contains a first step of carrying out blending and mixing, a second step of carrying out molding, such as transferring, laminating, mold pressing, mold through, or the like, a fourth step of overlaying a water permeable laying material or a water nonpermeable laying material, a sixth step of carrying out dehydration, such as aspiration dehydration, centrifuge dehydration, compression dehydration, decompression dehydration, compression and decompression dehydration and the like, a fourth step of overlaying the covering material or the base material and the coveringmaterial, and a seventh step of punching, and the first step, the second step, the fourth step, the sixth step, the fourth step and the seventh step are carried out in this order. The laying materials in the second step and the fourth step using the laying materials may be replaced with each other.

A seventh production process of the heat-generating body of the invention (hereinafter, referred to as a "seventh process of the invention") contains a first step of carrying out blending and mixing, a second step of carrying out molding, such as transferring, laminating, mold pressing, mold through, or the like, a fourth step of overlaying a water permeable laying material or a water nonpermeable laying material, a sixth step of carrying out dehydration, such as aspiration dehydration, centrifuge dehydration, compression dehydration, decompression dehydration, compression and decompression dehydration and the like, a seventh step of punching, a fourth step of overlaying the covering material on the heat-generating body thus punched and sealed, and a seventh step of punching, and the first step, the second step, the fourth step, the sixth step, the seventh step, the fourth step and the seventh step are carried out in this order. The laying materials in the second step and the fourth step using the laying materials may be replaced with each other.

A eighth production process of the heat-generating body of the invention (hereinafter, referred to as a "eighth process of the invention") contains an eighth step, in which the heat-generating body produced by the first to seventh processes of the invention is inserted between two films or sheets, and simultaneously with the insertion or after the insertion, the two films or sheets are sealed at a periphery of the heat-generating body to a size exceeding the size of the heat-generating body, and simultaneously with the sealing or after the sealing, it is punched, and the eighth step is carried out after the final step of the respective production processes. In the case where the films or sheets have airtightness, they have a function as an outer bag for storage.

It is possible that the first step, the second step, the second step A, the second step B, the third step, the third step A, the third step B, the fourth step, the fifth step, the sixth step, the seventh step and the eighth step are appropriately combined including duplication and random order, so as to produce the heat-generating body of the invention.

For example, such a production process can be constituted in that the third step B, the first step, the second step B, the third step B, the third step A, the fourth step and the seventh step are carried out in this order.

The respective steps may be carried out in an inert gas atmosphere, such as nitrogen, argon or the like, for preventing the iron powder from oxidation under contact with oxygen in the air.

The respective steps will be described in detail.

Examples of the heat-generating composition of the invention include those similar to the foregoing.

In the first step, iron powder, activate carbon, an oxidation accelerator, a dispersion stabilizer and water, as well as, depending on necessity, a water retainig agent, a heat-generating assistant, a silicone resin, a hydrogen generation suppressing agent, a foaming agent and the like are mixed.

The order of mixing is not particularly limited, and examples thereof include such operations that

(1) all the components are placed in a mixing apparatus and then uniformly mixed,
(2) the components are sequentially placed in a mixing apparatus and then uniformly mixed,
(3) only the solid contents among the all the components are divided into some groups, which are sequentially placed therein, and
(4) all the solid contents are placed in a mixing apparatus and uniformly mixed in the mixing apparatus, and then water or an aqueous solution or dispersion of a metallic chloride is placed therein, followed by mixing.

The mixing apparatus used in the first step of the invention is not particularly limited as far as it can uniformly mix the components constituting the heat-generating composition of the invention, and specific examples thereof include a screw blender, a ribbon mixer, a spartan mixer, a roll mixer, a banbury mixer, a mixing and extruding screw and the like.

Upon producing the heat-generating composition of the invention, an arbitrary mixing apparatus may be used as far as it can basically mix the heat-generating materials.

In the second step, the heat-generating composition obtained in the first step is molded into an arbitrary shape on at least one region on the base material or the laying material in a film form or a sheet form by mold-through molding, mold pressing molding or printing or coating, such as screen printing or the like. Furthermore, specific examples thereof include the second step A and the second step B, which may be appropriately used.

The base material and the layingmaterial referred therein are the same as those described for the heat-generating body of the invention.

In the second step A, molding, such as mold pressing molding, mold-through molding, lamination and the like, is carried out under application of vibration. The means for applying vibration may be any means that can apply vibration to the heat-generating composition of the invention in a clay form, and examples thereof include a vibrating apparatus that is ordinarily employed using an eccentric motor, a piezoelectric element or the air.

In this case, pressing of the heat-generating composition with a pressing plate may be carried out. The pressing plate may be any article that can press the heat-generating composition into a mold, and is preferably formed with plastics, such as an acrylic resin, a vinyl chloride resin, polyethylene and the like, or a metal or a composite material thereof, such as iron, stainless steel and the like, examples of which include a plate having spring elasticity.

In the second step B, a cylinder head having an agitation device is used for imparting flowability to the heat-generating composition, and flowabilityisimparted to the heat-generating composition under agitation of the heat-generating composition supplied into the head, followed by supplying to the mold. Vibration may be applied to the head at this time. The base material, the mold plate and a receiving plate for receiving them (such as a belt of a belt conveyer or the like) are passed as a unit between a leveling plate provided by fixing in somewhat front (proceeding direction of the mold plate) of the head on a lower part and a magnet placed thereunder. The heat-generating composition is laid on the base material through the mold by the magnet power, and simultaneously, the surface of the heat-generating composition is leveled with the leveling plate along the mold to accomplish molding. Thereafter, the mold is released from the base material. The magnet may be any one that has magnetism, and examples thereof include a permanent magnet and an electromagnet.

It is also possible that the mixing apparatus is simplified to a rotation bridge preventing apparatus, and a bridge caused upon supplying to the head the heat-generating composition supplied to the head is prevented.

In the second step C, a roll having a mold is attached to the cylinder head used in the second step B. The heat-generating composition is supplied from the head to the roll, and the surface of the heat-generating composition pressed into the mold is leveled with a leveling plate to accomplish molding. The base material, the mold plate and a receiving plate for receiving them (such as a belt of a belt conveyer or the like) are passed as a unit under the magnet provided under the roll. The heat-generating composition is laid on the base material through the mold by the magnet power, and simultaneously, the surface of the heat-generating composition is leveled with the leveling plate along the mold to accomplish molding. Thereafter, the heat-generating composition inside the mold is transferred to the base material by the magnet power. The magnet may be any one that has magnetism, and examples thereof include a permanent magnet and an electromagnet.

It is also possible that the mixing apparatus is simplified to a rotation bridge preventing apparatus, and a bridge caused upon supplying to the head the heat-generating composition supplied to the head is prevented.

In the second step, the heat-generating composition may be laminated on one location or two or more locations in the width direction on the upper surface of the base material, or may be laminated in a staggered form in the longitudinal direction of the base material.

In the third step, at least one selected from iron powder, a carbon component, ceramic powder radiating a far infrared ray, a fibrous material radiating a far infrared ray, an organic silicon compound, a water absorbing agent, a binder, a thickener, an excipient, a coagulating agent, a soluble adhesive material, a water absorbing polymer and a mesh polymer is laminated or diffused on the heat-generating composition thus molded, the base material or the laying material.

In the third step, at least one selected from iron powder, a carbon component, ceramic powder radiating a far infrared ray, a fibrous material radiating a far infrared ray, an organic silicon compound, a water absorbing agent, a binder, a thickener, an excipient, a coagulating agent, a soluble adhesive material, a water absorbing polymer and a mesh polymer is laminated or diffused on at least one prescribed region on the base material, the covering material, the laying material or the heat-generating composition thus laminated in the form of a film or sheet.

In the third step A, a mesh polymer is provided on at least one selected from the base material, the laying material, covering material and the heat-generating composition thus laminated or at least a part thereof. This is carried out by an ordinary working technique, such as melt blow, printing, coating and the like. According thereto, the laminated body of the heat-generating composition of the invention can be further strongly fixed on the base material and/or the laying material and/or the covering material. Furthermore, in the case where the polymer has adhesion, the base material and/or the laying material and/or the heat-generating composition and/or the covering material are adhered through the adhesion.

In the third step B, at least one selected form iron powder, a carbon component, ceramic powder radiating a far infrared ray, a fibrous material radiating a far infrared ray, an organic silicon compound, a water absorbing agent, a binder, a thickener, an excipient, a coagulating agent, a soluble adhesive material and a water absorbing polymer is laminated or diffused on the base material, the laying material or the covering material.

In the fourth step, the laying material and the covering material in a f ilmor sheet form is overlaid to cover the laminated body of the heat-generating composition of the invention and seal. The laying material and the covering material used herein are the same as those described for the heat-generating body of the invention. In this case, it is preferred that the base material and/or the laying material and/or the covering material are sealed by cohesion, heat adhesion or heat fusion at a periphery of the laminated body of the heat-generating composition.

At least one or one of, or a part of the base material, the laying material and the covering material has air permeability or water permeability.

In the fifth step, the shape of the laminated body of the heat-generating composition is adjusted by compression, planarization or the like of the laminated body with a press roll or the like. That is, a desired pressure is applied to the laminated body of the heat-generating composition with a press roll or the like to adjust the shape, whereby the shape maintenance property is improved.

In particular, the method for making the heat-generating material into a sheet is not particularly limited, as far as it is a method that can make the heat-generating material into a sheet, such as a method using a rolling device of a single stage press roll, where rolling carried out once or plural times with a single stage press roll, a method using a rolling device of a multistage press roll, where rolling is carried out plural times by one rolling operation with a multistage press roll, and the like.

In the case where the heat-generating material cannot be formed into a sheet by only one rolling operation due to the composition thereof in this case, or in the case where the heat-generating sheet is required to change the thickness or to have a high density, the pressing operation may be carried out plural times, on which the pressure may be increased stepwise.

It is possible that pressing is carried out with the pressing roll to adhere bypressing the heat-generatingmaterial to form into a sheet form, and the heat-generating sheet is wound into a roll form to improve the storage stability, the carrying property and workability, and in this case, it is possible that compression of the heat-generating sheet with the pressing roll and the winding are repeated plural times, so as to adjust the density and the air inflow property of the heat-generating sheet.

In the sixth step, dehydration, such as filtration, aspiration dehydration, centrifuge dehydration, compression dehydration with a pressing roll or the like, and the like are carried out. In some cases, removal of water by evaporation or the like by heating, blowing hot air or cold air, or aspiration at this time or thereafter.

The production process may be carried out in an inert gas atmosphere, such as nitrogen, argon and the like, for preventing the iron powder from oxidation under contact with oxygen in the air.

In the seventh step, the laminated body is punched into a prescribed shape. The step of punching into a prescribed shape may be carried out by standing still the laminated body, and in this case, plural pieces of the laminated body arranged in the conveyning direction of the laminated body and the width direction perpendicular thereto may be simultaneously punched to form a large amount of the heat-generating bodies at the same time, whereby the cost can be reduced as a result.

In this method, for example, in the case where the base material in the form of a roll film or roll sheet is conveyed at a rate of from 30 to 200 m/min, on which the heat-generating composition is laminated, and the covering material is overlaid on the heat-generating composition by guiding the covering material in the form of a roll film or roll sheet thereon, whereby the laminated body is obtained, the laminated body can be punched into an arbitrary shape by using a roll press or the like under conveying the laminated body at the conveying speed on production thereof, such as from 30 to 200 m/min, whereby the heat-generating bodies of the invention can be continuously obtained. It is needless to say that it is possible that the laminated body is once wound into a roll form, and the roll is punched by being intermittently fed.

At this time, it is possible that pressing is carried out with a pressing roll to adhere by pressing the heat-generating material to the base material constituting the packing material to laminate the heat-generating sheet on the base material, and the laminated sheet is wound into a roll form to improve the storage stability, the carrying property and workability, and in this case, it is possible that compression of the laminated sheet with the pressing roll and the winding are repeated plural times, so as to improve the adhesion property between the base material and the heat-generating sheet and to adjust the density and the air inflow property of the heat-generating sheet depending on necessity.

Subsequently, the heat-generating sheet is cut into prescribed size and shape with a roll cutter or the like corresponding to purposes and sealed in an air permeable packing material, and it is further sealed in an air nonpermeable packing material to prevent from contacting with the air, followed by subjecting to distribution.

In this case, cost reduction of a large extent can be attained in such a manner that the laminated body is punched at one location or two or more locations in the width direction, and is continuously punched at plural locations arranged in a staggered manner along the longitudinal direction of the laminated body, so as to produce a larger amount of the heat-generating bodies of the invention in a short period of time.

The laminated body is punched into a shape that covers an arbitrary region corresponding to the purposes of the resulting heat-generating body. That is, the laminated body obtained in the third method of the invention is punched into an arbitrary shape, and the heat-generating body of the invention thus obtained through punching is utilized for a foot, a shoulder, a hip or the like without any particular limitation and may have an arbitrary shape.

In the eighth step, the heat-generating body is inserted between two films or sheets, and simultaneously with the insertion or after the insertion, the two films or sheets are sealed at a periphery of the heat-generating body to a size exceeding the size of the heat-generating body, and simultaneously with the sealing or after the sealing, it is punched.

Examples of the invention will be specifically described with reference to the figures, but the invention is not limited thereto.

(Example 1)

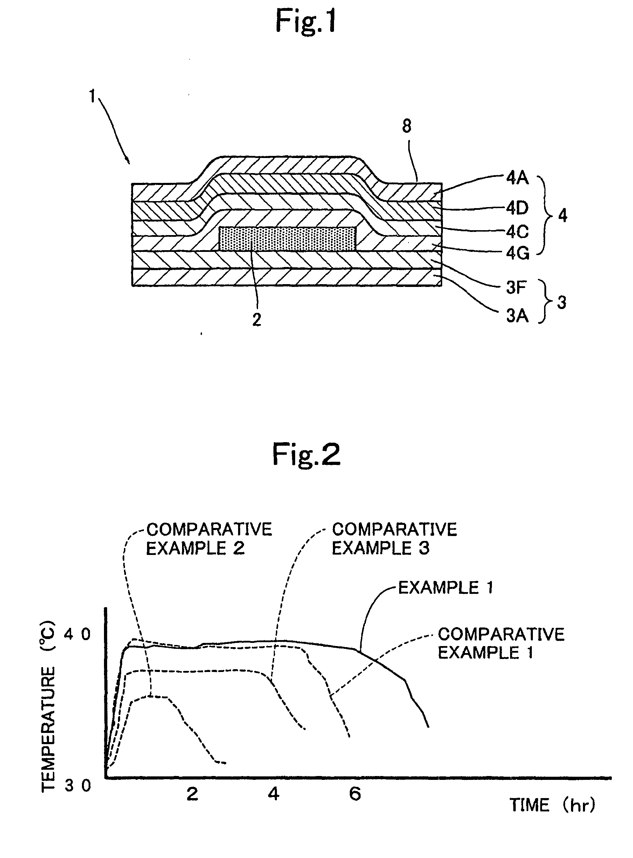

Fig. 1 is a cross sectional view of a heat-generating body 1 according to Example 1 of the invention. A mold not shown in the figure was set on a base material 3 having a size of a length of 85 mm and a width of 65 mm, a heat-generating composition 2 was placed in the mold and molded by mold-through molding by using a leveling plate not shown in the figure to laminate the heat-generating composition 2 on the base material 3, a covering material 4 in a film form having substantially the same size as the basematerial 3 was further overlaid thereon, and a periphery 8 thereof was sealed to a width of 7 mm to obtain the heat-generating body 1.

The heat-generating composition 2 was formed by mixing a water separation-preventing stabilizer with a heat-generating composition containing metallic powder as a major component, and 6 parts by mass of sodium chloride (NaCl) as a metallic chloride, 50 parts by mass of water, 5 parts by mass of activated carbon as a carbon component, 5 parts by mass of wood powder passing 100 mesh (containing particles having a size of 150 μm or less in an amount of 50% or more) and 0.04 part by mass of CMC were mixed with 100 parts by mass of iron powder (DKP, produced by Dowa Teppun Co., Ltd.).

The surface of the molded body was smoother owing to the use of the wood powder passing 100 mesh (containing particles having a size of 150 μm or less in an amount of 50% or more) .

As the base material 3, such an air nonpermeable water absorbing film was used that was formed by laminating, from the exposed surface, a polyethylene film 3A having a thickness of 50 μm using polyethylene polymerized with a metallocene catalyst and water absorbing craft paper 3F having a thickness of 200 μm.

As the covering material 4, such an air permeable laminated film was used that was formed by laminating, from the exposed surface, a polyester nonwoven fabric 4A (having a basis weight of 30 g/m$^2$), a polyethylene porous film 4D having a thickness of 40 μm, and craft paper 4C having a thickness of 50 μm. The symbol 4G shows a hot melt adhesive agent. The coveringmaterial had an air permeability in terms of the Lyssy method of 300 g/m$^2$·24hr.

In this case, molding was carried out in such a manner that the heat-generating composition 2 was laminated on the base material 3, the covering material 4 having a mesh hot melt adhesive agent 4G provided thereon was overlaid thereon, and then the heat-generating composition 2 was subjected to a pressure roll treatment with a pair of pressure rolls with a gap between the pressure rolls adjusted to 1.5 mm over the base material 3 and the covering material 4, so as to obtain the heat-generating body 1.

The heat-generating body 1 was sealed in an air nonpermeable outer bag not shown in the figure immediately after the production.

(Comparative Example 1)

A heat-generating body was obtained in the same manner as in Example 1 except that CMC as a water separation-preventing stabilizer was removed from the heat-generating composition of Example 1, i.e., the amount of CMC was changed to 0.00 part by mass.

(Comparative Example 2)

A heat-generating body was obtained in the same manner as in Example 1 except that 1.00 part by mass of CMC as a water separation-preventing stabilizer was mixed in the heat-generating composition.

(Comparative Example 3)

A heat-generating composition having a water mobility value of 6 in the form of powder, which was less than 7, was formed with the same constitutional components as in Comparative Example 1, and a heat-generating body was produced in the same manner as in Example 1 with careful leveling carried out.

The heat-generating compositions produced in Example 1, Comparative Example 1, Comparative Example 2 and Comparative Example 3 were measured for water mobility value, separation degree and incremental degree of viscosity, and the heat-generating bodies were subjected to a heat-generating test. The heat-generating characteristics are shown in Fig. 2, and the relationships between the water mobility value, the separation degree and the incremental degree of viscosity are shown in Table 2. The water mobility value, the separation degree and the incremental degree of viscosity were measured in the methods described in the foregoing. The heat-generating test was carried out in the following manner. One piece of flannel cloth (cotton 100%) having a thickness of 6 mm and a size of 500 x 500 mm was placed on a steel plate adjusted to 30°C, and a temperature sensor was attached to the center thereof. A center part of a heat-generating body having a size of 100 x 100 mm was placed on the temperature sensor. Two pieces of flannel cloth (cotton 100%) having a thickness of 6 mm and a size of 500 x 500 mm were placed thereon, and a weight of 1 kg was placed thereon.

The heat-generating characteristics (heat-generating attained temperature-heat-generating time curves) as heat-generating bodies were measured by the test. The measurement of the heat-generating attained temperatures was carried out by using a data collector in a temperature-controlled room at a temperature of 20°C and a humidity of 65%.

Table 2

| Water separation-preventing stabilizer (CMC) (Part by mass) | Water mobility value | Separation degree | Incremental degree of viscosity (CP) (cP) |
|---|---|---|---|
| Example 1 (0.04) | 23 | 10 | 100 |
| Comparative Example 1 (0.00) | 25 | 37 | 0 |
| Comparative Example 2 (1.00) | 0 | 0 | 180,000 |
| Comparative Example 3 (0.00) | 6 | 1 | 0 |

It was understood from Table 2 and Fig. 2 that differences of the water mobility value were found in Example 1, Comparative Example 1 and Comparative Example 2, which showed differences in drainage property. In the heat-generating characteristics, Comparative Example 2 was significantly deteriorated in heat-generating characteristics, which was assumed to be due to the amount of the water separation-preventing stabilizer and the incremental degree of viscosity.

The heat-generating body 1 of the invention thus constituted can also be used as a deoxygenating agent for foods and exerts such an effect that oxygen is removed by reacting with the air (oxygen) inside a packing material of the food, so as to maintain excellent eating quality and flavor for a long period of time. This was the case in the following examples.

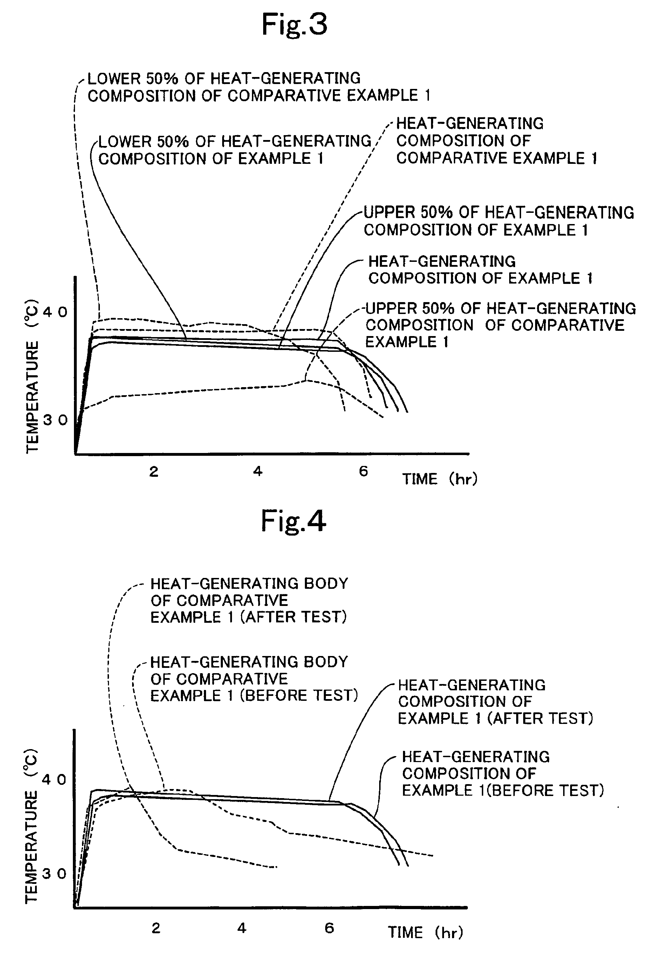

Heat-generating bodies were produced according to Example 1 with the compositions of upper 50% and lower 50% of Example 1 and upper 50% and lower 50% of Comparative Example 1 by using the heat-generating compositions collected upon measuring the separation degrees of the heat-generating compositions of Example 1 and Comparative Example 1, and the temperature characteristics there of were measured in the similar manner as in Example 1. The results are shown in Fig. 3.

A deterioration test was carried out by using the heat-generating bodies of Example 1 and Comparative Example 1 to obtain the results shown in Fig. 4. It was understood therefrom that the heat-generating body of Example 1 using the water separation-preventing stabilizer is considerably prevented from deterioration of the heat-generating duration. The deterioration test herein is such a measurement of the deterioration degree in that a heat-generating body charged in an airtight outer bag (having an air permeability of 0.9 cc/m$^2$·day and a moisture permeability of 0.3 g/m$^2$·day) was stored at 50°C for 30 days, and the heat-generating body was taken out from the bag and subjected to a heat-generating test for comparison between the heat-generating duration before and after the test.

The moisture permeability was a value measured by the Lyssy method (under conditions of atmospheric pressure at 40°C and 90% RH), and the oxygen permeability was a value measured with a measuring machine produced by Modern Controls, Inc. (under conditions of atmospheric pressure at 23°C and 90%RH).

(Comparative Example 4)

A heat-generating body was produced in the same manner as in Example 1 except that 1.00 part by mass of CMC as a water separation-preventing stabilizer was mixed in the heat-generating composition, followed by carrying out the same test as in Example 1. Thus, the temperature was 36°C for 2 hours, and sufficient heat-generating characteristics could not be obtained.

Heat-generating bodies having a thickness of 3 mm, a length of 100 mm and a width of 80 mm were obtained by using the heat-generating composition used in Example 1 and a powder heat-generating composition of a commercially available body warmer. As an air permeable inner bag, that of the commercially available body warmer was used, and the heat-generating composition of Example 1 was laminated on an air nonpermeable base material of the inner bag by mold-through molding to produce a heat-generating body. The air permeability of the air permeable part was 4.3 sec/100cc in terms of a Gurley air permeability. The heat-generating composition of the commercially available body warmer was difficult to maintain the shape, and thus a heat-generating body was produced by using a frame to make the same size as in the case where the same heat-generating composition as in Example 1 was used. That is, a heat-generating body having a size of a thickness of 5 mm, a length of 100 mm and a width of 80 mm was produced. One edge of each of the short edges (the edges having a length of 80 mm) of them was fixed on a plate, and two pieces of flannel cloth covers were superposed thereon. Subsequently, they were rose up with the plates to maintain perpendicularly. The surface temperatures of the body warmers at a position inside the short edges by 20 mm were measured, and two temperature measurement data were obtained per one body warmer. Upon comparing the temperature difference between the two positions inside the heat-generating body within the period of time where the temperature was maintained at 40°C or more, completely no deviation of the heat-generating composition was found, i.e., the temperature difference was from 2 to 4°C, in the case where the heat-generating composition of Example 1 was used, but the heat-generating composition was deviated downward in the case where the powder heat-generating composition of the commercially available body warmer was used, and the heat-generating agent was not present in the vicinity of the upper short edge to provide a temperature difference of from 40 to 45°C.

(Example 2)

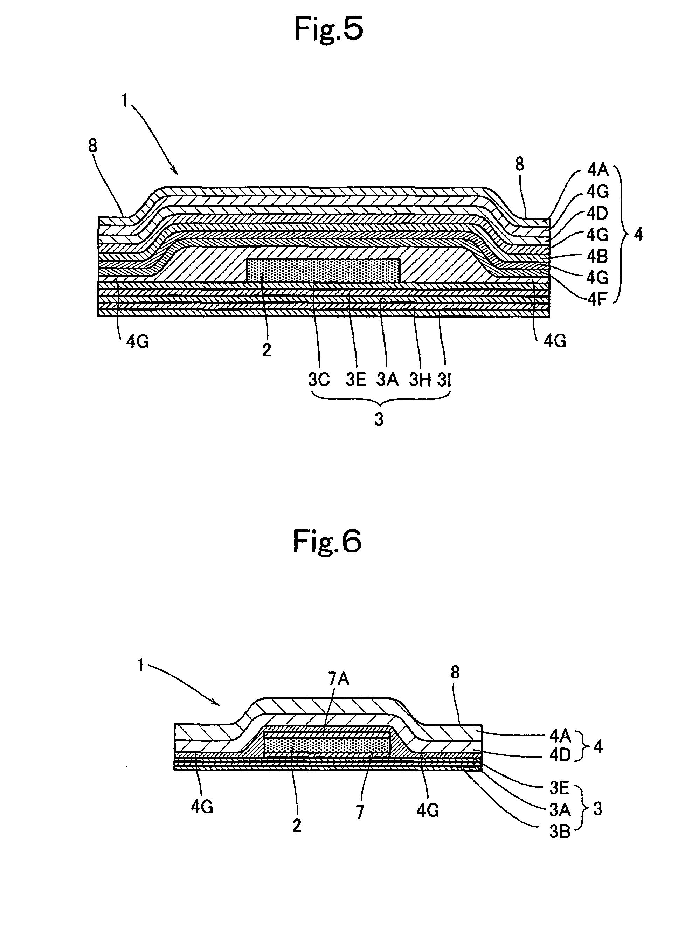

A heat-generating body 1 shown in Fig. 5 was obtained by using the heat-generating composition obtained in Example 1 in the same manner as in Example 1 except that the base material and the covering material were changed.

As the base material 3, such an air nonpermeable laminated film was used that was formed with, from the exposed surface, a releasing film 3I formed with a polyester film having a thickness of 100 μm, an adhesive agent layer 3H having a thickness of 30 μm, a polyethylene film 3A having a thickness of 50 μm, a polyethylene resin 3E having a thickness of 40 μm and a composite laminated nonwoven fabric 3C of a water absorbing cotton nonwoven fabric.

As the covering material 4, such a laminated film was used that was produced in such a manner that a polyester nonwoven fabric 4A (having a basis weight of 30 g/m$^2$) using fibers having kneaded therein a negative ion generating agent containing feldspar as a major component and a polyethylene porous film 4D having a thickness of 40 μm in this order from the exposed surface were sandwiched with mesh patterns of a hot melt adhesive agent 4G of a styrene-isoprene-styrene block copolymer (SIS) to achieve adhesive lamination, and then a hydrophilic water absorbing cotton nonwoven fabric 4B and a composite nonwoven fabric 4F of polyethylene fibers and polyester fibers were sequentially attached. The symbol 4G in the figure shows a hot melt adhesive agent. The covering material had an air permeability in terms of the Lyssy method of 300 g/m$^2$·24hr.

In this case, molding was carried out in such a manner that the heat-generating composition 2 was laminated on the base material 3, the hot melt adhesive agent 4G in a mesh form was provided thereon, the covering material 4 was overlaid thereon, and then the heat-generating composition 2 was subjected to a pressure roll treatment with a pair of pressure rolls with a gap between the pressure rolls adjusted to 1.5 mm over the base material 3 and the covering material 4, so as to obtain the heat-generating body 1.

The heat-generating body 1 was sealed in an air nonpermeable outer bag not shown in the figure immediately after the production.

Two pieces of the heat-generating bodies 1 were sealed in outer bags, respectively, and after lapsing 24 hours, they were taken out by breaking the bags and were attached on the surface of a human body to subject to ordinary use. The heat-generating bodies were increased in heating temperature until about 36°C within about 1 minute, and generated heat at about from 37 to 39°C for 6 hours or more. The heat-generating bodies 1 were fully flexible during the use to follow the expansion and contraction of the application region, and the heat-generating composition 2 was not moved inside the bags to provide uniform heat generation over the whole surface.

Furthermore, no breakage in shapes was found in both the bodies even when they were applied to a human body, and the body was moved, and uniform heat generation was found over the while surface.

(Example 3)

Fig. 6 is a cross sectional view of a heat-generating body 1 according to Example 3 of the invention. The heat-generating body 1 was formed in such a manner that a heat-generating composition 2 having the same composition as in the foregoing example having a water absorbing polymer (an N-vinylacetamide polymer crosslinked product not containing a cyclic anhydride and an acid and a salt derived therefrom) 7 and 7A uniformly diffused on both surfaces thereof in an amount of 0.5 part by mass per surface was laminated on a base material 3 in a film form, and a covering material 4 in a film form having a hot melt adhesive agent 4G in a mesh form provided thereon was overlaid thereon, followed by sealing by heat sealing to a width of 7 mm at a periphery 5 thereof.

That is, the heat-generating body 1 of this example is in the same form as in the heat-generating body 1 in Example 1 having a rectangular shape of a length of 85 mm and a width of 65 mm.

As the base material 3, such a material was used that was formed by laminating a polyester nonwoven fabric 3B (having a basis weight of 30 g/m$^2$) having a thickness of 50 μm on one surface of a polyethylene film 3A having a thickness of 50 μm, and laminating a polyethylene resin layer 3E polymerized with a metallocene catalyst on the other surface thereof. As the covering material 4, such a material was used that was formed by laminating a polyester nonwoven fabric 4A having a thickness of 50 μm on one surface of a porous polyethylene film 4D having a thickness of 50 μm in order to improve the mechanical strength and to obtain sufficient flexibility. The symbol 4G indicates a hot melt adhesive agent. The moisture permeability of the covering material 4 was adjusted to 300 g/m$^2$·24hr in terms of the Lyssy method.

In this case, molding was carried out in such a manner that the heat-generating composition 2 was laminated on the base material 3, the covering material 4 was provided thereon, and then the heat-generating composition 1 was subjected to a pressure roll treatment with a pair of pressure rolls with a gap between the pressure rolls adjusted to 1.5 mm over the base material 3 and the covering material 4, so as to obtain the heat-generating body 1.

The heat-generating body 1 was sealed in an air nonpermeable outer bag not shown in the figure immediately after the production.

The heat-generating body 1 was sealed in an outer bag, and after lapsing 24 hours, it was taken out by breaking the bag and was attached on the surface of a human body to subject to ordinary use. The heat-generating body was increased in heating temperature until about 36°C within about 1 minute, and generated heat at about from 37 to 39°C for 6 hours or more. The heat-generating body 1 was fully flexible during the use to follow the expansion and contraction of the application region, and the heat-generating composition 2 was not moved inside the bags to provide uniform heat generation over the whole surface.

Furthermore, no breakage in shapes was found in the body even when it was applied to a human body, and the body was moved, and uniform heat generation was found over the while surface.

(Comparative Example 5)

A heat-generating body was produced in the same manner as in Example 3 except that an isobutylene-maleic anhydride copolymer having a cyclic anhydride and an acid and a salt derived therefrom was used instead of the water absorbing polymer used in Example 3.

The heat-generating body produced in Example 2 and the heat-generating body produced in Comparative Example 5 were sealed in outer bags formed with an airtight film of polyethylene/biaxially stretched polypropylene/silicon oxide/polyethylene terephthalate, respectively, and stored at 50°C for 30 days, and then such a plumping test was carried out in that plumping of the outer bags was examined to measure the ratio of thickness change after the test with respect to the thickness of the outer bag containing the heat-generating body before the test (i.e., thickness after the test - thickness before the test).

It was 2% for the heat-generating body produced in Example 2, whereas it was 12% for that produced in Comparative Example 5. Under consideration of storage and the like, the heat-generating body of Example 2 had a far higher commercial value.

(Example 4)

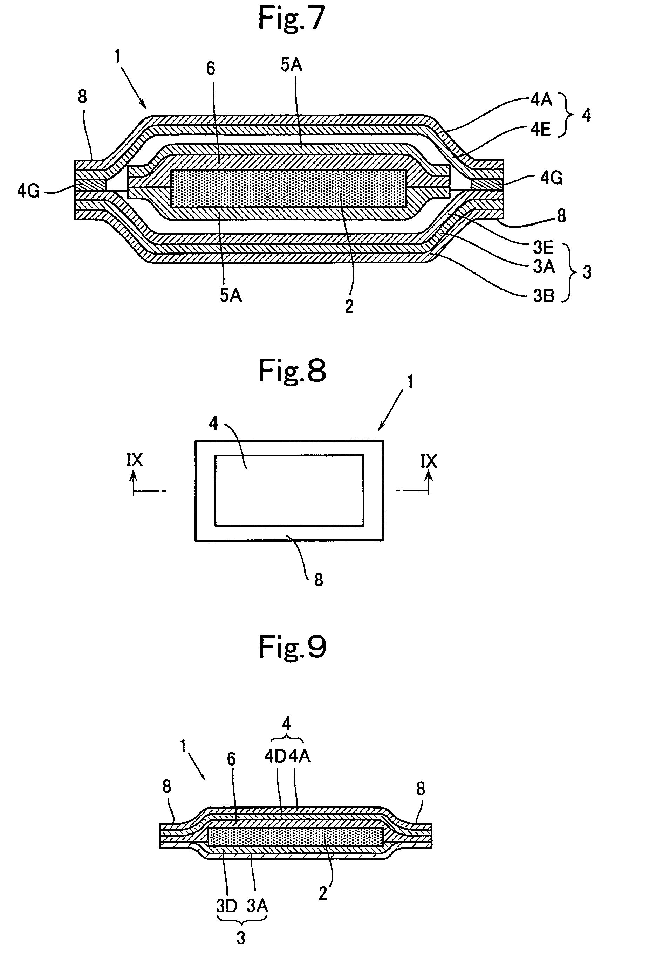

Fig. 7 is a cross sectional view of a heat-generating body 1 according to Example 4 of the invention. The heat-generating body 1 was formed in such a manner that a heat-generating composition 2 formed with the same composition as in Example 1 was laminated on a laying material 5A formed with a polyester nonwoven fabric having a basis weight of 200 g/m$^2$, an adhesive polymer 6 of a styrene-isoprene-styrene block copolymer (SIS) series was provided in a mesh form on the upper surface thereof by a melt flowing method, a laying material 5A formed with a polyester nonwoven fabric having a basis weight of 200 g/m$^2$ was overlaid thereon, and then the heat-generating composition was subjected to compression dehydration with a pair of pressure rolls with a gap between the pressure rolls adjusted to 1.5 mm over the laying material 5 and the laying material 5A, so as to attain mutual adhesion. It was then cut along the molded shape with a 3 mm margin outside the thus laminated heat-generating composition remaining.

The cut product was laminated on a base material 3 in a film form, and a covering material 4 having a mesh hot melt adhesive agent 4G provided thereon was overlaid thereon, followed by heat-sealing with a width of 7 mm at a periphery 8, so as to produce the heat-generating body 1.

As the base material 3, such a material was used that was formed by laminating a polyester nonwoven fabric 3B having a basis weight of 200 g/m$^2$ on one surface of a polyethylene film 3A having a thickness of 50 μm, and laminating a polyethylene resin 3E polymerized with a metallocene catalyst on the other surface thereof.

As the covering material 4, such a material was used that was formed by laminating a polyester nonwoven fabric 4A having a basis weight of 200 g/m$^2$ on one surface of a perforated polyethylene film 4E having a thickness of 100 μm in order to improve the mechanical strength and to obtain sufficient flexibility. The symbol 4G indicates a hot melt adhesive agent. The moisture permeability of the covering material 4 was adjusted to 300 g/m$^2$·24hr in terms of the Lyssy method.

The heat-generating body 1 was sealed in an air nonpermeable outer bag not shown in the figure immediately after the production.

The heat-generating body 1 was sealed in an outer bag, and after lapsing 24 hours, it was taken out by breaking the bag and was attached on the surface of a human body to subject to ordinary use. The heat-generating body was increased in heating temperature until about 36°C within about 1 minute, and generated heat at about from 37 to 39°C for 6 hours or more. The heat-generating body 1 was fully flexible during the use to follow the expansion and contraction of the application region, and the heat-generating composition 2 was not moved inside the bags to provide uniform heat generation over the whole surface.

(Example 5)

A heat-generating body 1 was produced in the same manner as in Example 1 except that the formulation of the heat-generating composition was changed to 6 parts by mass of sodium chloride (NaCl) as a metallic chloride, 50 parts by mass of water, 5 parts by mass of activated carbon as a carbon component, 7 parts by mass of wood powder passing through 18 mesh and 0.09 part by mass (in terms of solid content) of an adhesive agent aqueous emulsion (containing 100 parts by mass (in terms of solid content) of an acrylic polymer, 10 parts by mass (in terms of solid content) of an adhesiveness imparting resin and 0.5 part by mass (in terms of solid content) of an acrylic thickener as major components) , per 100 parts by mass of iron powder (DKP, produced by Dowa Teppun Co., Ltd.). The heat-generating composition had an incremental degree of viscosity of 400 cP, a water mobility value of 20 and a dispersion stabilization degree of 100.

According to the same manner as in Example 1, the heat-generating body 1 was sealed in an outer bag, and after lapsing 24 hours, it was taken out by breaking the bag and was attached on the surface of a human body to subject to ordinary use. It was increased in heating temperature until about 36°C within about 1 minute, and generated heat at about from 37 to 39°C for 6 hours or more. The heat-generating body 2 was fully flexible during the use to follow the expansion and contraction of the application region, and the heat-generating composition 2 was not moved inside the bags to provide uniform heat generation over the whole surface.

(Example 6)

100 parts by mass of iron powder, 4 parts by mass of activated carbon, 62 parts by mass of a potassium chloride aqueous solution (containing 7 parts by mass of KCl and 55 parts by mass of water) , 3 parts by mass of wood powder passing 100 mesh and 0.09 part by mass of CMC were well mixed to obtain a heat-generating composition. Herein, the incremental degree of viscosity was 500 cP, the water mobility value was 22, and the dispersion stabilization degree was 10.

On a base material 3 formed by providing a polyester nonwoven fabric 3D (having a thickness of 210 μm) containing a water absorbing polymer on one surface of an air nonpermeable polyethylene film 3A having a thickness of 40 μm, the heat-generating composition 2 was molded by a mold-through molding method using a leveling plate to a rectangular shape having a thickness of about 0.9 mm, a width of 5 cm and a length of 10 cm on the side of the nonwoven fabric 3D of the base material 3. An adhesive polymer 6 of a styrene-isoprene-styrene block copolymer series was provided in a mesh form on the upper surface by a melt blowing method, and a covering material 4 was overlaid thereon. Subsequently, the periphery of the laminated region was sealed by adhesion under pressure, and the circumference was cut to obtain a heat-generating body 1 having an ultrathin form and a width of the sealed part at the periphery 8 of 7 mm as shown in Figs. 8 and 9.

As the covering material 4, such a material was used that was formed by laminating a nylon nonwoven fabric 4A having a thickness of 150 μm on an outer surface of a polyethylene porous film 4D having a thickness of 100 μm. The covering material 4 had a moisture permeability in terms of a moisture permeable amount by the Lyssy method of 400 g/m$^2$·24hr.

The resulting heat-generating body 1 was then sealed in an outer bag to be sealed in the outer bag.

Thereafter, the outer bag was broken to carry out the heat-generating test.

As a result of the test, it was increased in heating temperature until about 36°C within about 1 minute, and generated heat at about from 37 to 39°C for 6 hours or more, and uniform heat generation was observed over the whole surface.

(Example 7)

It was a product that used a heat-generating composition 2 formed by mixing a fibrous substance in a heat-generating composition containing metallic powder as a major component, and a heat-generating body 1 was produced in the same manner as in Example 1 except that 6 parts by mass of sodium chloride (NaCl) as a metallic chloride, 50 parts by mass of water, 5 parts by mass of activated carbon as a carbon component, 3 parts by mass of wood powder passing 18 mesh as a water retainig agent, 0.04 part by mass of CMC as a water separation-preventing stabilizer and 0.1 part by mass of pulp as a fibrous substance were added to 100 parts by mass of iron powder (DKP, produced by Dowa Teppun Co., Ltd.)

The heat-generating body 1 was sealed in an air nonpermeable outer bag not shown in the figure immediately after the production.

When a test was carried out in the same manner as in Example 1, it was increased in heating temperature until about 36°C within about 1 minute, and generated heat at about from 37 to 39°C for 6 hours or more. The heat-generating body was fully flexible during the use to follow the expansion and contraction of the application region, and the heat-generating composition was not moved inside the bags to provide uniform heat generation over the whole surface.

Furthermore, no breakage in shapes was found in the body even when it was applied to a human body, and the body was moved, and uniform heat generation was found over the while surface.

(Example 8)

A heat-generating body 1 was produced in the same manner as in Example 1 except that 6 parts by mass of sodium chloride (NaCl) as a metallic chloride, 50 parts by mass of water, 5 parts by mass of activated carbon as a carbon component, 5 parts by mass of wood powder passing 100 mesh as a water retainig agent, 0.09 part by mass of CMC as a water separation-preventing stabilizer and 0.5 part by mass of toilet roll tissue paper as a fibrous substance were added to 100 parts by mass of iron powder (DKP, produced by Dowa Teppun Co., Ltd.).

According to the same manner as in Example 1, the heat-generating body 1 was sealed in an outer bag, and after lapsing 24 hours, it was taken out by breaking the bag and was attached on the surface of a human body to subject to ordinary use. It was increased in heating temperature until about 38°C within about 1 minute, and generated heat at about from 38 to 41°C for 7.5 hours or more. The heat-generating body was fully flexible during the use to follow the expansion and contraction of the application region, and the heat-generating composition was not moved inside the bags to provide uniform heat generation over the whole surface.

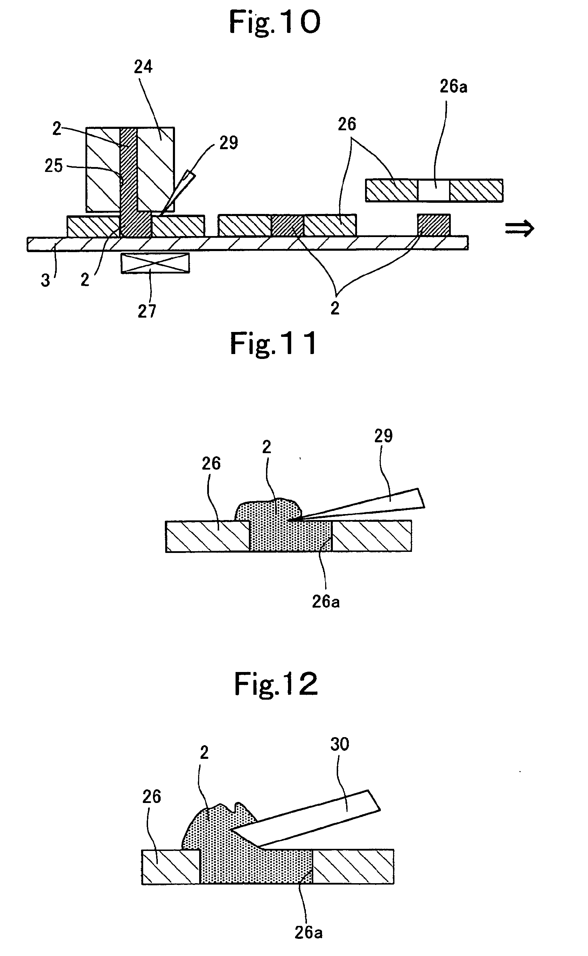

Fig. 10 shows an example of a mold-through molding method using a leveling plate 29. That is, a base material 3 in a form of a roll film having a width of 130 mm is horizontally conveyed at a prescribed speed between a dice 24 and a magnet 26, which are arranged in such a manner that, accommodating with a mold 26 for molding having a thickness of 1 mm and having a mold vacancy 26a having a prescribed shape at a center of the mold, the dice 24 is arranged on the upper surface thereof, and the magnet 27 is arranged on the lower surface thereof. Upon feeding the heat-generating composition 2 in a sherbet form of the invention to the mold vacancy 26a from the upper surface of the mold 26 through a hole 25 of the dice 24, the heat-generating composition 2 is leveled to the same surface of the mold 26 with the leveling plate 29 placed ahead in the conveying direction and simultaneously charged in the mold vacancy 26a, so as to be molded to a desired shape with a thickness of 1 mm on the base material 3. Thereafter, the mold 26 is released to obtain a mold article laminated on the base material 3.

While not shown in the figure, an adhesive polymer of a styrene-isoprene-styrene block copolymer (SIS) series is provided in a mesh form on the molded article by a melt blowing method, a covering material is overlaid thereon, and the periphery of the molded article region is sealed by heat sealing, followed by cutting into a desired shape, so as to obtain a heat-generating body having a desire shape. Furthermore, the heat-generating body of the invention thus cut is fed to a packing step to be sealed in an outer bag having airtightness. The same molding can be carried out by using a pressing and leveling plate instead of the leveling plate. Fig. 11 shows the leveling plate, and Fig. 12 shows the pressing and leveling plate. The numeral 30 in Fig. 12 denotes the pressing and leveling plate.

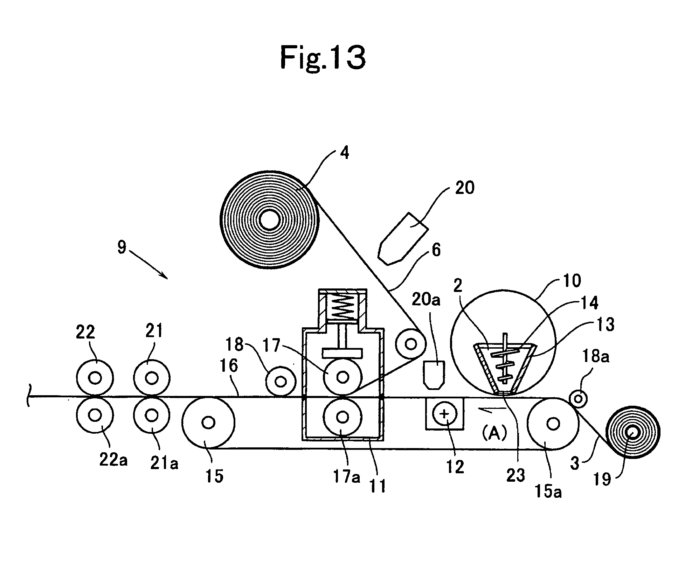

Fig. 13 shows a production apparatus 9 for preferably producing a heat-generating body according to the invention, as shown in the figure, the production apparatus 9 is constituted with a drum molding device 10 for molding the heat-generating composition of the invention, a rolling device 11 for adjusting by rolling the thickness of the heat-generating composition in a squamous form or a sheet form obtained in the drum molding device 10, die rolls 21 and 21a, and die cut rolls for cutting 22 and 22a. In this case, the heat-generating composition used in Example 1 was used as the heat-generating composition.

The drum molding device 10 is equipped with a screw 14 inside a hopper 13 positioned on the upstream (upper right in the figure) of the production apparatus 9.

The rolling device 11 has press rolls 17 and 17a provided in the course of conveying direction of a belt conveyer 16 positioned under the molding device 10, so as to sandwich the belt conveyer 16 vertically.

A driving device 12 is a driving power source for the belt conveyer 16 and the press rolls 17 and 17a. Furthermore, a tension roller 18 is provided, and a roll 18a is provided on one side of the belt conveyer 16 for pressing the base material.

The numerals 15 and 15a in the figure denote supporting rollers for the belt conveyer 16. While not shown in the figure, the belt conveyer 16 and the press rolls 17 and 17a can be driven inversely through operation of a switch after releasing the die rolls 21 and 21a and the die cut rolls 22 and 22a.

The rolling device 11 having such a constitution includes rolling methods of a single stage press rolling method using repeated rolling and a multistage press rolling method, which will be described later.

The process for producing a heat-generating body in a sheet form (heat-generating sheet) will be described with reference to Fig. 13.

The base material 3 wound in a roll form is fed from the feeding roll 19, and the heat-generating composition 2 of Example 1 is placed in the hopper 13. Upon rotating the screw 14, the composition 2 is molded from the hopper 13 to the belt conveyer 16 through the drum molding device 16 as a molded article 23 in a squamous form or a sheet form on a center part of a water absorbing film as the base material 3 by mold-through molding to a size of 110 mm x 70 mm with an interval of 20 mm. Furthermore, the heat-generating composition 2 thus molded is covered with a covering material 4 fed from the covering material in a roll form 4, which is formed with a porous film having coated on the whole surface thereof a hot melt adhesive agent (JM6041, a trade name, produced by Nippon Fular Co., Ltd.) with a melt blowing machine 20 at a temperature of 160°C to 5 g/m$^2$, in the course of conveying in the direction (A) on the belt conveyer 16, so as to make in contact with the layer 6 of the hot melt adhesive agent (JM6041, a trade name, produced by Nippon Fular Co., Ltd.), and then it is rolled with the press rolls 17 and 17a and further sealed at a periphery of the base material 3 and the covering material 4 with a die rolls 21 and 21a for sealing to a size of 130 mm x 80 mm, followed by cutting with the die cut rolls 22 and 22a, so as to obtain a heat-generating body. It is possible that the melt blowing machine 20 is changed to a melt blowing machine 20a, so that the hot melt adhesive agent layer 6 is made in contact with the laminated product of the heat-generating composition or the base material 3.

In this case, the base material 3 is formed with a five-layer film with retractility having a width of 130 mm. That is, a polyester spunless nonwoven fabric having a basis weight of 40 g/m$^2$ is laminated on a polyester releasing film having a thickness of 38 μm with a hot melt adhesive agent layer having a basis weight of 150 g/m$^2$ intervening therebetween, and a water absorbing film formed by containing a water absorbing polymer in an amount of 25 g/m$^2$ in a polyester spunless nonwoven fabric having a basis weight of 30 g/m$^2$ is adhered under heat on the side of the polyester spunless nonwoven fabric of the three-layer film with a polyethylene film (polymer formed with a metallocene catalyst) with rubber elasticity having a thickness of 30 μm intervening therebetween, so as to provide the five-layer film.

On the other hand, the covering material 4 is formed with a three-layer film with retractility having a width of 130 mm. That is, a hot melt adhesive agent layer having a basis weight of 5 g/m$^2$ is formed on a porous film having a basis weight of 50 g/m$^2$ at a temperature of 160°C with a melt blowing machine, and a spunless nonwoven fabric having a basis weight of 30 g/m$^2$ is laminated on the hot melt adhesive agent layer to form the three-layer film.

The covering material 4 has a moisture permeability in terms of a moisture permeable amount of 350 g/m$^2$·24hr by the ASTM method (E-96-80D method).

As the heat-generating composition 2, the following was used.

The heat-generating composition 2 is formed by mixing a fibrous substance in a heat-generating composition containing metallic powder as a major component, and the heat-generating composition 2 used in this example is formed by mixing 6 parts by mass of sodium chloride (NaCl) as a metallic chloride, 50 parts by mass of water, 5 parts by mass of activated carbon as a carbon component, 5 parts by mass of wood powder passing 100 mesh and 0.05 part by mass of CMC as a water separation-preventing stabilizer with 100 parts by mass of iron powder (DKP, produced by Dowa Teppun Co., Ltd.).

By stabilizing dispersion of the heat-generating composition, stable lamination of the heat-generating composition by mold-through molding on a central part of the water absorbing film in the base material 3 can be carried out, whereby the lamination region can be controlled with high accuracy, and the film thickness can be controlled to a very small thickness with uniformity. Furthermore, the heat-generating composition 2 is prevented from moving inside the bag owing to the bonding force between the water absorbing film in the base material 3 and the heat-generating composition 2. The thickness of the heat-generating composition layer 2 is made small, and thus the heat-generating body can be formed into an ultrathin form.

In this example, the water absorbing base material 3 in the form of a roll film having a width of 130 mm is horizontally conveyed at a speed of 20 mm/min, and the heat-generating composition 2 is molded through the mold on the upper surface thereof at a thickness of about 1.0 mm. Immediately after the mold-through molding, a hot melt adhesive agent is coated on the whole surface of the porous film of the water absorbing covering material 4 by a melt blowing method at a temperature of 160°C to 5 g/m$^2$, and simultaneously, the covering material 4 is overlaid thereon in such a manner that the hot melt adhesive agent layer 6 is in contact therewith, followed by sealing a periphery thereof with the hot melt adhesive agent layer, so as to produce a heat-generating body in an ultrathin form having a thickness of about 0.94 mm. Such a rolling method in that the composition and the components of the heat-generating composition of the invention is formed into a heat-generating sheet having a desired thickness by rolling once using a pair of rolling means is referred to as the single stage press roll method.

It is needless to say that in the case where a heat-generating sheet having a desired thickness cannot be obtained by rolling once or in the case where change in thickness or a high density is required, the belt conveyer 16 and the press rolls 17 and 17a are driven in the invert direction before cutting with the die cut rolls, whereby the heat-generating composition 2 in a sheet form sandwiched by the base material and the covering material is conveyed and again subjected the heat-generating composition 2 to rolling with the press rolls 17 and 17a.

Furthermore, it is also possible that the process steps are repeated to produce sheets having various densities and thickness. The heat-generating body in a sheet form thus again rolled is further cut with the die cut rolls 22 and 22a.

The thus cut heat-generating body is conveyed to a packing step and sealed in an outer bag having air tightness not shown in the figure.

The multistage press rolling method for continuously producing a functional sheet having a prescribed thickness by omitting the repeating operation will be described. The main constitution thereof is the same as the single stage press rollingmethod, but in the case of the multistage pressingmethod, what is different from the single stage press rolling method is that plural pressing rolls are arranged on the belt conveyer 16, whereby the sheet production process is completed in a considerably short period of time to provide an advantage in considerable improvement of productivity. Of course, any of these two methods may be employed in the invention.

In the case where a water permeable belt, such as a belt with holes and the like, is used as the belt of the belt conveyer, and a water permeable laying material (such as a polypropylene nonwoven fabric) is used instead of the base material and the covering material, compression dehydration can be carried out upon compression with the pressing rolls, so as to provide a heat-generating body in a heating state. The heat-generating body in a sheet form thus obtained by cutting, or the heat-generating body in a strip form before cutting may be laminated on a water nonabsorbing base material and then covered with a water nonabsorbing covering material, followed by sealing a periphery thereof, to make a heat-generating body.

In the rolling device 11, the packing material containing the base material 3 and the covering material 4 may also be used as a sealing device for sealing the heat-generating composition.

The excessive water content of the heat-generating composition 2 is controlled with the water mobility value, and thus it is gradually absorbed by the base material 3 aftermolding through the mold on the base material 3, and the covering material 4 is overlaid thereon. However, the period of time from the mold-through molding of the heat-generating composition 2 on the base material 3 to the sealing in the outer bag is extremely small, and thus, there are substantially no case where the excessive water content of the heat-generating composition 2 is absorbed by the base material 3 in such an extent that causes a heat-generating reaction.

Therefore, there is substantially no possibility of causing a heat-generating reaction of the heat-generating composition in the production process, and there is substantially no possibility of causing loss due to the heat-generating reaction and quality deterioration of the heat-generating composition. Furthermore, there is substantially no possibility that the heat-generating composition solidified in the'process steps from the mixing of the heat-generating composition to the mold-through molding on the base material 3, whereby various kinds of problems can be prevented, such as reduction in yield due to solidification, termination of the operation, restriction in operation time, difficulty and danger in cleaning the production apparatus, frequency in cleaning the production apparatus, difficulty in processing solidified products, and the like.

After sealed in an outer bag, it was taken out by breaking the bag after lapsing 24 hours and was attached on the surface of a human body to subject to ordinary use. It was increased in heating temperature until about 36°C within about from 1 to 2 minutes, and generated heat at about from 37 to 39°C for 6 hours or more. During the use, the heat-generating composition layer was not moved inside the bags to provide uniform heat generation over the whole surface.

Another example of the heat-generating body according to the invention is formed by sealing a heat-generating layer 2 in a flat bag body (packing material) in a rectangular shape having a length of 130 mm and a width of 80 mm, and the bag body is formed with a base material 3 having airtightness and a covering material 4 having air permeability. Furthermore, an adhesive agent layer 3H having a basis weight of 150 $g/m^2$ is formed on the exposed surface of the base material 3, and in this case, the exposed surface of the adhesive agent layer 3H is covered with a polyester releasing film 3I.

Fig. 15 is a side cross sectional view showing a sealing apparatus for the heat-generating composition 2 in a sheet form. In order to seal the heat-generating composition 2 in a sheet form with the base material 3 and the covering material 4, one end of the heat-generating composition 2 sandwiched by the base material 3 and the covering material 4 formed with nonwoven fabrics is laminated on the base material 3 placed on a belt conveyer 16b driven in the direction (B) before subjecting to a die cut treatment, and the covering material 4 is overlaid and is heated and sealed at the periphery thereof with heat rollers 21 and 21a, followed by cutting the sealed part, so as to produce a functional product having a desired size. In the case where the adhesive agent is used as a sealing means, the heat rollers may be replaced by pressing rollers or heating and pressing rollers.

In the case where a water permeable belt, such as a belt with holes and the like, is used as the belt of the belt conveyer, a water permeable laying material (such as a polypropylene nonwoven fabric) is used instead of the base material and the covering material, and compression dehydration can be carried out upon compression with the pressing rolls, so as to provide a heat-generating body in a heating state, followed by sealing the heat-generating body with the water nonabsorbing base material and covering material to form a heat-generating body, the apparatus shown in Fig. 15 is used instead of the die cut rolls 22 and 22a or the die cut rolls 22 and 22a and the sealing device 11a in Fig. 13.

Furthermore, instead of the method carried out in Example 1, i.e., the hot melt adhesive agent is previously coated on the whole surface of the porous film of the covering material by a melt flowing method to ensure air permeability, the covering material is overlaid on the heat-generating composition 2 to make the hot melt adhesive agent layer in contact therewith, such a method may be employed in that a hot melt adhesive agent is coated by a melt blowing method on the heat-generating composition and the base material formed in the same manner as in Example 1, and the covering material is overlaid thereon to make the porous film side in contact with the hot melt adhesive agent.

Instead of the method of coating a hot melt adhesive agent by a melt blowing method on the whole surface of the porous film of the covering material, such a method may be employed in that an isoprene adhesive agent is transferred by gravure printing on the surface periphery of the porous film of the covering material, and the base material and the covering material are sealed with the isoprene adhesive agent.

It is also possible to obtain a heat-generating body in the following manner. An acrylic adhesive agent layer having a thickness of 50 μm is formed on the whole surface of the water absorbing film of the base material by a known method. The base material wound in a roll form is conveyed, and simultaneously, the heat-generating composition is magnetically transferred by a known magnetic transferring method to the center part of the acrylic adhesive agent layer, i.e., the heat-generating composition is attached to a magnet sheet. Furthermore, the heat-generating composition is magnetically transferred to the base material with another magnet sheet at regular time intervals, and the coveringmaterial is overlaid thereon, i.e., on the base material and the heat-generating composition, in such a manner that the porous film thereof is in contact therewith. The periphery of the base material and the covering material is sealed with the acrylic adhesive agent by passing through nip rolls, and cut into a prescribed size, so as to obtain a heat-generating body.

In the case the periphery of the heat-generating body, i.e., the periphery of the base material and the covering material, is sealed by using an adhesive agent, it is possible that they are subjected to a heat treatment at 60°C partially with a prescribed interval in a linear form, whereby the sealing of the base material and the covering material is further ensured but is difficult to be released with improved reliability.

## Claims

1. A heat-generating composition comprising, as essential components, a heat-generating substance generating heat upon reaction with oxygen, a carbon component, an oxidation promoter and water, **characterized in that** the composition further contains a water separation-preventing stabilizer in a proportion of from 0.001 to 0.25 part by mass per 100 parts by mass of the heat-generating substance, and has a water mobility value of from 7 to 40 and a separation degree of from 0 to 30.

2. A heat-generating composition as described in claim 1, **characterized in that** at least one selected from a water retainigagent, asurfactant, adefoamingagent, a pH controlling agent, a hydrophobic polymer compound, a pyroelectric substance, a far infrared radiating substance, a negative ion generating substance, a hydrogen formation inhibitor, an antioxidant, an aggregate, a fibrous material, a fertilizer component and a heat-generating assistant is mixed with the heat-generating composition.

3. A heat-generating composition as described in claim 2, **characterized in that** the water retainig agent contains an organic water retainig agent, and particles having a particle diameter of 1,000 μm or less occupies at least 50% or more by mass of the water retainig agent.

4. A heat-generating composition as described in one of claims 1 to 3, **characterized in that** an incremental degree of viscosity (at a temperature of 20°C) is less than 1,000 cP.

5. Aheat-generating composition as described in claim 4, **characterized in that** at least one selected from a binder, a thickening agent, an excipient, an aggregating agent, a soluble adhesive material and a water absorbing polymer is mixed with the heat-generating composition.

6. Aheat-generating composition as described in claim 5, **characterized in that** the water absorbing polymer does not contain a cyclic anhydride, an acid derived therefrom, and a salt thereof.

7. A heat-generating body **characterized by** charging a heat-generating composition as described in claim 1 in a container bag having air permeability in at least a part thereof.

8. A heat-generating body as described in claim 7, **characterized in that** the container bag contains a base material in a film form, a sheet form or a nonwoven fabric form, and a covering material in a film form, a sheet form or a nonwoven fabric form, at least one of, or at least a part of the base material and the covering material has air permeability.

9. A heat-generating body characteri zedby laminating a heat-generating composition as described in claim 1 on a laying material in a film form, a sheet form or a nonwoven fabric form, and optionally further laminating another laying material thereon, and at least one, or at least a part of the laying material has water permeability.

10. A heat-generating body as described in claim 9, **characterized in that** at least a part of the heat-generating composition of the heat-generating body is in a state dehydrated to enable substantial heat generation in air by at least one means selected from physical forced drainage by compression, decompression, compression and decompression, and the like, radiation of water content by allowing to stand, and water absorption with a material such as a water absorbing base material, a water absorbing agent, a water absorbing material or the like.

11. A heat-generating body as described in claim 10, **characterized by** charging the heat-generating body in a container bag having air permeability in at least a part thereof.

12. A heat-generating body as described in claim 8 or 9, **characterized in that** a part of water content of the heat-generating composition is absorbed and/or held by at least one, or at least a part of the container bag, the base material, the covering material and the laying material.

13. A heat-generating body as described in claim 12, **characterized in that** at least one selected from iron powder, a carbon component, a fibrous material, a binder, a thickener, an excipient, an aggregating agent, a soluble adhesivematerial, a far infrared radiating substance, a negative ion generating substance, a pyroelectric substance, an organic silicon compound, a water absorbing agent, a water absorbing polymer and a water separation-preventing stabilizer is laminated, diffused or coated on one surface or both surfaces of the heat-generating composition.

14. A heat-generating body as described in claim 13, **characterized in that** the water absorbing polymer does not contain a cyclic anhydride, an acid derived therefrom, and a salt thereof.

15. A heat-generating body as described in claim 12, **characterized in that** a part or whole of a surface of at least one selected from the covering material, the heat-generating composition and the material laminated or diffused on the heat-generating composition is covered with an air permeable polymer.

16. A heat-generating body as described in claim 15, **characterized in that** in at least a periphery of the heat-generating composition, the base material and the covering material of the container bag are sealed by adhesion, cohesion or thermal fusion over a full circumference or in a part thereof.

17. A heat-generating body as described in claim 16, **characterized in that** at least one, or at least a part of the base material, the covering material and the laying material is formed with a water absorbing material having water absorbing property in a film form, a sheet form or a nonwoven fabric form.

18. A heat-generating body as described in claim 17, **characterized in that** a water absorbing layer containing a water absorbing material or a water absorbing agent is provided on at least a contact part or a part of the contact part of the base material and/or the covering material and/or the laying material in contact with the heat-generating composition.

**19.** A heat-generating body as described in claim 18, **characterized in that** a part having water absorbing power of the base material, the covering material and the layingmaterial, and the water absorbing layer have a water absorbing power of 1 g/m$^2$ or more.

**20.** A heat-generating body as described in claim 19, **characterized in that** at least one of the base material, the covering material and the laying material has an elongation property.

**21.** A heat-generating body as described in claim 20, **characterized in that** at least one, or at least a part of the base material, the covering material and the laying material is laminated with, is coated with, or contains at least one of a far infrared radiating substance, a negative ion generating material and a pyroelectric substance.

**22.** A heat-generating body as described in claim 21, **characterized in that** unevenness is formed on a whole surface or a part thereof of a surface layer of the heat-generating composition.

**23.** A heat-generating body as described in claim 22, **characterized in that** unevenness is formed on a part or whole of a surface of a layer of the heat-generating composition or a part or whole of a surface of a material having the heat-generating composition laminated thereon.

**24.** A heat-generating body as described in claim 12, **characterized in that** an adhesive agent layer or a gel layer is laminated on an exposed surface or a part thereof of one of the base material and the covering material.

**25.** A heat-generating body as described in claim 24, **characterized in that** the adhesive agent layer or the gel layer is a fomentation layer containing a fomentation drug, or a drug-containing layer having a percutaneous absorption drug contained or supported therein.

**26.** A heat-generating body as described in claim 25, **characterized in that** a heat-generating composition as described in claim 1 is provided in a prescribed form on at least one selected from a base material, a laying material and a covering material, and is then made into such a state that it is charged in a container bag having air permeability on at least a part thereof, whereby the heat-generating composition under a heat-generating state is retained at a prescribed position irrespective to a pressure inside the container bag with respect to an outer pressure, the bag is maintained to have a flat form during a period where the body is applied to an applicable position, and a deviation of the heat-generating composition is 10% or less in comparison to that before heat generation.

**27.** A process for producing a heat-generating body **characterized by** molding, such as laminating, a heat-generating composition as described in claim I on at least one prescribed region on a base material in a film form, a sheet form or a nonwoven fabric form; overlaying a covering material in a film form, a sheet form or a nonwoven fabric form to cover and charge the heat-generating composition; and imparting air permeability to at last one, or at least a part of the base material and the covering material.

**28.** A process for producing a heat-generating body **characterized by**, upon laminating a heat-generating composition as described in claim 1 on a base material in a film form, a sheet form or a nonwoven fabric form, laminating, diffusing or coating at least one selected from iron powder, a carbon component, a fibrous material, a binder, a thickener, anexcipient, anaggregatingagent, asolubleadhesivematerial, a far infrared radiating substance, a negative ion generating substance, a pyroelectric substance, an organic silicon compound, a water absorbing agent, a water absorbing material, awaterabsorbingpolymer, awaterretainigagent, and dispersing stabilizer on one surface or both surfaces of the laminated body of the heat-generating composition; overlaying a covering material in a film form, a sheet form or a nonwoven fabric form to cover and charge the laminated body of the heat-generating composition and those thus laminated, diffused or coated; and imparting air permeability to at last one, or at least a part of the base material and the covering material.

**29.** A process for producing a heat-generating body **characterized by** laminating a heat-generating composition as described in claim 1 on a base material in a film form, a sheet form or a nonwoven fabric form; overlaying a covering material in a film form, a sheet form or a nonwoven fabric form thereon; adhering the base material and the covering material with at least one selected from the base material, the laminated material and the covering material, or an air permeable polymer provided on a part thereof; punching out the resulting laminated material into an arbitrary shape; and imparting air permeability to at last one, or at least a part of the base material and the covering material.

**30.** A process for producing a heat-generating body **characterized by** laminating a heat-generating composition as described in claim 1 on a water permeable material; covering the laminated material with a water permeable material; carrying out physical forced drainage by aspiration, centrifuge, compression, decompression, drying, or compression and decompression, to form a laminated body, and optionally laminating it to form a laminated body; punching out the laminated body into an arbitrary shape, and placing the punched laminated body on a base material, or placing the laminated body without punching on a base material; overlaying a covering material in a film form, a sheet form or a nonwoven fabric form thereon; sealing at least the base material and the covering material among the base material, the covering material and the water permeable material on a periphery thereof by at least one selected from cohesion, adhesion and thermal fusion; and in the case where the laminated body has not been punched out into an arbitrary shape, punching the laminated body into an arbitrary shape; and imparting air permeability to at last one, or at least a part of the base material and the coveringmaterial.

31. A process for producing a heat-generating body as described in claim 30, **characterized in that** at least one, or at least a part of the base material, the covering material and the laying material is laminated with, is diffused with, or contains at least one of a far infrared radiating substance, a negative ion generating substance and a pyroelectric substance.

32. A process for producing a heat-generating body as described in claim 30 or 31, **characterized in that** the heat-generating body is inserted between air non-permeable films or sheets; and simultaneously with the insertion or after the insertion, the films or sheets are sealed on a periphery of the heat-generating body to a size exceeding a size of the heat-generating body, and simultaneously with the sealing or after the sealing, the resulting assembly is punched out, or simultaneously with the insertion or after the insertion, the two films or sheets are punched out to a size exceeding a size of the heat-generating body, and simultaneously with the punching or after the punching, the films or sheets are sealed on a periphery of the heat-generating body to a size exceeding a size of the heat-generating body.

# Fig.1

# Fig.2

## Fig.3

LOWER 50% OF HEAT-GENERATING
COMPOSITION OF COMPARATIVE EXAMPLE 1

LOWER 50% OF HEAT-GENERATING
COMPOSITION OF EXAMPLE 1

HEAT-GENERATING
COMPOSITION OF
COMPARATIVE EXAMPLE 1

UPPER 50% OF HEAT-GENERATING
COMPOSITION OF EXAMPLE 1

HEAT-GENERATING
COMPOSITION OF EXAMPLE 1

UPPER 50% OF HEAT-GENERATING
COMPOSITION OF COMPARATIVE
EXAMPLE 1

## Fig.4

HEAT-GENERATING BODY
OF COMPARATIVE
EXAMPLE 1 (AFTER TEST)

HEAT-GENERATING BODY
OF COMPARATIVE
EXAMPLE 1 (BEFORE TEST)

HEAT-GENERATING
COMPOSITION OF
EXAMPLE 1 (AFTER TEST)

HEAT-GENERATING
COMPOSITION OF
EXAMPLE 1(BEFORE TEST)

# Fig.5

4A
4G
4D
4G
4B
4G
4F
} 4

1
8
8
4G
4G
2
3C 3E 3A 3H 3I
3

# Fig.6

1
7A
8
4A } 4
4D
3E } 3
3A
3B
4G
2
7
4G

# Fig.7

# Fig.8

# Fig.9

## Fig.10

## Fig.11

## Fig.12

# Fig.13

## Fig.14

## Fig.15

## Fig.16

# Fig.17

# Fig.18

## Fig.19

## Fig.20

## Fig.21

## INTERNATIONAL SEARCH REPORT

| | International application No. |
|---|---|
| | PCT/JP02/11097 |

A. CLASSIFICATION OF SUBJECT MATTER
    Int.Cl⁷ C09K5/18, A61F7/08

According to International Patent Classification (IPC) or to both national classification and IPC

B. FIELDS SEARCHED

Minimum documentation searched (classification system followed by classification symbols)
    Int.Cl⁷ C09K5/18, A61F7/08

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2000-260 A (Kabushiki Kaisha Genchi Kenkyusho),<br>07 January, 2000 (07.01.00),<br>Claims 1 to 9; column 12, line 40; Par. Nos.<br>[0080], [0085] to [0090], [0105] to [0111];<br>examples 1 to 3<br>(Family: none) | 1-3,5-32<br>4 |
| X<br><br>Y | JP 11-206801 A (Kabushiki Kaisha Genchi<br>Kenkyusho),<br>03 August, 1999 (03.08.99),<br>Claims 1 to 7; Par. Nos. [0085] to [0088]; column<br>16, line 8; Par. Nos. [0108], [0116] to [0119];<br>examples 1 to 5<br>(Family: none) | 1-3,5-21,<br>24-32<br>22-23 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    16 January, 2003 (16.01.03) | Date of mailing of the international search report<br>    28 January, 2003 (28.01.03) |
|---|---|
| Name and mailing address of the ISA/<br>    Japanese Patent Office | Authorized officer |
| Facsimile No. | Telephone No. |

Form PCT/ISA/210 (second sheet) (July 1998)

**EP 1 439 213 A1**

## INTERNATIONAL SEARCH REPORT

| | International application No. |
|---|---|
| | PCT/JP02/11097 |

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | JP 10-263002 A (Kabushiki Kaisha Genchi Kenkyusho), 06 October, 1998 (06.10.98), Claim 1 (Family: none) | 22-23 |
| A | JP 10-216167 A (Kabushiki Kaisha Genchi Kenkyusho), 18 August, 1998 (18.08.98), Claims 1 to 2 (Family: none) | 1-32 |

Form PCT/ISA/210 (continuation of second sheet) (July 1998)

58